# EUROPEAN PATENT APPLICATION

(11) **EP 3 848 387 A1**
(43) Date of publication of application: **14.07.2021**
(21) Application number: 19858411.2
(22) Date of filing: 05.09.2019
(51) Int. Cl.: C07K 14/54, C07K 14/725, C07K 14/715, C07K 16/24, C12N 5/0783, A61K 38/00

(54) **CHIMERIC ANTIGEN RECEPTOR FOR SOLID CANCER AND T CELLS EXPRESSING CHIMERIC ANTIGEN RECEPTOR**

(30) Priority: 05.09.2018 US 201862727254 P
(71) Applicant: Kong, Seogkyoung, Seoul 06335 (KR)
(72) Inventor: Kong, Seogkyoung, Seoul 06335 (KR)
(74) Representative: Schiweck Weinzierl Koch Patentanwälte Partnerschaft mbB
(86) International application number: PCT/KR2019/011516
(87) International publication number: WO 2020/050667

(57) **Abstract**

Disclosed is a chimeric antigen receptor with improved persistency.

## Description

### [Technical Field]

The present invention relates to a novel chimeric antigen receptor (CAR) for effectively treating blood cancer or solid cancer, and to a CAR-T cell in which a chimeric antigen receptor targeting a specific cancer antigen is expressed. In addition, the present invention relates to a vector which expresses a novel CAR for effectively treating blood cancer or solid cancer.

More specifically, the present invention relates to a chimeric antigen receptor comprising an antigen binding domain; a hinge region; a transmembrane domain; a costimulatory domain; and a cytoplasmic signaling domain, in which any one immunoreceptor tyrosine-based activation motif (hereinafter described as "ITAM") present within a CD3ζ domain, which is a cytoplasmic signaling domain, is mutated for excellent *in vivo* persistence and anti-tumor effects of CAR-T cells, and to a CAR-T cell in which the chimeric antigen receptor is expressed.

Further, in order to induce a cytokine signal in an antigen-dependent manner during stimulation of cancer antigens, a cleaved cytoplasmic domain of interleukin-7 receptor-a (IL7Rα) or interleukin-2 receptor-β (IL-2Rβ) in addition to the CD3ζ signaling domain and a 4-1BB costimulatory domain was added to a CAR gene so as to reduce the differentiation and exhaustion of CAR-T cells caused by CAR tonic signaling of CAR-T cells, thereby making it capable of exhibiting excellent *in vivo* persistence and anti-tumor effects.

In addition, in the present invention, a chimeric switch receptor was introduced to convert the immunosuppressive signal in the hostile tumor microenvironment into an activation signal in CAR-T cells.

In addition, in the present invention, the cytokine IL-21 is released out of CAR-T cells so as to aid the activation of innate immune-related cells and increase the content of less-differentiated memory CAR-T cells.

The chimeric antigen receptor according to the present invention can show excellent *in vivo* persistence and anti-tumor effects of CAR-T cells in a hostile tumor microenvironment, and can simultaneously reduce toxicity, thereby reducing side effects in the patient while improving the treatment effect. In addition, it is possible to expand the scope of use of the present invention by preparing a chimeric antigen receptor-provided allogenic (non-self-derived) CAR-T treatment according to the present invention.

### [Background]

T cells expressing chimeric antigen receptor (CAR) (CAR-T cells) refers to a type of T cell with a recombinant gene, in which a gene encoding a receptor that recognizes cancer cell surface antigens specifically expressed on the surface of cancer cells is introduced into a T cell to kill cancer cells.

Dr. Zelig Eshhar, an Israeli chemist and immunologist at the Weizmann Institute of Science, *et al.* previously proposed a hypothesis that when T cells are artificially made with a receptor that binds to an antigen specifically expressed in a cancer cell, it is possible to target only cancer cells and trigger an immune response to kill cancer cells. They succeeded in preparing T cells provided with a chimeric antigen receptor, and this was reported in PNAS in 1989.

In the CAR-T cells prepared in the early days *(i.e.,* the first-generation CAR-T cells), only CD3ζ was used as a signaling domain. However, these CAR-T cells had drawbacks in that the treatment effect was insignificant and that the persistence was also short. Therefore, efforts have been made to improve the reactivity of CAR-T cells, and as a result, second-generation CAR-T cells were prepared in which a costimulatory domain (CD28 or CD137/4-1BB) and CD3ζ were combined, and these CAR-T cells showed an increase in the number of CAR-T cells remaining in the body compared to the first-generation CAR-T cells.

In the second-generation CAR-T cells, only one kind of costimulatory domain was used, and CAR-T cells using two kinds of costimulatory domains are referred to as third-generation CAR-T.

With regard to the method of treating cancer using CAR-T cells, three late-stage chronic lymphoid leukemia (CCL) patients were injected with cytotoxic T cells which were modified to recognize CD19. As a result, leukemia was completely cured in two of the patients, and there were reports that the conditions lasted for about 10 months *(*N Engl J Med 2011, 365:725-733, August 25, 2011; Sic Transl Med 2011 Aug 10, 3(95):95ra73). In particular, the CAR-T used corresponded to the second-generation CAR-T cells, in which 4-1BB was used as the costimulatory domain and CD3ζ was used as the signaling domain. The antigen binding domain of the CAR-T cells recognizes CD19 found on the surface of leukemia cancer cells as an antigen.

In addition, there was a report that when CTL019 was administered to patients with acute leukemia, 27 out of 30 patients experienced complete remission, 67% of the patients experienced complete remission for over 2 years, and 78% of the patients survived for two years. Considering that the target patients above were relapsed or refractory patients, these results were very surprising *(*N Engl J Med 2014, 371:1507-1517, October 16, 2014).

Currently, clinical trials for various blood cancers (e.g., lymphoma, myeloma, *etc.)* are in progress with regard to therapeutic methods using various CAR-T cells, and CAR-T, as a pharmaceutical drug that can be used for the treatment of blood cancer, has entered the market.

However, there are several obstacles to using CAR-T cells for the treatment of solid cancer. For example, when CAR-T cells are administered intravenously to a cancer patient, it is difficult for CAR-T cells to migrate to the tumor site; and in a hostile tumor microenvironment, CAR-T cells are functionally inhibited while simultaneously exhibiting limited persistence and proliferation.

Therefore, there is a need to develop CAR-T cells which can treat solid cancer by overcoming these problems.

### [Detailed Description of the Invention]

### [Technical Problem]

An object of the present invention is to provide CAR platforms, CAR-T cells, and CAR expression vectors which have remarkably excellent therapeutic effects in solid cancer compared to the previously known CAR-T cells.

The CAR-T cell therapeutic of the present invention for treating solid cancer comprises: (1) a mutated ITAM introduced into the cytoplasmic signaling domain for excellent *in vivo* persistence and antitumor effects of CAR-T cells; and/or (2) CAR tonic signaling controlled by adding a cleaved cytoplasmic domain of IL7Rα or IL-2Rβ in addition to a CD3ζ signaling domain and a 4-1BB costimulatory domain to a CAR gene so as to induce a cytokine signal in an antigen-dependent manner; and/or (3) a chimeric switch receptor introduced so as to convert the immunosuppressive signal in the hostile tumor microenvironment into an activation signal in CAR-T cells; and/or (4) cytokine IL-21 allowed to release out of CAR-T cells so as to aid the activation of innate immune-related cells and increase the content of less-differentiated memory CAR-T cells.

Due to CAR tonic signaling, CAR-T cells promote anergy, differentiation, exhaustion, and activation-induced CAR-T cell death. In order to increase the therapeutic efficacy of CAR-T cells for treating solid cancer, excellent *in vivo* persistence and anti-tumor effects of CAR-T cells are essential. Therefore, it is necessary to control the CAR tonic signaling system in strategies for solid cancer treatment.

Since the CAR tonic signaling can be controlled by substitution of a cytosolic signaling domain of CAR-T cell, an antigen-dependent cytokine signal was added into CAR-T cell. Additionally, in order to control the CAR tonic signaling that may occur due to uncontrolled excessive ITAM-based signaling within the CD3ζ domain, one mutated ITAM was introduced into the cytoplasmic signaling domain. By controlling CAR tonic signaling, the excellent *in vivo* persistence and anti-tumor effects of CAR-T cells were made to last longer.

In addition, a chimeric switch receptor was introduced, which converts the inhibitory signal of TGF-β *(i.e.,* an immunosuppressive cytokine overexpressed in a hostile tumor microenvironment) into an activation signal in CAR-T cells. In addition, it was prepared such that cytokine IL-21 could be released out of CAR-T cells so as to aid the activation of innate immune-related cells and increase the content of less-differentiated memory CAR-T cells.

The chimeric antigen receptor of the present invention can be used to prepare CAR-T cell therapeutics for various carcinomas by changing the cancer antigen target in the antigen binding domain.

### [Technical Solution]

To solve the problems described above, the present invention provides a polypeptide comprising a chimeric antigen receptor, which comprises an antigen binding domain; a hinge region; a transmembrane domain; a costimulatory domain; and a signaling domain. In particular, the costimulatory domain may comprise a 4-1BB domain or CD28 domain or both a 4-1BB domain and a CD28 domain, in which the signaling domain comprises a CD3ζ domain.

In the present invention, the polypeptide comprises IL-7Rα or a part thereof interposed between the 4-1BB domain and the CD3ζ domain. In particular, a part of IL-7Rα may be a sequence of SEQ ID NO: 15, and a part of IL-2Rβ may be a sequence of SEQ ID NO: 17. A part of the cytoplasmic domain of the IL-7Rα (SEQ ID NO: 15) is a sequence between the 266^{th} and the 328^{th} positions in the entire sequence of IL-7Rα of SEQ ID NO: 14, and a part of the cytoplasmic domain of the IL-2Rβ (SEQ ID NO: 17) is a sequence between the 266^{th} and the 369^{th} positions in the entire sequence of IL-2Rβ of SEQ ID NO: 16.

For example, *in vivo* persistence and anti-tumor effects of CAR-T cells were increased by controlling the CAR tonic signaling by linking a cleaved cytoplasmic domain of IL-7Rα (amino acids at positions from 265 to 328; SEQ ID NO: 15) or cleaved cytoplasmic domain of IL2Rβ (amino acids at positions from 266 to 369; SEQ ID NO: 17) between 4-1BB (SEQ ID NO: 11) as a costimulatory domain and mutant CD3ζ (in which the positions from 91 to 113 of SEQ ID NO: 18 are substituted with either SEQ ID NO: 31 or SEQ ID NO: 32), in a CAR which is specific to IL-13Ra2, based on the known anti-IL13Ra2 CAR-T cells (YYB-103 of SEQ ID NO:22: see PCT International Publication No. WO 2017/023138).

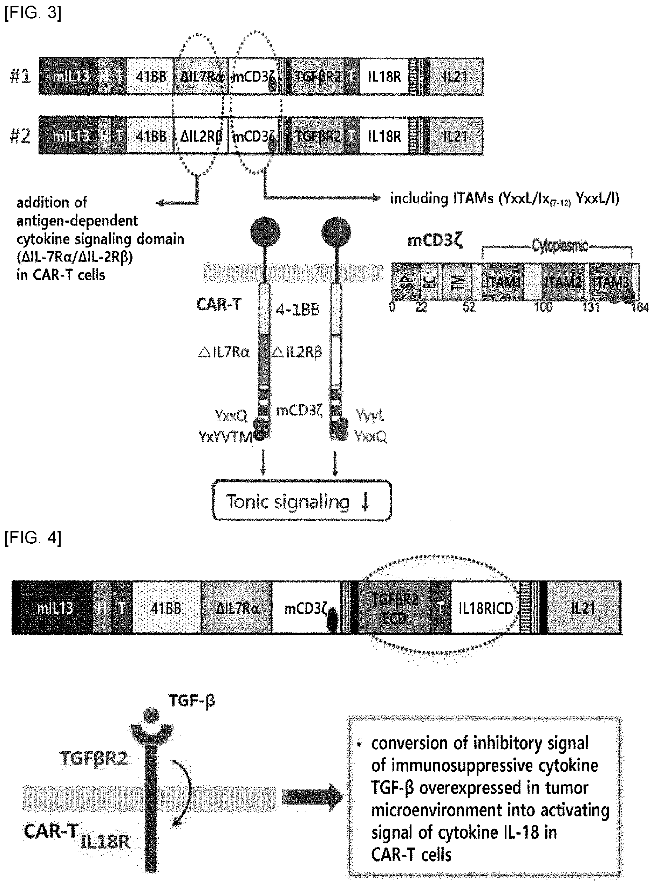

In the present invention, three immunoreceptor tyrosine-based activation motifs (ITAMs) are included within the CD3ζ domain, and in a third ITAM region among these, it is preferred that a first motif, YxxL, and a second motif, YxxL, be substituted (in which x and y each represent any amino acid) (see FIG. 3).

Generally, the ITAM within the CD3ζ domain includes two forms of YxxL (or isoleucine (I)) containing tyrosine (Y), which is spaced apart from leucine (L) or isoleucine (I) by a distance of a two amino acid sequence. Since the YxxL/I forms are usually present to be spaced apart by 6 to 8 amino acids, the ITAM can be represented by the structure of YxxL/Ix₍₆₋₈₎YxxL/I.

In the present invention, three ITAMs (positions 21 to 35; positions 60 to 75; and positions 91 to 105 of SEQ ID NO: 18) are included within the CD3ζ domain, which is represented by SEQ ID NO: 18, in which in the CD3ζ domain, it is preferred that in a third ITAM region, a first motif, YxxL (positions 91 to 94 of SEQ ID NO: 18), be substituted with YxxQ, and a second motif region, YxxLHM (positions 102 to 107 of SEQ ID NO: 18), be substituted with YxYVTM; or in a third ITAM region, a first motif, YxxL (positions 91 to 94 of SEQ ID NO: 18), be substituted with YyyL, and a second motif, YxxL (positions 102 to 105 of SEQ ID NO: 18), be substituted with YxxQ (in which x and y each represent any amino acid).

For example, in the present invention, the region including a third ITAM region of the CD3ζ domain may be mutated to a sequence of SEQ ID NO: 31 or 32.

Specifically, a chimeric antigen receptor (CAR) can be used, in which among the three ITAM motifs present within the CD3ζ domain (see SEQ ID NO: 18) (i.e., a known signaling domain of anti-IL13Ra2 CAR-T cells (YYB-103)), the sequence of a third ITAM motif *(i.e.,* positions 91 to 106 of SEQ ID NO:18) is mutated from "YQGLSTATKDTYDALHMQALPPR" to "YLPQSTATKDTYDYVTMQALPPR" (SEQ ID NO: 31); or among the three ITAM motifs present within the CD3ζ domain (see SEQ ID NO: 18) *(i.e.,* a known signaling domain of anti-IL13Ra2 CAR-T cells (YYB-103)), the sequence of a third ITAM motif (i.e., positions 91 to 105 of SEQ ID NO: 18) is mutated from "YQGLSTATKDTYDALHMQALPPR" to "YLSLSTATKDTYLPQHMQALPPR" (SEQ ID NO:32) (see FIG. 3).

In particular, in the present invention, it is preferred that in a third ITAM region of the CD3ζ domain, a first motif, YxxL (positions 91 to 94 of SEQ ID NO: 18), be substituted with YxxQ, and a second motif, YxxLHM (positions 102 to 106 of SEQ ID NO: 18), be substituted with YxYVTM; or in a third ITAM region of the CD3ζ domain, a first motif, YxxL (positions 91 to 94 of SEQ ID NO: 18), be substituted with YyyL, and a second motif, YxxL (positions 102 to 105 of SEQ ID NO: 18), be substituted with YxxQ (in which x and y each represent any amino acid).

The polypeptide of the present invention may further contain a cytokine. For example, the polypeptide of the present invention may contain IL-21 as a cytokine to be added. In particular, it is preferred that the cytokine be linked to a chimeric antigen receptor by a self-cleaving peptide.

As the self-cleaving peptide, for example, the known 2A peptides of SEQ ID NOS: 40 to 43 may be used. The self-cleaving peptide undergoes a post-translational cleavage, in which the peptide bond between a proline (P) and a glycine (G) in the C-terminus is digested by cleavage. Therefore, the proteins located upstream and downstream of the self-cleaving peptide are expressed independently of each other.

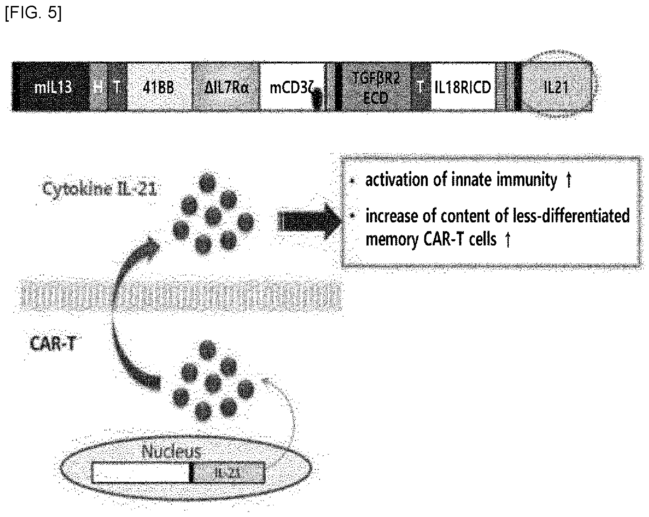

Accordingly, when the polypeptide according to the present invention is expressed in a T cell, the cytokine can be separated from a chimeric antigen receptor (CAR), and the separated cytokine can be released to the outside of the T cell. Innate immune-related cells can be activated by the separated cytokine (e.g., IL-21), and thereby the content of less-differentiated CAR-T cells can be increased (see FIG. 5).

That is, *in vitro* differentiation is prevented due to the IL-21 expression during the process of CAR-T cell preparation, which results in an increase of the content of less-differentiated memory CAR-T cells, thereby making it possible to provide excellent in *vivo* persistence and enhanced anti-tumor effects of CAR-T cells, and which additionally results in activation of the innate immune cells around the solid cancer, thereby making it possible to more effectively treat solid cancer.

The polypeptide of the present invention may further include a TGF-βR2 exodomain, an IL18R transmembrane domain, and an IL18R endodomain, in addition to a chimeric antigen receptor. In particular, it is preferred that an IL18R transmembrane domain be included between the TGF-βR2 exodomain and the IL18R endodomain (see FIG. 4).

In the present invention, it is preferred that the TGF-βR2 exodomain be linked to a chimeric antigen receptor by a self-cleaving peptide. Therefore, when the polypeptide according to the present invention is expressed in T cells, the polypeptide comprising a TGF-βR2 exodomain, an IL18R transmembrane domain, and an IL18R endodomain is separated from a chimeric antigen receptor (CAR). The TGF-βR2 domain of the separated polypeptide is exposed to the outside of T cells, the IL18R transmembrane domain is located in the cell membrane of T cells, and the IL18R endodomain is located in the cytoplasm of T cells.

Accordingly, when TGF-β, which is an immunosuppressive cytokine, is present outside of T cells, TGF-β binds to the TGF-βR2 exodomain, thereby activating the IL18R endodomain. That is, the inhibitory signal of TGF-β *(i.e.,* an immunosuppressive cytokine in a hostile tumor microenvironment) is converted into an activation signal of the cytokine IL-18 in CAR-T cells. Therefore, a polypeptide, which includes the TGF-βR2 exodomain, IL18R transmembrane domain, and IL18R endodomain, becomes a chimeric switch receptor.

IL-18, which is a cytokine, inhibits expression of immunosuppressive materials in T cells and differentiation of regulatory T cells, and enhances immune responses against cancer cells. When the effects of the CAR-T cell therapeutic by IL-18 in advanced solid tumors were examined, it was confirmed that FoxO1 expression in CD4⁺ T cells was decreased by IL-18, and additionally that T-bet expression was increased by IL-18 in CD8⁺ T cells.

Ultimately, IL-18 shows an excellent anticancer effect in advanced solid cancer through the T-bethigh FoxO1low CAR-T, persistent CAR-T cells. In particular, in the present invention, a chimeric switch receptor, which includes a TGF-βR2 exodomain (amino acid positions of 23 to 166; SEQ ID NO: 19), which is a receptor for binding to TGF-β *(i.e.,* an immunosuppressive cytokine around solid cancer); and a transmembrane domain and an endodomain of IL-18R (amino acid positions of 323 to 541; SEQ ID NO: 20), which is a cytokine receptor, was prepared to be expressed in CAR-T cells, thereby changing the immunosuppressive signal into an activation signal in CAR-T cells by making a reverse use of the hostile tumor microenvironment.

As described above, even a polypeptide, which additionally includes a TGF-βR2 exodomain, an IL18R transmembrane domain, and an IL18R endodomain in the chimeric antigen receptor, may further include the cytokine described above *(e.g.,* IL-21).

That is, the cytokine described above *(e.g.,* IL-21) may be linked by a self-cleaving peptide to the IL18R endodomain of the polypeptide, which additionally includes a TGF-βR2 exodomain, an IL18R transmembrane domain, and an IL18R cytoplasmic endodomain in the chimeric antigen receptor.

Accordingly, when the polypeptide according to the present invention is expressed in T cells, the polypeptide which includes the TGF-βR2 exodomain, IL18R transmembrane domain, and IL18R endodomain can be separated from the chimeric antigen receptor (CAR), and additionally, the cytokine can also be independently separated. Therefore, the separated cytokine can be released to the outside of T cells, the TGF-βR2 exodomain is exposed to the outside of the T cells, the IL18R transmembrane domain is located in the cell membrane of T cells, and the IL18R endodomain is located in the cytoplasm of T cells.

The polypeptide according to the present invention may be, for example, a polypeptide represented by any one of SEQ ID NOS: 24 to 30 and 34 to 37.

In addition, the present invention relates to a CAR-T cell in which the CAR-containing polypeptide is expressed as described above.

In addition, the present invention relates to a chimeric antigen receptor (CAR) expression vector, which includes an antigen binding domain; a hinge region; a transmembrane domain; a costimulatory domain; and a cytoplasmic signaling domain. In particular, the CAR expression vector includes a nucleic acid encoding a CAR, in which the nucleic acid encoding a CAR comprises a nucleic acid encoding a 4-1BB domain as the costimulatory domain and a nucleic acid encoding a CD3ζ domain as the signaling domain, and further comprises a nucleic acid encoding IL-7Rα or a part thereof or a nucleic acid encoding IL-2Rβ or a part thereof between the nucleic acid encoding the 4-1BB domain and the nucleic acid encoding the CD3ζ domain. In particular, a part of IL-7Rα may be, for example, a sequence of SEQ ID NO: 15, and a part of IL-2Rβ may be, for example, a sequence of SEQ ID NO: 17.

In the present invention, the nucleic acid encoding the CD3ζ domain may be a nucleic acid encoding a CD3ζ domain, in which in a third ITAM region among the three ITAMs present within the CD3ζ domain, a first motif, YxxL, and a second motif, YxxLHM, are substituted. Preferably, the nucleic acid encoding the CD3ζ domain may be a nucleic acid encoding a CD3ζ domain, in which in a third ITAM region, a first motif, YxxL, is substituted with YxxQ; and a second motif region, YxxLHM, is substituted with YxYVTM; or one in which a first motif, YxxL, is substituted with YyyL, and a second motif region, YxxL, is substituted with YxxQ (in which x and y each represent any amino acid).

In addition, the CAR expression vector of the present invention may further include a nucleic acid encoding cytokine IL-21. In particular, the nucleic acid encoding cytokine IL-21 is linked to a nucleic acid encoding a CAR through a nucleic acid encoding a self-cleaving peptide.

In addition, the CAR expression vector of the present invention may further include a nucleic acid encoding a TGF-βR2 exodomain, a nucleic acid encoding an IL18R transmembrane domain, and a nucleic acid encoding an IL18R endodomain. In particular, the nucleic acid encoding the TGF-βR2 exodomain is linked to a nucleic acid encoding a CAR through a nucleic acid encoding a self-cleaving peptide.

As described above, the CAR expression vector, which includes a nucleic acid encoding a TGF-βR2 exodomain, a nucleic acid encoding an IL18R transmembrane domain, and a nucleic acid encoding an IL18R endodomain, may further include a nucleic acid encoding cytokine IL-21. In particular, the nucleic acid encoding cytokine IL-21 is linked to a nucleic acid encoding an IL18R endodomain through a nucleic acid encoding a self-cleaving peptide.

The present invention relates to a CAR-T cell prepared by introducing the CAR expression vector described above.

In addition, the present invention relates to an anticancer agent containing the CAR-T cell described above. The anticancer agent of the present invention containing the CAR-T cell may further contain a pharmaceutically acceptable additive as necessary.

In the CAR-containing polypeptides according to the present invention, CAR-T cells for various carcinomas may be prepared by changing the cancer antigen target of the antigen binding domain as necessary. For example, the antigen binding domain may be prepared as those which bind to IL13Rα2, an antigen associated with an angiogenesis activity (anti-angiogenesis), and antigens such as EGFRvIII, EphA2, αVβMesothelin, and glypican1. That is, if any ligand or antibody targeting IL-13Ra2, anti-angiogenesis, EGFRvIII, EphA2, aVβglypican1, and mesothelin (see SEQ ID NOS: 1 to 7 and 33) is introduced, these CAR-T cells may be used as an anticancer agent for these targets. Therefore, it is possible to prepare CAR-T cells having an anticancer effect against a specific carcinoma.

In the case of EGFRvIII *(i.e.,* a major tumor antigen expressed in glioblastoma, lung cancer, *etc.),* in order to reduce side effects of CAR-T cells that appear through a non-specific binding, a CAR expression rate and persistence in CAR-T cells were optimized while simultaneously changing the target sequence so as to minimize the binding affinity with EGFR wild-type while maintaining the specificity for EGFRvIII.

While changing positions 52 to 57 of SEQ ID NO: 3 from STGGYN to DPENDE (a CDR2 part of a heavy chain), position 101 of SEQ ID NO: 3 from S to G (a CDR3 part of a heavy chain), and position 229 of SEQ ID NO: 3 from V to G (a CDR3 part of a light chain) in the target sequence (SEQ ID NO: 33), a CAR expression rate and persistence in CAR-T cells were improved using CD8 signal sequence (SEQ ID NOS: 34 and 35).

In order to increase a CAR expression rate and persistence in CAR-T cells targeting aVβ, a *Gaussia princeps* luciferase signal sequence or CD8 signal sequence was used (SEQ ID NOS: 36 and 37).

### [Advantageous Effects]

The CAR-T cells disclosed in the present invention have an excellent expression rate and persistence; therefore, these cells show the effects of persistence and anti-tumor effects in the human body, thereby having an improved therapeutic effect against solid cancer.

### [Brief Description of Drawings]

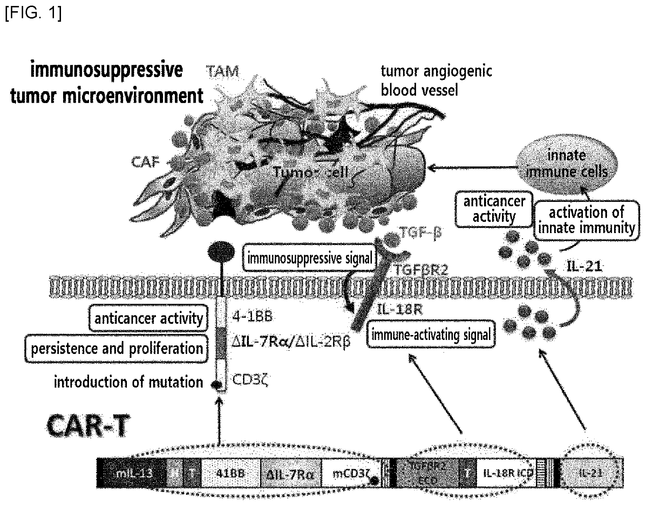

FIG. 1 shows a diagram illustrating a process of effectively treating solid cancer by a CAR-containing polypeptide according to the present invention.

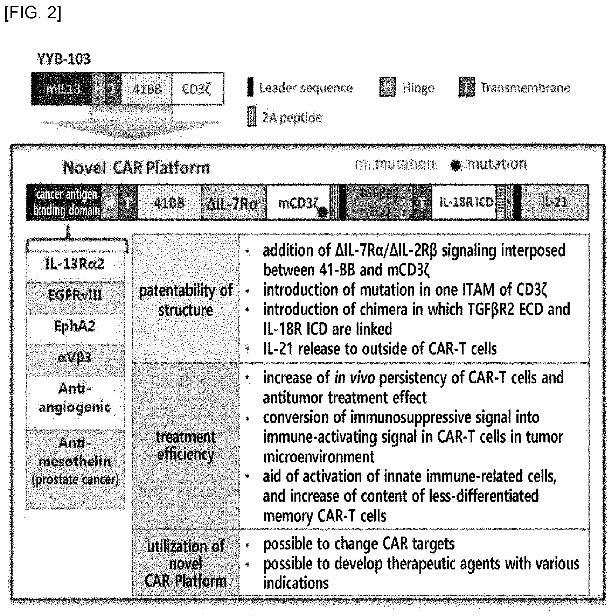

FIG. 2 shows a diagram and a table illustrating an antigen binding domain which can be used for a CAR-containing polypeptide platform according to the present invention.
FIG. 3 shows diagrams illustrating the introduction of a cytokine signaling domain into CAR-T cells so as to control CAR tonic signaling in the CAR-containing polypeptide according to the present invention.
FIG. 4 shows diagrams illustrating the introduction of a chimeric switch receptor so as to convert an immune suppressive signal in a hostile tumor microenvironment into an activation signal in CAR-T cells.
FIG. 5 shows diagrams illustrating the release of cytokine IL-21 to the outside of CAR-T cells so as to aid the activation of innate immune-related cells and to increase the content of less-differentiated memory CAR-T cells.
FIG. 6 shows diagrams illustrating the structures of CAR-containing polypeptides according to the present invention.
FIG. 7 shows diagrams illustrating the self-cleaving peptides used in the CAR-containing polypeptides according to the present invention.
FIG. 8 shows a graph and tables illustrating the growth rate and viability of CAR-T cells, which are transformed with the CAR of the present invention.
FIG. 9 shows graphs and a table illustrating the analysis results of the CAR expression rate of CAR-T cells, which were transformed with the CAR of the present invention, by flow cytometry analysis.
FIG. 10 shows a graph and a table illustrating the analysis results of the chimeric switch receptor expression rate of TGF-βR2 and IL-18R of CAR-T cells, which were transformed with the CAR of the present invention, by flow cytometry analysis.
FIG. 11 shows the results of Day 10 phenotypes of CAR-T cells, which were transformed with the CAR of the present invention, by flow cytometry analysis.
FIG. 12 shows a graph, which illustrates the cytotoxicity of CAR-T cells, which were transformed with the CAR of the present invention, confirmed through LDH assay after 24 hours of co-culture with U87 cells (human brain cancer cell line) and 293FT target cells (which were used as a control group for normal cells); and a table, which illustrates the purity and viability of CAR-T cells used in a CAR-T cytotoxicity test.
FIG. 13 shows graphs illustrating the analysis results of spontaneous toxicity of CAR-T cells, which were transformed with the CAR of the present invention, after 24 hours or 96 hours.
FIGS. 14 to 16 each show graphs and a table illustrating the analysis results of changes in a CAR expression rate of CAR-T cells, which were transformed with the CAR of the present invention, after 24 hours (FIG. 14), 48 hours (FIG. 15), and 96 hours (FIG. 16) of co-culture of the CAR-T cells with U87 cells (human brain cancer cell line) and 293FT target cells (which were used as a control group for normal cells), by flow cytometry analysis.
FIG. 17 shows graphs and a table illustrating the analysis results of changes in the chimeric switch receptor expression rate of TGF-βR2 and IL-18R of CAR-T cells, which were transformed with the CAR of the present invention, after 24 hours of co-culture of the CAR-T cells, which were transformed with the CAR of the present invention, with U87 cells (human brain cancer cell line) and 293FT target cells (which were used as a control group for normal cells), by flow cytometry analysis.
FIG. 18 shows graphs illustrating the comparison results of IFN-γ cytokine production after 24 hours and 48 hours of co-culture of CAR-T cells, which were transformed with the CAR of the present invention, with U87 cells (human brain cancer cell line) and 293FT target cells (which were used as a control group for normal cells).
FIG. 19 shows graphs illustrating the comparison results of IL-21 cytokine production after 24 hours and 48 hours of co-culture of CAR-T cells, which were transformed with the CAR of the present invention, with U87 cells (human brain cancer cell line) and 293FT target cells (which were used as a control group for normal cells).

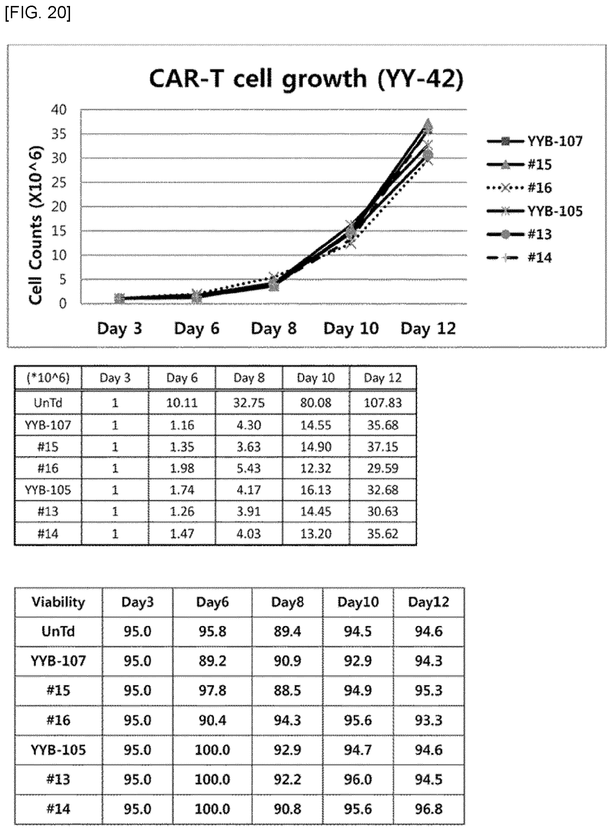

FIG. 20 shows a graph and tables illustrating the growth rate and viability of CAR-T cells, which were transformed with a chimeric antigen receptor (CAR), when the cancer antigen is EGFRvIII, in which the CAR expression rate and persistence in CAR-T cells were optimized, while simultaneously changing the target sequence so as to minimize the binding affinity for EGFR wild-type, where the specificity for EGFRvIII is maintained so as to reduce the side effects of CAR-T cells that appear through non-specific binding (YYB105, #13, #14); and a graph and tables illustrating the growth rate and viability of CAR-T cells, which were transformed with a chimeric antigen receptor (CAR), when the cancer antigen is aVβ, in which the CAR expression rate in CAR-T cells was optimized (YYB107, #15, #16).

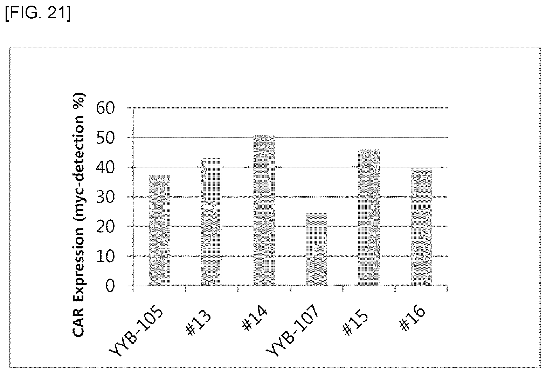

FIG. 21 shows a graph illustrating the CAR expression rate in CAR-T cells, which were transformed with a chimeric antigen receptor (CAR), when the cancer antigen is EGFRvIII, in which the CAR expression rate in CAR-T cells was optimized, while simultaneously changing the target sequence so as to minimize the binding affinity for EGFR wild-type, where the specificity for EGFRvIII is maintained so as to reduce the side effects of CAR-T cells that appear through non-specific binding (YYB105, #13, #14); and a graph illustrating the CAR expression rate in CAR-T cells, which were transformed with a chimeric antigen receptor (CAR), when the cancer antigen is aVβ, in which the CAR expression rate in CAR-T cells was optimized (YYB107, #15, #16).

### [Best Mode for Carrying Out the Invention]

Hereinafter, the present invention will be described in detail through examples. However, it should be noted that the following Examples are only for illustrating the present invention, and the spirit and technical scope of the present invention are not limited in any sense. Further, it should be understood that the present invention is not limited to the precise arrangement and means of the embodiments shown in the drawings, which are cited as reference.

### Example 1: Preparation of novel chimeric antigen receptor (CAR) platform for effective treatment by specifically binding to IL13Rα2 overexpressed in solid cancer cells

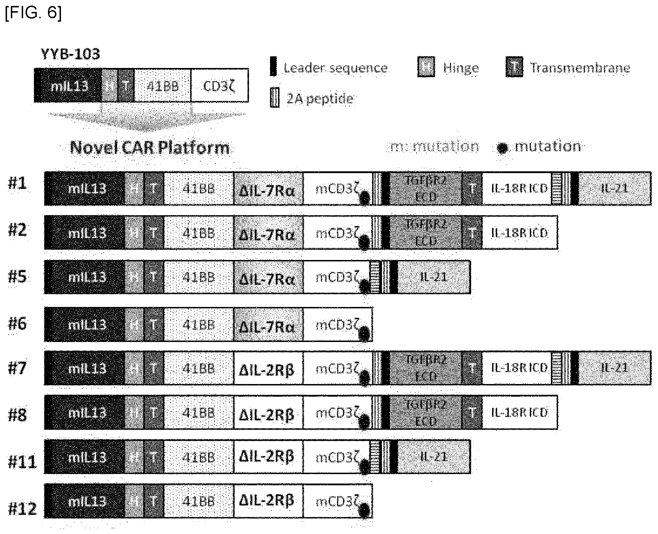

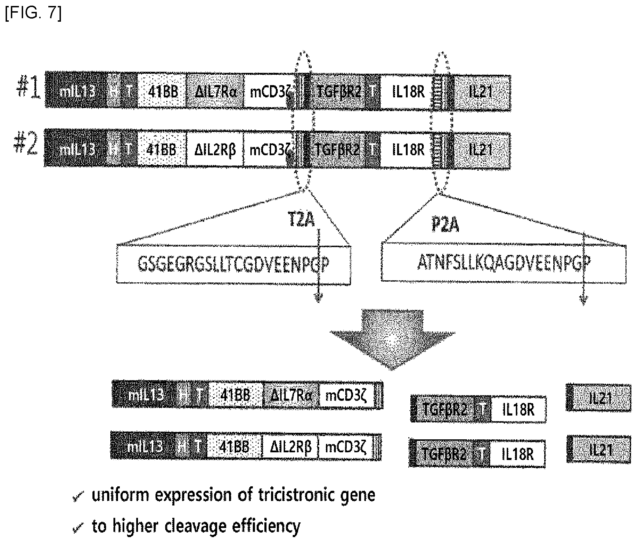

With regard to human IL13 (P35225.1), human CD3 (P20963-1), human CD8A (P01732), human CD28 (P10747), human CD3ζ (P20963), human 41BB (Q07011), IL7RA (P16871), IL2RB (P14784), TGFR2 (P37173), IL18R (Q13478), IL21 (Q9HBE4), IL2 (P60568), T2A, P2A, and human kappa light chain signal sequence (HuVHCAMP), CAR-containing polypeptides were prepared using a chimeric antigen-containing polypeptide (see SEQ ID NOS: 23 to 30 and 34 to 37; and #1, #2, #5, #6, #7, #8, #11, and #12 of FIG. 6) expression vector consisting of codon-optimized synthetic DNA, using scientific literature and publicly available databases (see FIGS. 6 and 7).

Specifically, T cells, in which a CAR-containing polypeptide is expressed, can be prepared by preparing a vector that expresses a CAR-containing polypeptide via conjugation of synthetic DNA to an MFG retrovirus expression vector digested with *Xho*I/*Not*I, followed by transduction of the vector into the T cells.

The completed structure of polypeptide #1 of FIG. 6 includes a Kozak consensus ribosome-binding sequence, a human kappa light chain signal sequence (HuVHCAMP), human IL13.E11K.R64D.S67D.R107K mature protein sequence (see YYB-103 of PCT International Publication No. WO 2017/023138), three glycines (GGG) which are introduced between an antigen binding domain and a hinge region so as to increase the expression of a chimeric antigen receptor by increasing the solubility of a CAR protein (see YYB-103 of PCT International Publication No. WO 2017/023138), a hinge region of human CD8α, a human CD8 transmembrane domain, a costimulatory signal domain of cytoplasmic 4-1BB, a cleaved cytoplasmic domain of cytokine IL-7RA (amino acid positions of 265 to 328; SEQ ID NO: 15), a mutant CD3ζ (positions 91 to 113 of SEQ ID NO: 18 are mutated to a sequence of SEQ ID NO: 31), T2A, a CD8A signal sequence (leader sequence), a chimeric switch receptor which comprises TGF-βR2 exodomain (amino acid positions of 23 to 166; SEQ ID NO: 19) and a transmembrane domain and an endodomain of IL-18R (i.e., a cytokine receptor) (amino acid positions of 323 to 541; SEQ ID NO:20), P2A, a human IL2 signal sequence (leader sequence), an IL-21 (amino acid positions of 23 to 155; SEQ ID NO: 21) sequence, and an *Xho*I/*Not*I cleavage site (see CAR-containing polypeptide #1 of FIG. 6; and FIG. 7).

The completed structure of CAR-containing polypeptide #2 of FIG. 6 includes a Kozak consensus ribosome-binding sequence, a human kappa light chain signal sequence (HuVHCAMP), human IL13.E11K.R64D.S67D.R107K mature protein sequence, three glycines (GGG) which are introduced between an antigen binding domain and a hinge region so as to increase the expression of a chimeric antigen receptor by increasing the solubility of a CAR protein, a hinge region of human CD8α, a human CD8 transmembrane domain, a costimulatory signal domain of cytoplasmic 4-1BB, a cleaved cytoplasmic domain of a cytokine IL-7RA (amino acid positions of 265 to 328; SEQ ID NO: 15), a mutant CD3ζ (positions 91 to 113 of SEQ ID NO: 18 are mutated to a sequence of SEQ ID NO: 31), T2A, a CD8A signal sequence (leader sequence), a chimeric switch receptor which comprises TGF-βR2 exodomain (amino acid positions of 23 to 166; SEQ ID NO: 19) and a transmembrane domain and an endodomain of IL-18R *(i.e.,* a cytokine receptor) (amino acid positions of 323 to 541; SEQ ID NO: 20), and an *Xho*I/*Not*I cleavage site (see CAR-containing polypeptide #2 of FIG. 6; and FIG. 7).

The completed structure of CAR-containing polypeptide #5 of FIG. 6 includes a Kozak consensus ribosome-binding sequence, a human kappa light chain signal sequence (HuVHCAMP), human IL13.E11K.R64D.S67D.R107K mature protein sequence, three glycines (GGG) which are introduced between an antigen binding domain and a hinge region so as to increase the expression of a chimeric antigen receptor by increasing the solubility of a CAR protein, a hinge region of human CD8α, a human CD8 transmembrane domain, a costimulatory signal domain of cytoplasmic 4-1BB, a cleaved cytoplasmic domain of a cytokine IL-7RA (amino acid positions of 265 to 328; SEQ ID NO: 15), a mutant CD3ζ (positions 91 to 113 of SEQ ID NO: 18 are mutated to a sequence of SEQ ID NO: 31), T2A, a human IL2 signal sequence, an IL-21 (amino acid positions of 23 to 155; SEQ ID NO: 21) sequence, and an *Xho*I/*Not*I cleavage site (see CAR-containing polypeptide #5 of FIG. 6; and FIG. 7).

The completed structure of CAR-containing polypeptide #6 of FIG. 6 includes a Kozak consensus ribosome-binding sequence, a human kappa light chain signal sequence (HuVHCAMP), human IL13.E11K.R64D.S67D.R107K mature protein sequence, three glycines (GGG) which are introduced between an antigen binding domain and a hinge region so as to increase the expression of a chimeric antigen receptor by increasing the solubility of a CAR protein, a hinge region of human CD8α, a human CD8 transmembrane domain, a costimulatory signal domain of cytoplasmic 4-1BB, a cytoplasmic domain of a cytokine IL-7RA (amino acid positions of 265 to 328; SEQ ID NO: 15), a mutant CD3ζ (positions 91 to 113 of SEQ ID NO: 18 are mutated to a sequence of SEQ ID NO: 31), and an *Xho*I/*Not*I cleavage site (see CAR-containing polypeptide #6 of FIG. 6; and FIG. 7).

The completed structure of polypeptide CAR-containing #7 of FIG. 6 includes a Kozak consensus ribosome-binding sequence, a human kappa light chain signal sequence (HuVHCAMP), human IL13.E11K.R64D.S67D.R107K mature protein sequence, three glycines (GGG) which are introduced between an antigen binding domain and a hinge region so as to increase the expression of a chimeric antigen receptor by increasing the solubility of a CAR protein, a hinge region of human CD8α, a human CD8 transmembrane domain, a costimulatory signal domain of cytoplasmic 4-1BB, a cleaved cytoplasmic domain of a cytokine IL2Rβ (amino acid positions of 266 to 369; SEQ ID NO: 17), a mutant CD3ζ (positions 91 to 113 of SEQ ID NO: 18 are mutated to a sequence of SEQ ID NO: 32), T2A, a CD8A signal sequence, a chimeric switch receptor which comprises a TGF-βR2 exodomain (amino acid positions of 23 to 166; SEQ ID NO: 19) and a transmembrane domain and an endodomain of IL-18R *(i.e.,* a cytokine receptor) (amino acid positions of 323 to 541; SEQ ID NO: 20), P2A, a human IL2 signal sequence (leader sequence), an IL-21 (amino acid positions of 23 to 155; SEQ ID NO: 21) sequence, and an *Xho*I/*Not*I cleavage site (see #7 of FIG. 6; and FIG. 7).

The completed structure of CAR-containing polypeptide #8 of FIG. 6 includes a Kozak consensus ribosome-binding sequence, a human kappa light chain signal sequence (HuVHCAMP), human IL13.E11K.R64D.S67D.R107K mature protein sequence, three glycines (GGG) which are introduced between an antigen binding domain and a hinge region so as to increase the expression of a chimeric antigen receptor by increasing the solubility of a CAR protein, a hinge region of human CD8α, a human CD8 transmembrane domain, a costimulatory signal domain of cytoplasmic 4-1BB, a cleaved cytoplasmic domain of a cytokine IL2Rβ (amino acid positions of 266 to 369; SEQ ID NO: 19), a mutant CD3ζ (positions 91 to 113 of SEQ ID NO: 18 are mutated to a sequence of SEQ ID NO: 32), T2A, a CD8A signal sequence, a chimeric switch receptor which comprises a TGF-βR2 exodomain (amino acid positions of 23 to 166; SEQ ID NO: 19) and a transmembrane domain and an endodomain of IL-18R *(i.e.,* a cytokine receptor) (amino acid positions of 323 to 541; SEQ ID NO: 20), and an *Xho*I/*Not*I cleavage site (see CAR-containing polypeptide #8 of FIG. 6; and FIG. 7).

The completed structure of CAR-containing polypeptide #11 of FIG. 6 includes a Kozak consensus ribosome-binding sequence, a human kappa light chain signal sequence (HuVHCAMP), human IL13.E11K.R64D.S67D.R107K mature protein sequence, three glycines (GGG) which are introduced between an antigen binding domain and a hinge region so as to increase the expression of a chimeric antigen receptor by increasing the solubility of a CAR protein, a hinge region of human CD8α, a human CD8 transmembrane domain, a costimulatory signal domain of cytoplasmic 4-1BB, a cleaved cytoplasmic domain of a cytokine IL2Rβ (amino acid positions of 266 to 369; SEQ ID NO: 17), a mutant CD3ζ (positions 91 to 113 of SEQ ID NO: 18 are mutated to a sequence of SEQ ID NO: 32), T2A, a human IL2 signal sequence, an IL-21 (amino acid positions of 23 to 155; SEQ ID NO: 21) sequence, and an *Xho*I/*Not*I cleavage site (see CAR-containing polypeptide #11 of FIG. 6; and FIG. 7).

The completed structure of polypeptide CAR-containing #12 of FIG. 6 includes a Kozak consensus ribosome-binding sequence, a human kappa light chain signal sequence (HuVHCAMP), human IL13.E11K.R64D.S67D.R107K mature protein sequence, three glycines (GGG) which are introduced between an antigen binding domain and a hinge region so as to increase the expression of a chimeric antigen receptor by increasing the solubility of a CAR protein, a human hinge region of CD8α, a human CD8 transmembrane domain, a costimulatory signal domain of cytoplasmic 4-1BB, a cleaved cytoplasmic domain of a cytokine IL2Rβ (amino acid positions of 266 to 369; SEQ ID NO: 17), a mutant CD3ζ (positions 91 to 113 of SEQ ID NO: 18 are mutated to a sequence of SEQ ID NO: 32), and an *Xho*I/*Not*I cleavage site (see #12 of FIG. 6; and FIG. 7).

The entire sequences of CAR-containing polypeptides #1, #2, #5, #6, #7, #8, #11, and #12 are shown in SEQ ID NOS: 23 to 30.

The finally prepared CAR gene fragments were conjugated to the MFG retrovirus expression vector digested with *Xho*I/*Not*I (Emtage PC et a/., Clin Cancer Res, 2008, 14:8112-8122). In this Example, in order to compare the activity of chimeric antigen receptors, YYB103 (SEQ ID NO: 22) was further prepared.

### Example 2: Preparation of CAR-T cells transformed with novel chimeric antigen receptors

High-titer CAR-expressing PG13 clones were prepared such that Phoenix-Ampho and Phoenix-Eco cells were transiently transfected with the retroviral expression vector prepared in Example 1, and then, cell-free vector stocks were prepared from the transfected Phoenix-Ampho and Phoenix-Eco cells by transfecting PG13 cells.

For high-titer monoclones, PG13/#1, PG13/#2, PG13/#5, PG13/#6, PG13/#7, PG13/#8, PG13/#11, or PG13/#12 cells were stained using an anti-IL-13 monoclonal antibody (BD Pharmingen), and these single clones were isolated using a flow cytometer. The PG13/#1, PG13/#2, PG13/#5, PG13/#6, PG13/#7, PG13/#8, PG13/#11, or PG13/#12 clones were prepared by the second subcloning according to the limiting dilution method. These subclones stably showed high CAR expression and were selected for the efficient transduction ability in the peripheral blood.

The transduction degree of PG13/#1, PG13/#2, PG13/#5, PG13/#6, PG13/#7, PG13/#8, PG13/#11, or PG13/#12 cells, which were transduced using an anti-IL-13 monoclonal antibody (BD Pharmingen), was analyzed using a flow cytometer. The supernatants of the transduced PG13/#1, PG13/#2, PG13/#5, PG13/#6, PG13/#7, PG13/#8, PG13/#11, or PG13/#12 cells contained retrovirus, and the supernatants were collected for genetic modification of T cells.

The peripheral blood mononuclear cells (PBMCs) were separated using centrifugation by adding the whole blood obtained from a healthy human donor into Ficoll Paque (GE Healthcare). The separated PBMCs were cultured by adding an anti-CD3 monoclonal antibody (eBioscience) at a concentration of 100 ng/mL under the condition of human IL-2 (Novartis) at a concentration of 100 IU/mL to activate the T cell fraction (BL Levine, Cancer Gene Therapy, 2015, 22:79-84). Two to three days after the cultivation, most of the cells were T cells and included natural killer cells at a percentage of 0% to 2%. Two to three days after the activation step, the T cells were subjected to transduction two times over two days using the retroviral supernatant and washed, and then proliferated for four to seven days in a flask. The cells were cultured in a stirring platform device (a WAVE bio-reactor system) for 12 to 28 days. IL-2 was maintained at a concentration of 100 IU/mL. The T cells modified as such were used for an analysis experiment.

### Experimental Example 1: Checking of growth rate and viability of CAR-T cells transformed with novel chimeric antigen receptors

### Experimental Results

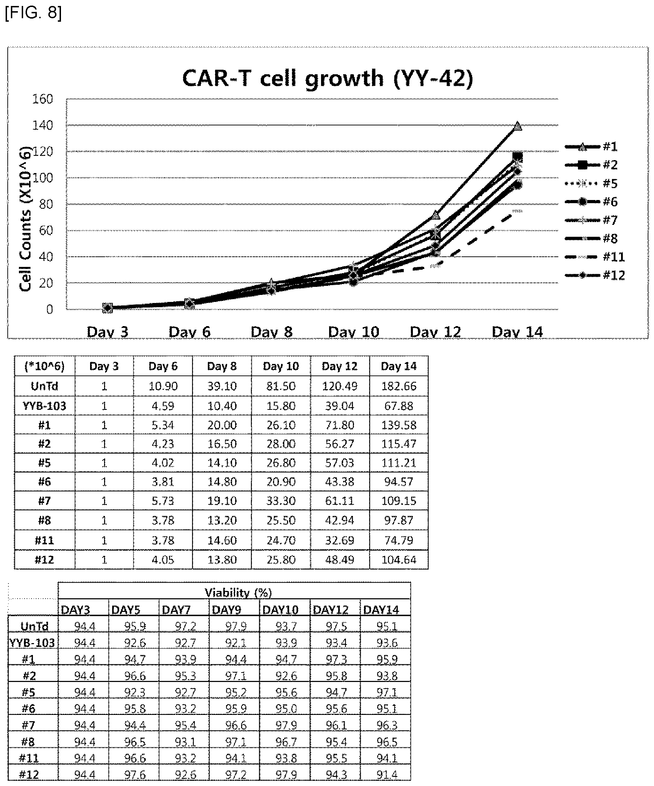

For the T cells prepared in Example 2, the number of cells was counted to confirm the growth rate and viability of CAR-T, and the results are shown in FIG. 8.

The number of cells and growth rate of all of the groups (#1, #2, #5, #6, #7, #8, #11, or #12) were shown to be higher than those of the control group *(i.e.,* YYB103) depending on the day of the week, the cells were shown to grow rapidly from Day 12 of culture, and the viability was also shown to be 90% or higher (see FIG. 8).

### Experimental Example 2: Checking of CAR expression rate on surface of CAR-T cells transformed with novel chimeric antigen receptors

### Experimental Method (flow cytometric analysis)

For flow cytometry (>30,000 events), a BD LSRII device (Becton Dickinson) and BD FACSDiva software (Becton Dickinson) were used. Specifically, the cells were washed once with PBS containing 2% bovine serum albumin before adding a PE-conjugated anti-human IL-13 monoclonal antibody (BD Pharmingen) thereto. After washing, the cells were reacted with each antibody at 4°C for 30 minutes in a state where light was blocked, and washed once, and thereafter, the expression rate of CAR on the surface of transduced T cells was checked.

### Experimental Results

In order to confirm whether the 8 kinds of IL13Rα2-specific, CAR-containing polypeptides prepared in Example 2 (#1, #2, #5, #6, #7, #8, #11, or #12) were expressed on the T cell surface, T cell culture was performed for 28 days according to Example 2, and then flow cytometric analysis was performed according to the experimental method.

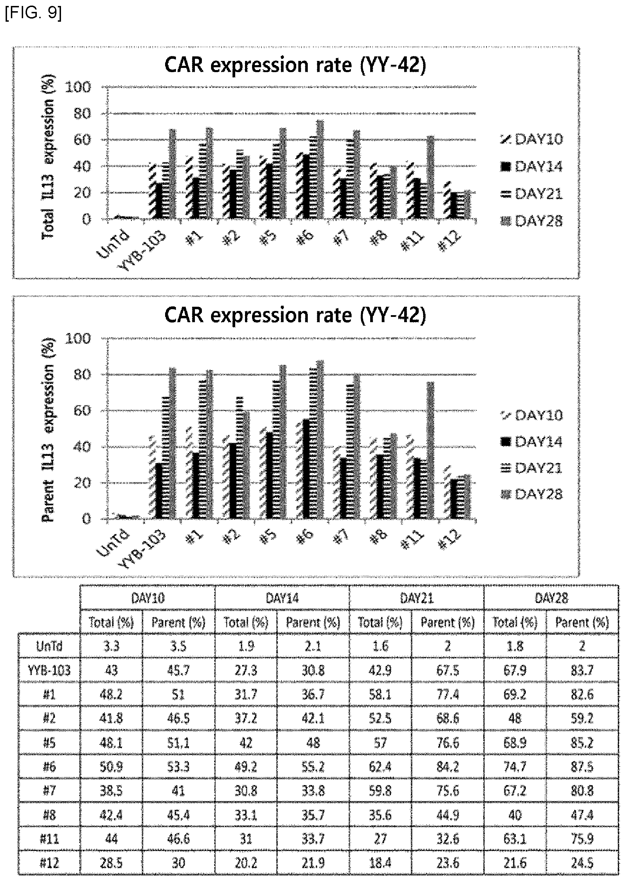

As a result of the analysis, as shown in FIG. 9, the expression rate of the chimeric antigen receptors expressed on the cell surface of live T cells was shown to be in the range of 24.5% to 84.2%, and the expression of IL13Rα2-specific chimeric antigen receptors was stably maintained for 4 weeks, without additional activation or transduction of T cells (see FIG. 9).

### Experimental Example 3: Checking of chimeric switch receptor expression rates of TGF-βR2 and IL-18R onthe cell surface of CAR-T cells transformed with novel chimeric antigen receptors

### Experimental Methods (flow cytometric analysis)

For flow cytometry (>30,000 events), a BD LSRII device (Becton Dickinson) and BD FACSDiva software (Becton Dickinson) were used. Specifically, the cells were washed once with PBS containing 2% bovine serum albumin before adding a human TGF-βR2 fluorescein-conjugated antibody (FAB2411F) (BD Pharmingen) thereto. After washing, the cells were reacted with each antibody at 4°C for 30 minutes in a state where light was blocked and washed once, and thereafter, the chimeric switch receptor expression rates of TGF-βR2 and IL-18R on the cell surface of transduced T cells were checked.

### Experimental Results

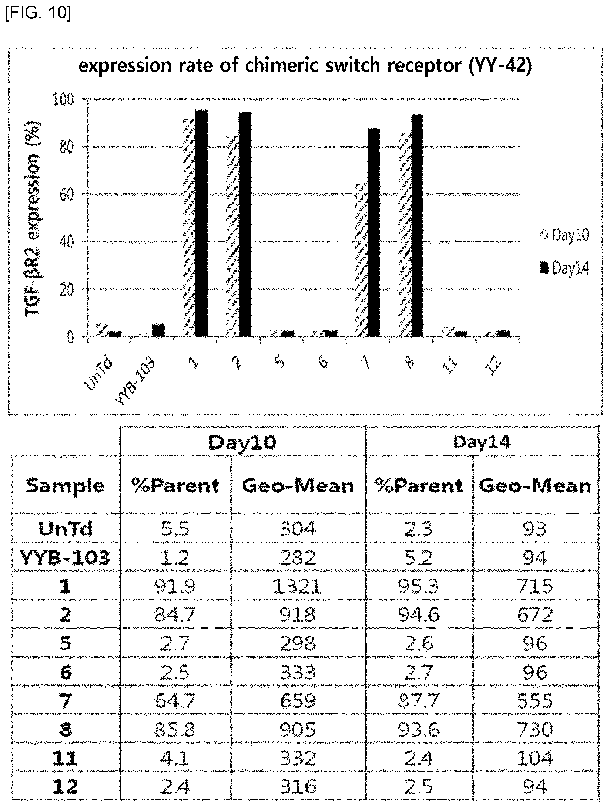

In order to confirm whether the chimeric switch receptors of TGFβR2 and IL-18R were expressed on the cell surface of CAR-T cells prepared in Example 2 (#1, #2, #5, #6, #7, #8, #11, or #12), T cell cultivation was performed for 14 days according to Example 2, and then flow cytometric analysis was performed according to the experimental method. As a result of the analysis, the expression rates of the chimeric switch receptors of TGFβR2 and IL-18R expressed on the cell surface of live T cells were shown to be about 85% (see FIG. 10).

### Experimental Example 4: Checking of phenotypes on the cell surface of CAR-T cells transformed with novel chimeric antigen receptors

### Experimental Methods (flow cytometric analysis)

For flow cytometry (>30,000 events), a BD LSRII device (Becton Dickinson) and a BD FACSDiva software (Becton Dickinson) were used. Specifically, the cells were washed once with PBS containing 2% bovine serum albumin before adding an FITC-conjugated CD45RA Ab (HI100) (Biolegend), a PE-conjugated CCR7Ab (G043H7) (Biolegend), and a PE-Cy7-conjugated CD62L Ab (DREG-56) (Biolegend) thereto. After washing, the cells were reacted with each antibody at 4°C for 30 minutes in a state where light was blocked and washed once, and thereafter, the content of less-differentiated memory CAR-T cells and CCR7⁺CD45RA⁺CD62L⁺ phenotypes on the surface of transduced T cells were checked.

### Experimental Results

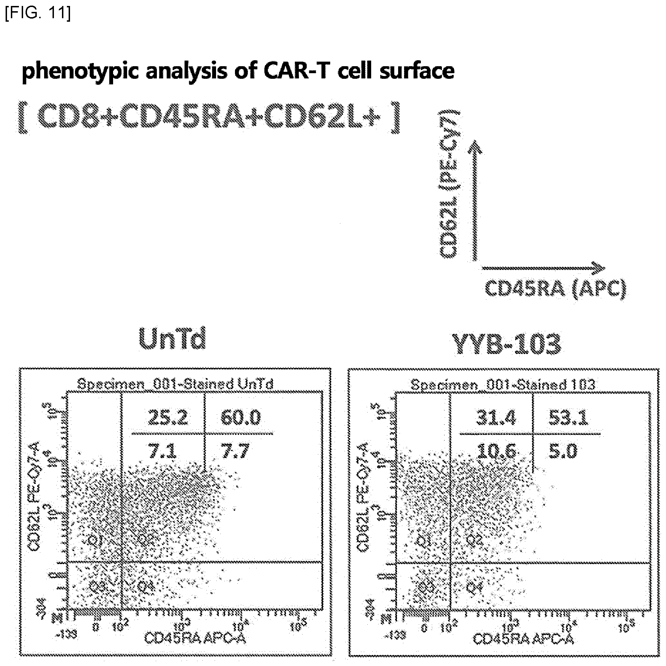

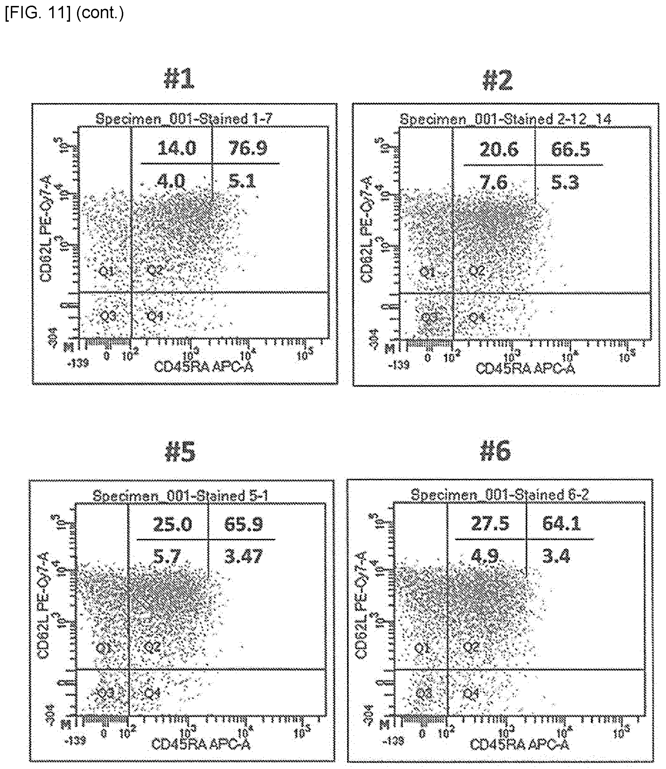

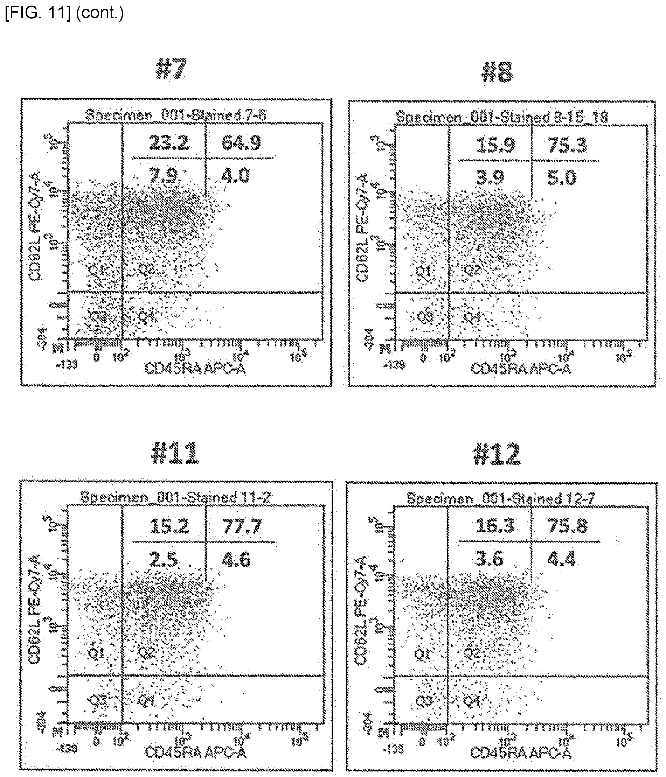

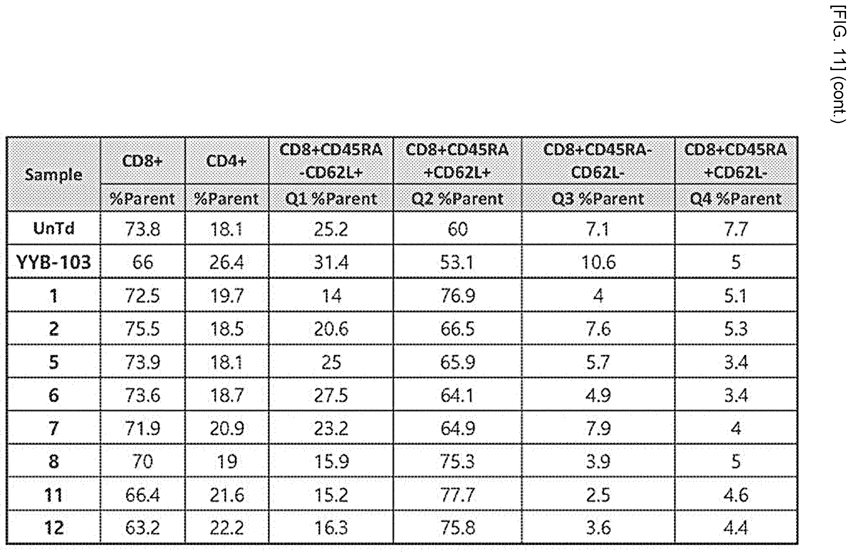

In order to confirm the content of the less-differentiated memory CAR-T cells on the cell surface of CAR-T cells prepared in Example 2 (#1, #2, #5, #6, #7, #8, #11, or #12), T cell cultivation was performed for 10 days according to Example 2, and then flow cytometric analysis was performed according to the experimental method. As a result of the analysis, the content of less-differentiated memory CAR-T cells showing CCR7⁺CD45RA⁺CD62L⁺ phenotypes, which were expressed on live T cells, compared to a non-transduced sample *(i.e.,* a control group) by 5% or higher, and compared to YYB103 *(i.e.,* the transduced sample, CAR-T control group) by 10% or higher, on the surface of all of the groups of CAR-T cells prepared in Example 2 (#1, #2, #5, #6, #7, #8, #11, or #12), was confirmed (see FIG. 11).

### Experimental Example 5: Checking of cytotoxicity of CAR-T cells transformed with novel chimeric antigen receptors against glioma cell line in which IL13Rα2 is overexpressed

### Experimental Methods

In order to measure the cytotoxicity of IL13Rα2-specific CAR-T cells prepared in Example 2 (#1, #2, #5, #6, #7, #8, #11, or #12), cytotoxicity assay was performed using an LDH (Promega) kit. Specifically, CAR-T cells (effector cells) were used 10 days after activation of the cells with an anti-CD3 Ab, and the CAR-T cells (having a CAR expression rate of 20% to 40%) were added to a 6-well plate at a ratio 1:2, in which effector: target was 1×10⁶ cells : 2×10⁶ cells and reacted at 37°C for 24 hours. As the used target cells, U87 cells (expressing IL13Rα2) and 293FT cells (the control group for normal cells) were used.

### Experimental Results

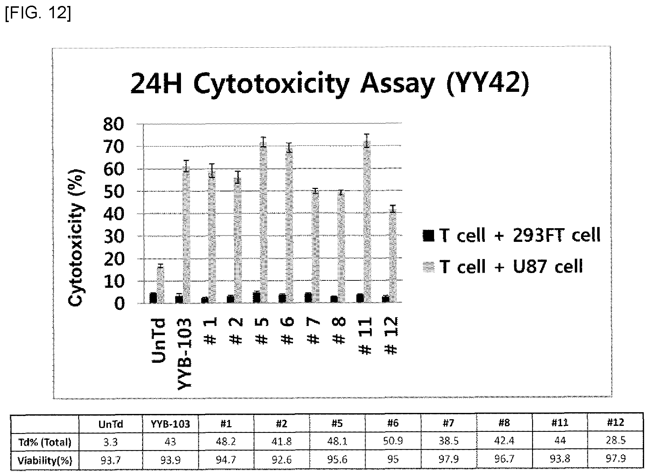

An analysis was performed to examine whether IL13Rα2-specific CAR-T cells prepared in the present invention can effectively kill the target cancer cells (U87). The method used was such that where target cancer cells (U87) and normal cells (293FT) described above were cultured together with each of the activated CAR-T cells and cytotoxicity was compared and analyzed. As shown in FIG. 12, all of the CAR-T cells prepared according to the present invention showed a higher effect by 50% to 70% of inducing the death of the target cancer cell (U87) compared to non-transduced activated T cells. In particular, CAR-T #5, #6, and #11 of the present invention showed high cytotoxicity. In addition, CAR-T cells (#1, #2, #5, and #6) containing IL-7Rα exhibited higher overall cytotoxicity compared to CAR-T cells (#7, #8, #11, and #12) containing IL-2Rβ.

From the experimental result where 293FT cells, which do not express IL13Rα2, were used as the control group for normal cells, as an object to be compared with the target cells, it was confirmed that the CAR-T cells specific to IL13Rα2 showed very weak cytotoxicity (2% to 4%). This result shows that the chimeric antigen receptors used in this experiment bind specifically to IL13Rα2.

Through this Experimental Example, it was found that IL13Rα2-specific chimeric antigen receptor T cells do not show cytotoxicity to a normal cell (293FT) and significantly kill target cancer cells (U87), which express IL13Rα2.

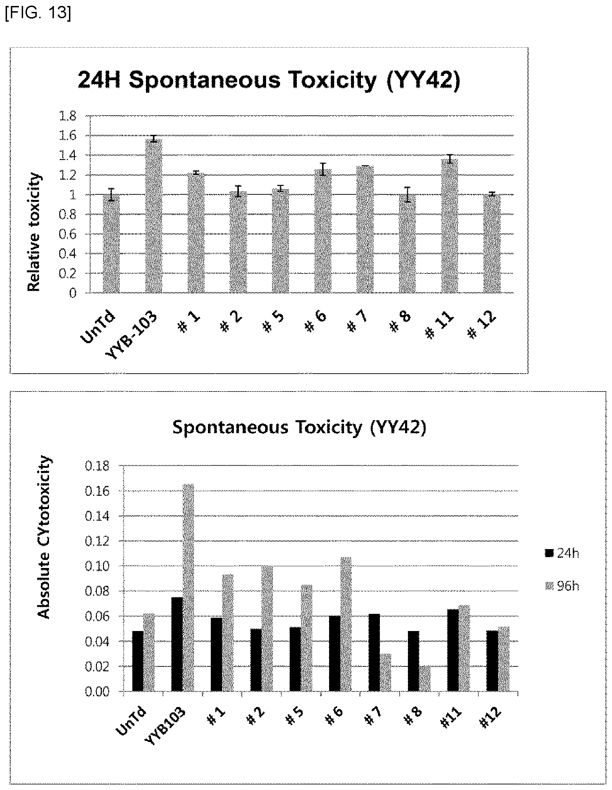

FIG. 13 shows the results in which the cytotoxicity of the CAR-T cells, which were transformed with the chimeric antigen receptor (CAR) of the present invention, was confirmed through LDH assay after 24 hours or 96 hours without co-culture with target cells. Since the spontaneous toxicity of YYB103 is higher than that of other groups, the 8 groups are expected to show higher cytotoxicity compared to YYB103 in the co-culture with U87 cells, and the CAR-T cells (#1, #2, #5, #6, #7, #8, #11, or #12), which were transformed with the chimeric antigen receptor (CAR) of the present invention, are expected to have a comparative advantage in terms of safety and stability.

### Experimental Example 6: Checking of changes in CAR expression rate when CAR-T cells, which were transformed with novel chimeric antigen receptors, are co-cultured with glioma cell line in which IL13Rα2 is overexpressed

### Experimental Methods

In order to check the changes in CAR expression rate when the CAR-T cells prepared in Example 2 (#1, #2, #5, #6, #7, #8, #11, or #12) are co-cultured with glioma cell line in which IL13Rα2 is overexpressed, the CAR-T cells (effector cells) having a CAR expression rate of 20% to 40% were used 10 days after activation of the cells with an anti-CD3 Ab. The CAR-T cells were added to a 6-well plate at a ratio 1:2, in which effector: target was 1×10⁶ cells: 2×10⁶ cells and reacted at 37°C for 24, 48, and 96 hours. To the sample, which was reacted for 96 hours, were added 2×10⁶ cells of the target cells after 48 hours, and a BD LSRII device (Becton Dickinson) and BD FACSDiva software (Becton Dickinson) were used for flow cytometry (>30,000 events). Specifically, the cells were washed once with PBS containing 2% bovine serum albumin before adding a PE-conjugated anti-human IL-13 monoclonal antibody (BD Pharmingen) thereto. After washing, the cells were reacted with each antibody at 4°C for 30 minutes in a state where light was blocked and washed once, and thereafter, the changes in the CAR expression rate were checked.

### Experimental Results

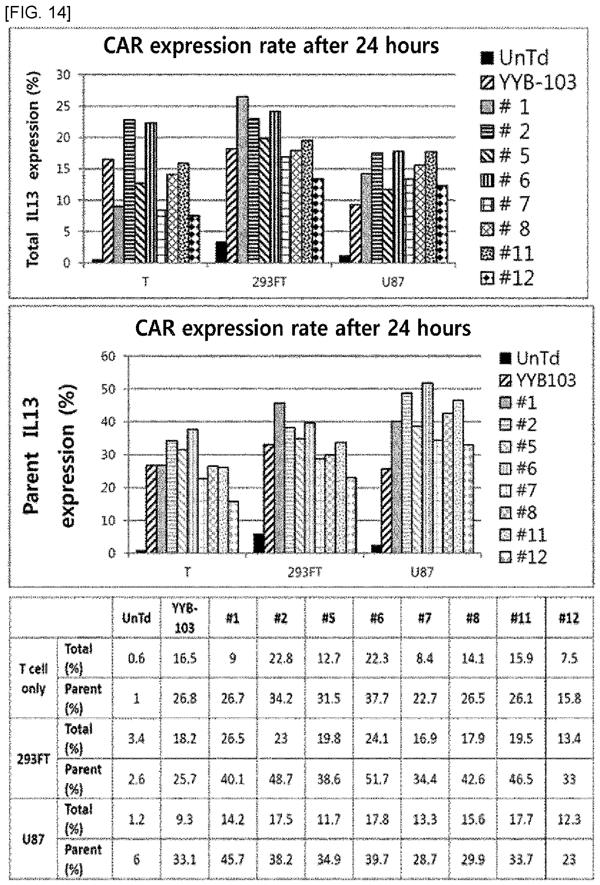

In order to confirm the changes in the CAR expression rate when the 8 kinds of IL13Rα2-specific CAR-T cells prepared in Example 2 (#1, #2, #5, #6, #7, #8, #11, or #12) are co-cultured with glioma cell line in which IL13Rα2 is overexpressed, the T cells were cultured for 10 days, and then flow cytometric analysis was performed according to the experimental method according to Example 2. As a result of the analysis, as shown in FIG. 14, the CAR-T cells which were co-cultured with U87 cells for 24 hours showed a smaller change in CAR expression in all of the 8 groups compared to that of YYB103 (FIG. 14).

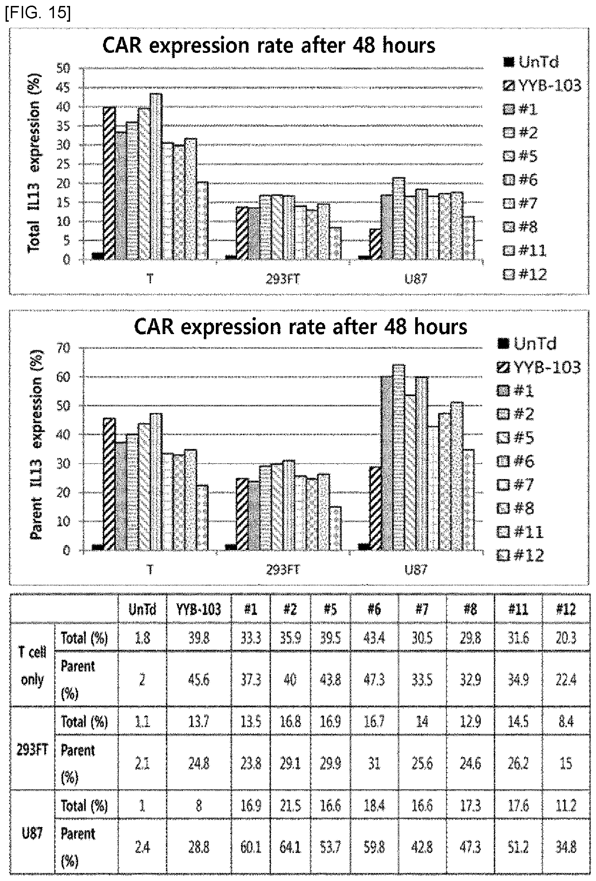

In co-culture with U87 cells for 48 hours, the change in CAR expression was shown to be smaller than that of YYB103 in all of the 8 groups (FIG. 15).

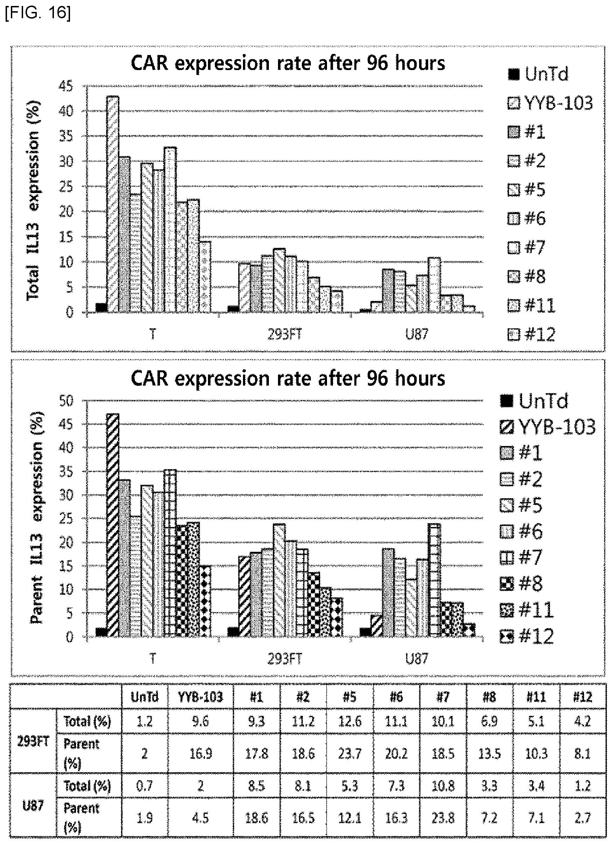

In co-culture with U87 cells for 96 hours, the change in CAR expression was shown to be smaller than that of YYB103 in 5 groups (i.e., #1, #2, #5, #6, and #7) (FIG. 16). The CAR expression of YYB103 after co-culture with U87 cells for 96 hours was shown to be insignificant (FIG. 16).

The changes in CAR expression during the co-culture with glioma cell line, in which IL13Rα2 is overexpressed, the changes in the CAR expression rate without co-culture with target cells or during the co-culture with 293FT cells (which were used as the control group for normal cells) can confirm that the chimeric antigen receptor (CAR) according to the present invention is expected to show excellent *in vivo* persistence and anti-tumor effects of CAR-T cells in a hostile tumor microenvironment, while simultaneously reducing toxicity.

### Experimental Example 7: Checking of changes in chimeric switch receptor expression rate of TGFβR2 and IL-18R when CAR-T cells which were transformed with novel chimeric antigen receptors are co-cultured with glioma cell line, in which IL13Rα2 is overexpressed

### Experimental Methods

In order to check the changes in the chimeric switch receptor expression rate of TGFβR2 and IL-18R when CAR-T cells prepared in Example 2 (#1, #2, #5, #6, #7, #8, #11, or #12) are co-cultured with glioma cell line, in which IL13Rα2 is overexpressed, the CAR-T cells (effector cells) having a CAR expression rate of 20% to 40% were used 10 days after activation of the cells with an anti-CD3 Ab. The CAR-T cells were added to a 6-well plate at a ratio 1:2, in which effector: target was 1×10⁶ cells: 2×10⁶ cells and reacted at 37°C for 24, 48, and 96 hours. To the sample, which was reacted for 96 hours, were added 2×10⁶ cells of the target cells after 48 hours, and a BD LSRII device (Becton Dickinson) and BD FACSDiva software (Becton Dickinson) were used for flow cytometry (>30,000 events). Specifically, the cells were washed once with PBS containing 2% bovine serum albumin before adding an anti-human TGF-βR2 fluorescein-conjugated antibody (FAB2411F) (BD Pharmingen) thereto. After washing, the cells were reacted with each antibody at 4°C for 30 minutes in a state where light was blocked and washed once, and thereafter, the changes in the chimeric switch receptor expression rate of TGFβR2 and IL-18R were checked.

### Experimental Results

In order to confirm the changes in the chimeric switch receptor expression rate of TGFβR2 and IL-18R when the 8 kinds of IL13Rα2-specific CAR prepared in Example 2 (#1, #2, #5, #6, #7, #8, #11, or #12) are co-cultured with glioma cell line in which IL13Rα2 is overexpressed, the T cells were cultured for 10 days according to Example 2, and then flow cytometric analysis was performed according to the experimental method.

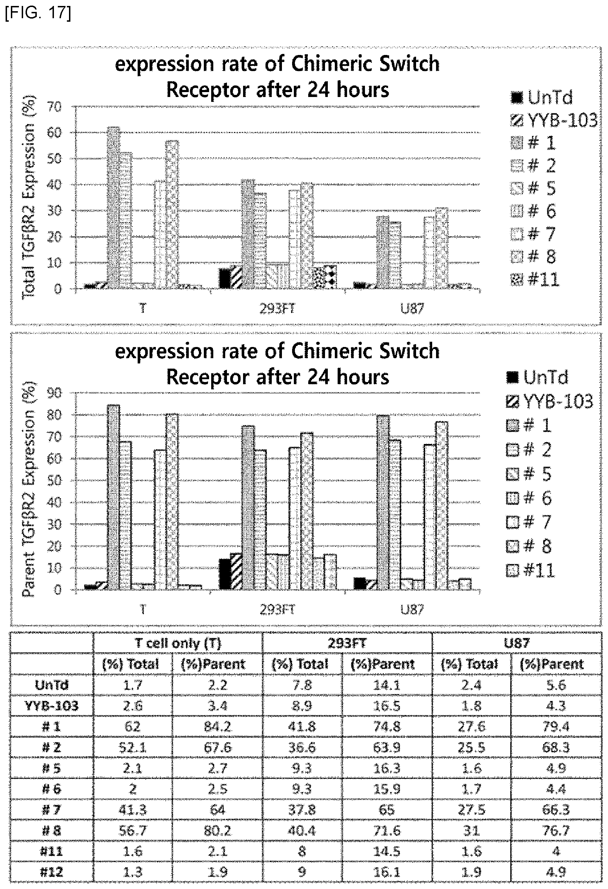

As a result of the analysis, as shown in FIG. 17, the expression of the chimeric switch receptors of TGFβR2 and IL-18R in the 4 groups (#1, #2, #7, and #8) after 24 hours of co-culture showed a tendency of a decrease in the groups co-cultured with glioma cell line, U87 cells compared to those cultured with CAR-T cells only, but the average expression rate was 30% (FIG. 17). The expression of the chimeric switch receptors of TGFβR2 and IL-18R in the 4 groups (#1, #2, #7, and #8) after 48 hours of co-culture showed a tendency of a decrease in the groups co-cultured with U87 cells compared to those cultured with CAR-T cells only, but the average expression rate was 25%. The expression of the chimeric switch receptors of TGFβR2 and IL-18R in the 4 groups (#1, #2, #7, and #8) after 72 hours of co-culture showed a tendency of a decrease in the groups co-cultured with U87 cells compared to those cultured with CAR-T cells only, but the average expression rate was 15%. Although the changes in the expression rate were greater compared to one without co-culture with target cells or the co-culture with 293FT cells (which were used as the control group for normal cells), by the confirmation of the results of the chimeric switch receptor expression rate of TGFβR2 and IL-18R against glioma cell line in which IL13Rα2 is overexpressed, the chimeric antigen receptor (CAR) according to the present invention is expected to show excellent *in vivo* persistence and anti-tumor effects of CAR-T cells in a hostile tumor microenvironment.

### Experimental Example 8: Checking of cytokine (IFN-v) production by T cells, which were transformed with novel chimeric antigen receptors, against target cells

### Experimental Methods

In order to confirm the cytokine (IFN-y) production after 24 hours and 48 hours of co-culture between the CAR-T cells prepared in Example 2 (#1, #2, #5, #6, #7, #8, #11, or #12) and glioma cell line (U87 cells) and 293FT target cells (which were used as a control group for normal cells), the CAR-T cells (effector cells) in which the CAR expression rate is in the range of 20% to 40% were used 10 days after activation of the cells with an anti-CD3 Ab. The CAR-T cells were added to a 6-well plate at a ratio in which effector: target was 1×10⁶ cells: 2×10⁶ cells, 6 mL of a culture medium was added per well, and the cells reacted at 37°C for 24 hours. 100 µL of the supernatant was collected and transferred to a 1.5 mL tube, the cells were cultured further up to 48 hours, and 100 µL of the supernatant was collected from the culture in the same manner.

The IFN-γ analysis experiment was performed as follows according to the manufacturer's instructions of the ELISA analyzer (R&D systems). 3 mL of the calibrator diluent RD6-21 was added to an IFN-γ standard bottle and mixed in a shaker for 15 minutes, dispensed in an amount of 1 mL per 1.5 mL tube to prepare two "standard 1"s. 500 µL was taken from 1 mL of the "standard 1" dispensed and was subjected to serial dilution to prepare up to "standard 7". In order to prepare blanks, 500 µL of the culture medium and 500 µL of the calibrator diluent RD6-21 were mixed.

In order to dilute the samples by 1/20, 190 µL of the calibrator diluent RD6-21 was added into each of new 1.5 mL tubes as many as the number of samples. The assay samples were each prepared to a total volume of 200 µL by collecting 10 µL of the supernatant of each sample. To prepare a wash buffer, 500 mL of distilled water and 20 mL of wash buffer concentrate were mixed well in a 500 mL storage bottle.

Assay diluent RD1-51 (blue dye) in an amount of 100 µL each was added to the IFN-γ microplate for each well of "standard 1" to "standard 7", blank, and samples. After adding 100 µL each of the blank, standards, and samples prepared above, a plate sealer was attached thereto and reacted at room temperature for 2 hours. After the reaction, the reaction solution was discarded, and 400 µL of the prepared wash buffer was added thereto, and the wells were washed 4 times. 200 µL of IFN-γ conjugate was added to each well, a plate sealer was attached thereto, and the reaction was performed at room temperature for 2 hours. The reaction solution was discarded, and the wells were washed 4 times using 400 µL of the wash buffer. After mixing the color reagent A: B in a 1:1 ratio, the mixture was added in an amount of 200 µL per well, a plate sealer was attached thereto, the light was blocked with foil, and the reaction was performed at room temperature for 30 minutes. After the reaction, 50 µL of the stop solution was added to each well and measured at 450 nm within 30 minutes.

### Experimental Results

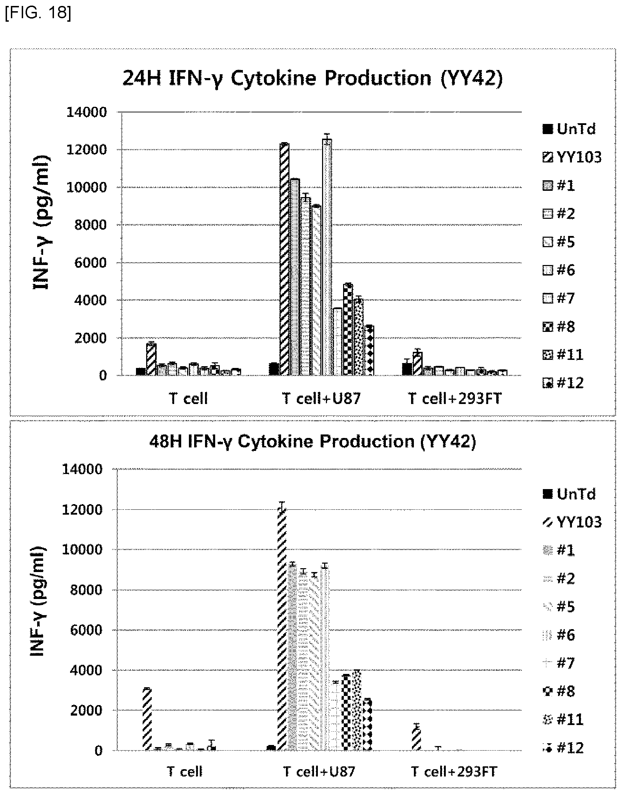

YYB103 was shown to secrete a relatively high amount of IFN-γ compared to other samples. When CAR-T cells were cultured in the presence of U87 cells, CAR-T (#1, #2, #5, and #6) containing an IL-7Rα signaling domain were shown to contain more IFN-γ compared to CAR-T (#7, #8, #11, and #12) containing an IL-2Rβ signaling domain. Results between samples after 24 hours and 48 hours of incubation showed similar patterns (FIG. 18).

As shown in FIG. 12, when the cytotoxicity was confirmed through LDH assay after 24 hours of co-culture between the CAR-T (which were transformed with the chimeric antigen receptor (CAR) of the present invention), U87 cells (human glioma cell line), and 293FT target cells (which are used as a control group for normal cells), the 8 groups showed similar abilities of killing target cells. However, considering that the production of IFN-γ was small and thus target cells could be killed without adverse effects caused by cytokines (cytokine release syndrome, CRS), in terms of safety, the effect of the CAR-T cell therapeutic is expected to have a fewer side effects while having excellent therapeutic effects.

### Experimental Example 9: Checking of cytokine (IL-21) production by CAR-T cells, which were transformed with novel chimeric antigen receptors

### Experimental Methods

In order to confirm the cytokine (IL-21) production after 24 hours and 48 hours of co-culture between the CAR-T cells prepared in Example 2 (#1, #2, #5, #6, #7, #8, #11, or #12) and glioma U87 cells and 293FT target cells (which were used as a control group for normal cells), the CAR-T cells (effector cells) in which the CAR expression rate is in the range of 20% to 40% were used 10 days after activation of the cells with an anti-CD3 Ab. The CAR-T cells were added to a 6-well plate at a ratio in which effector: target was 1×10⁶ cells : 2×10⁶ cells, 6 mL of a culture medium was added per well, and the cells were reacted at 37°C for 24 hours. Then, 100 µL of the supernatant was collected and transferred to a 1.5 mL tube, the cells were cultured further up to 48 hours, and 100 µL of the supernatant was collected from the culture in the same manner.

The IL-21 analysis experiment was performed as follows according to the manufacturer's instructions of the ELISA analyzer (Invitrogen). 100 µL of a capture antibody was added to each well of a 96-well ELISA plate and reacted at 4°C overnight. After the reaction, the wells were washed 3 times using 250 µL of wash buffer. ELISA/ELISASPOT diluent was added in an amount of 200 µL per well and reacted at room temperature for one hour.

After the reaction, 100 µL each of the prepared blank, cytokine IL-21 standard, and samples were added thereto, and ELISA/ELISASPOT diluent was added in an amount of 100 µL per well, and a plate sealer was attached thereto and the reaction was performed at room temperature for two hours. After the reaction, the wells were washed 3 times using 250 µL of wash buffer. 100 µL of a detection antibody was added to each well, and a plate sealer was attached thereto and the reaction was performed at room temperature for one hour. After the reaction, the wells were washed 3 times using 250 µL of wash buffer. 100 µL of Avidin-HRP was added to each well, a plate sealer was attached thereto, and the reaction was performed at room temperature for 30 minutes. After the reaction, the wells were washed 5 times using 250 µL of wash buffer. 100 µL of 1× TMB solution was added to each well, a plate sealer was attached thereto, and the reaction was performed at room temperature for 30 minutes. After the reaction, 50 µL of the stop solution was added to each well and measured at 450 nm within 30 minutes.

### Experimental Results

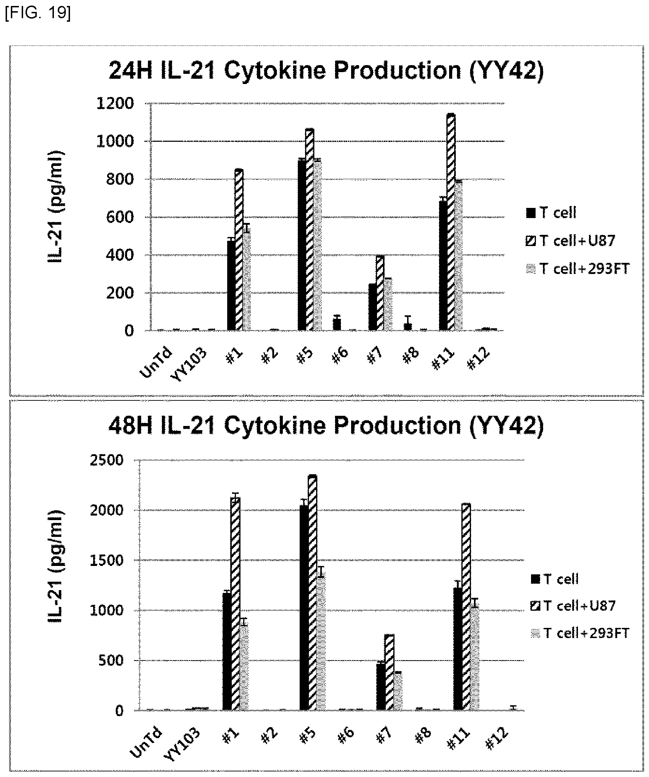

It was confirmed that IL-21 was secreted in IL-21 gene-containing CAR-T cells (#1, #5, #7, and #11). When the CAR-T cells were co-cultured with U87 cells, a greater amount of IL-21 was secreted, and considering that the overall experimental results at 24 hours and 48 hours were similar, no significant difference according to the incubation time was observed. As the result of the experiment at 48 hours showed a higher IL-21 concentration than that at 24 hours, the secretion of IL-21 appeared to proceed steadily (FIG. 19).

The secretion of IL-21 in an environment of cancer cells is speculated to increase the ability to kill cancer cells by assisting the activation of innate immune-related cells.

### Example 3: Preparation of cancer antigen EGFRvIII targeting CAR vector and aVβ targeting CAR vector

In the case of EGFRvIII, which is a major antigen for tumors such as glioblastoma and lung cancer, in order to reduce the side effects of CAR-T cells through non-specific binding while minimizing the binding affinity for EGFR wild-type in which the specificity for EGFRvIII is maintained, in SEQ ID NO: 33, positions 52 to 57 of SEQ ID NO: 3 were first changed from STGGYN to DPENDE (a CDR2 part of a heavy chain), position 101 of SEQ ID NO: 3 was changed from S to G (a CDR3 part of a heavy chain), and position 229 of SEQ ID NO: 3 was changed from V to G (a CDR3 part of a light chain).

Human CD3 (P20963-1), human CD8A (P01732), human 4-1BB (Q07011), human CD3Z (P20963), a *Gaussia princeps* luciferase, and human kappa light chain signal sequence (HuVHCAMP) were optimized using scientific literature and publicly available databases, and thereby chimeric antigen receptor-containing polypeptides consisting of codon-optimized synthetic DNA (#13, #14, #15, and #16 of SEQ ID NOS: 34 to 37) were prepared.
The completed structure of CAR-containing polypeptide #13 includes a Kozak consensus ribosome-binding sequence, a CD8A signal sequence, an antigen binding domain which binds to EGFRvIII of SEQ ID NO: 3, three glycines (GGG) which are introduced between an antigen binding domain and a hinge region so as to increase the expression of a chimeric antigen receptor by increasing the solubility of a CAR protein, a hinge region of human CD8A, a human CD8 transmembrane domain, a cytoplasmic 4-1BB costimulatory signal domain, a CD3ζ cytoplasmic domain (SEQ ID NO: 18), and an *Xho*I/*Not*I cleavage site.

The finally prepared CAR gene fragment was conjugated to the MFG retrovirus expression vector digested with *Xho*I/*Not*I (Emtage PC et al., Clin Cancer Res, 2008, 14:8112-8122). In this Example, in order to compare the activity of chimeric antigen receptors, YYB105 (SEQ ID NO: 39; see PCT International Publication No. WO 2017/023138) was additionally prepared.

The completed structure of CAR-containing polypeptide #14 includes a Kozak consensus ribosome-binding sequence, a CD8A signal sequence, an antigen binding domain which binds to EGFRvIII of SEQ ID NO: 32, three glycines (GGG) which are introduced between an antigen binding domain and a hinge region so as to increase the expression of a chimeric antigen receptor by increasing the solubility of a CAR protein, a hinge region of human CD8A, a human CD8 transmembrane domain, a cytoplasmic 4-1BB costimulatory signal domain, a CD3ζ cytoplasmic domain (SEQ ID NO: 18), and an *Xho*I/*Not*I cleavage site. The finally prepared CAR gene fragment was conjugated to the MFG retrovirus expression vector digested with *Xho*I/*Not*I (Emtage PC et al., Clin Cancer Res, 2008, 14:8112-8122).

In the case of aVβ anti-angiogenic, the CAR expression rate and persistence in CAR-T cells targeting these were optimized. In the case of aVβ, the CAR expression rate and persistence in CAR-T cells targeting these were optimized using *Gaussia princeps* luciferase signal sequence or CD8 signal sequence (SEQ ID NO: 36 and SEQ ID NO: 37).

The completed structure of CAR-containing polypeptide #15 includes a Kozak consensus ribosome-binding sequence, a *Gaussia princeps* luciferase signal sequence, SEQ ID NO: 5 (an antigen binding domain which binds to αVβ), three glycines (GGG) which are introduced between an antigen binding domain and a hinge region so as to increase the expression of a chimeric antigen receptor by increasing the solubility of a CAR protein, a hinge region of human CD8A, a human CD8 transmembrane domain, a cytoplasmic 4-1BB costimulatory signal domain, a CD3ζ cytoplasmic domain (SEQ ID NO: 18), and an *Xho*I/*Not*I cleavage site. The finally prepared CAR gene fragment was conjugated to the MFG retrovirus expression vector digested with *Xho*I/*Not*I (Emtage PC et al., Clin Cancer Res, 2008, 14:8112-8122). In this Example, in order to compare the activity of chimeric antigen receptors, YYB107 (SEQ ID NO: 38; see PCT International Publication No. WO 2017/023138) was additionally prepared.

The completed structure of CAR-containing polypeptide #16 includes a Kozak consensus ribosome-binding sequence, a *Gaussia princeps* luciferase signal sequence, SEQ ID NO: 5 (an antigen binding domain which binds to αVβ), three glycines (GGG) which are introduced between an antigen binding domain and a hinge region so as to increase the expression of a chimeric antigen receptor by increasing the solubility of a CAR protein, a hinge region of human CD8A, a human CD8 transmembrane domain, a cytoplasmic 4-1BB costimulatory signal domain, a CD3ζ cytoplasmic domain (SEQ ID NO: 18), and an *Xho*I/*Not*I cleavage site. The finally prepared CAR gene fragment was conjugated to the MFG retrovirus expression vector digested with *Xho*I/*Not*I (Emtage PC et al., Clin Cancer Res, 2008, 14:8112-8122).

### Example 4: Preparation of CAR-T cells transformed with chimeric antigen receptors

High-titer CAR-expressing PG13 clones were prepared such that Phoenix-Ampho and Phoenix-Eco cells were transiently transfected with the retroviral expression vector prepared in Example 1, and then, cell-free vector stocks were prepared from the transfected Phoenix-Ampho and Phoenix-Eco cells by transfecting PG13 cells. For high-titer monoclones, PG13/#13, PG13/#14, PG13/#15, and PG13/#16 cells were stained using an anti-myc Ab (BD Pharmingen), and these monoclones were isolated using a flow cytometer. The transduction degree of PG13/#13, PG13/#14, PG13/#15, and PG13/#16 cells, which were transduced using an anti-myc Ab, was analyzed using a flow cytometer. The supernatants of the transduced PG13/#13, PG13/#14, PG13/#15, and PG13/#16 cells contained retrovirus, and the supernatants were collected for genetic modification of T cells. The peripheral blood mononuclear cells (PBMCs) were separated using centrifugation by adding the whole blood obtained from a healthy human donor into Ficoll Paque (GE Healthcare). The separated PBMCs were cultured by adding an anti-CD3 monoclonal antibody (eBioscience) at a concentration of 100 ng/mL under the condition of human IL-2 (Novartis) at a concentration of 100 IU/mL to activate the T cell fraction (BL Levine, Cancer Gene Therapy, 2015, 22:79-84). Two to three days after the cultivation, most of the cells were T cells and included natural killer cells at a percentage of 0% to 2%. Two to three days after the activation step, the T cells were subjected to transduction two times over two days using the retroviral supernatant and washed, and then proliferated for 14 days in a flask. IL-2 was maintained at a concentration of 100 IU/mL. The T cells modified as such were used for an analysis experiment.

### Experimental Example 1: Checking of growth rate and viability of CAR-T cells transformed with chimeric antigen receptors

### Experimental Results

For the T cells prepared in Example 4 above, the number of cells was counted to confirm the growth rate and viability rate of CAR-T, and the results are shown in FIG. 20. The number of cells and growth rate of all of the groups (#13, #14, #15, and #16) were shown to be similar to those of the control group *(i.e.,* YYB105) depending on the day of the week, the cells were shown to grow rapidly from Day 12 of culture, and the viability was also shown to be 90% or higher (see FIG. 20).

### Experimental Example 2: Checking of CAR expression rate on the cell surface of CAR-T cells transformed with chimeric antigen receptors

### Experimental Methods (flow cytometric analysis)

For flow cytometry (>30,000 events), a BD LSRII device (Becton Dickinson) and BD FACSDiva software (Becton Dickinson) were used. Specifically, the cells were washed once with PBS containing 2% bovine serum albumin before adding a PE-conjugated anti-myc antibody (BD Pharmingen) thereto. After washing, the cells were reacted with each antibody at 4°C for 30 minutes in a state where light was blocked and washed once, and thereafter, the expression rate of CAR on the surface of transduced T cells was checked.

### Experimental Results

In order to confirm whether the 4 kinds of CAR prepared in Example 2 (#13, #14, #15, and #16) were expressed on the T cell surface, T cell cultivation was performed for 14 days according to Example 4, and then flow cytometric analysis was performed according to the experimental method.

As a result of the analysis, as shown in FIG. 21, the expression rate of the chimeric antigen receptors expressed on the surface of live T cells was shown to increase in #13 (43.0%) and #14 (50.8%) compared to YYB105 (37.4%) *(i.e.,* a control group) (FIG. 21); and was shown to increase in #15 (45.9%) and #16 (40.0%) compared to YYB107 (24.6%) *(i.e.,* a control group) (FIG. 21).

### [Industrial Applicability]

The present invention relates to rapidly developing a CAR-T cell in the field of cancer treatment. The CAR-T cell according to the present invention has a remarkably excellent expression rate and persistence, and thus has an improved therapeutic effect for solid cancer, *etc.,* and can be effectively used in the field of customized cancer treatment.

### [SEQUENCE LISTING]

SEQ ID NO: 1 {antigen binding wild type IL13 domain binding to IL13Ra2}
   Length: 112
   Type: ligand protein
   Organism: human
   Sequence: 
GPVPPSTALRELIEELVNITQNQKAPLCNGSMVWSINLTAG
MYCAALESLINVSGCSAIEKTQRMLSGFCPHKVSAGQFSSL
HVRDTKIEVAQFVKDLLLHLKKLFREGQFN
SEQ ID NO: 2 {antigen binding domain capable of binding to an antigen associated with an angiogenic activity}
   Length: 92
   Type: ligand protein
   Organism: human
   Sequence: 
E V V A A T P T S L L I S W R H P H F P T R Y Y R I T Y G E T G G N S P V Q E F T
V L Q P P S T A T I S G L K P G V D Y T I T V Y A V V E R N G R E L N T P P I S I N Y
R T H H H H H H
SEQ ID NO: 3 {antigen binding domain binding to EGFRvIII}
   Length: 252
   Type: scFv protein
   Organism: human
   Sequence: 
Q V Q L Q E S G G G L V K P G G S L K L S C A A S G F T F S K F G M S W V R Q T
P D K R L E W V A S I S T G G Y N T F Y S D N V K G R F T I S R D N A K N T L Y L
Q M S S L K S E D T A M Y Y C A R G Y S S T S F A M D Y W G Q G T M V T V S S G
S T S G S G K P G S G E G S D I Q M T Q S P S S L S A S V G D R V T I T C M T S T
D I D D D M N W Y Q Q K P G K T P K L L I Y E G N T L R P G V P S R F S G S G S G
T D F I F T I S S L Q P E D I A T Y Y C L Q S F N V P L T F G G G T K V E I K E Q K L
I S E E D L
SEQ ID NO: 4 {antigen binding domain binding to EphA2}
   Length: 141
   Type: ligand protein
   Organism: human
   Sequence: 
D R H T V F W N S S N P K F R N E D Y T I H V Q L N D Y V D I I C P H Y E D H S V
A D A A M E Q Y I L Y L V E H E E Y Q L C Q P Q S K D Q V R W Q C N R P S A K H
G P E K L S E K F Q R F T A F A L A K E F K A G H S Y Y Y I S K P I H Q H E D R C L
R L K V T V S G E Q K L I S E E D L
SEQ ID NO: 5 {antigen binding domain binding to αVβ}
   Length: 104
   Type: ligand protein
   Organism: human
   Sequence: 
V S D V P R D L E V V A A T P T S L L I S W D A P A V T V R Y Y R I T Y G E T G G
N S P V Q E F T V P G S K S T A T I S G L K P G V D Y T I T V Y A V T P R G D W N
E G S K P I S I N Y R T E Q K L I S E E D L
SEQ ID NO: 6 {antigen binding domain binding to glypican1}
   Length: 418
   Type: ligand protein
   Organism: human
   Sequence: 
T S P C D N F D C Q N G A Q C I V R I N E P I C Q C L P G Y Q G E K C E K L V S V
N F I N K E S Y L Q I P S A K V R P Q T N I T L Q I A T D E D S G I L L Y K G D K D H
I A V E L Y R G R V R A S Y D T G S H P A S A I Y S V E T I N D G N F H I V E L L A
L D Q S L S L S V D G G N P K I I T N L S K Q S T L N F D S P L Y V G G M P G K S
N V A S L R Q A P G Q N G T S F H G C I R N L Y I N S E L Q D F Q K V P M Q T G I
L P G C E P C H K K V C A H G T C Q P S S Q A G F T C E C Q E G W M G P L C D
Q R T N D P C L G N K C V H G T C L P I N A F S Y S C K C L E G H G G V L C D E E
E D L F N P C Q A I K C K H G K C R L S G L G Q P Y C E C S S G Y T G D S C D R
E I S C R G E R I R D Y Y Q K Q Q G Y A A C Q T T K K V S R L E C R G G C A G G
Q C C G P L R S K R R K Y S F E C T D G S S F V D E V E K V V K C G C T R C V S
E Q K L I S E E D L
SEQ ID NO: 7 {antigen binding domain binding to mesothelin}
   Length: 262
   Type: scFv protein
   Organism: human
   Sequence: 
QVQLQESGPGLVKPSETLSLTCTVSGGSVSSGSYYWSWIRQPPGKGLEWIGYIYYSG
STNYNPSLKSRVTISVDTSKNQFSLKLSSVTAADTAVYYCAREGKNGAFDIWGQGTMV
TVSSGSTSGSGKPGSGEGSQVQLQQSGPGLVTPSQTLSLTCAISGDSVSSNSATWN
WIRQSPSRGLEWLGRTYYRSKWYNDYAVSVKSRMSINPDTSKNQFSLQLNSVTP
EDTAVYYCARGMMTYYYGMDV WGQGTTVTVSSGILGS
SEQ ID NO: 8 {hinge region sequence -1}
   Length: 47
   Type: protein
   Organism: human
   Sequence: 
K P T T T P A P R P P T P A P T I A S Q P L S L R P E A C R P A A G G A V H T R G
L D F A C D
SEQ ID NO: 9 {hinge region sequence -2}
   Length: 45
   Type: protein
   Organism: human
   Sequence: 
KPTTTPAPRPPTPAPTIASQPLSLRPEAARPAAGGAVHTRG
LDFA
SEQ ID NO: 10 {transmembrane domain sequence-1}
   Length: 21
   Type: protein
   Organism: human
   Sequence:
   IYIWAPLAGTCGVLLLSLVIT
SEQ ID NO: 11 {transmembrane domain sequence-2}
   Length: 23
   Type: protein
   Organism: human
   Sequence:
   LAYLLDGILFIYGVILTALFLRV
SEQ ID NO: 12{4-1BB}
   Length: 42
   Type: protein
   Organism: human
   Sequence: 
K R G R K K L L Y I F K Q P F M R P V Q T T Q E E D G C S C R F P E E E E G G C E
L
SEQ ID NO: 13 {Wild type CD28}
   Length: 41
   Type: protein
   Organism: human
   Sequence: 
R S K R S R L L H S D Y M N M T P R R P G P T R K H Y Q P Y A P P R D F A A Y R
S
SEQ ID NO: 14 {IL-7Rα}
   Length: 459
   Type: protein
   Organism: human
   Sequence:
SEQ ID NO: 15 {part of IL-7Rα}
   Length: 64
   Type: protein
   Organism: human
   Sequence: 
KKRIKPIVWPSLPDHKKTLEHLCKKPRKNLNVSFNPESFLDCQIHRVDDIQARDEVEGF
LQDTF
SEQ ID NO: 16 {IL-2Rβ}
   Length: 551
   Type: protein
   Organism: human
   Sequence: 
MAAPALSWRLPLLILLLPLATSWASAAVNGTSQFTCFYNSRANISCVWSQDGALQDTS
CQVHAWPDRRRWNQTCELLPVSQASWACNLILGAPDSQKLTTVDIVTLRVLCREGVR
WRVMAIQDFKPFENLRLMAPISLQVVHVETHRCNISWEISQASHYFERHLEFEARTLSP
GHTWEEAPLLTLKQKQEWICLETLTPDTQYEFQVRVKPLQGEFTTWSPWSQPLAFRT
KPAALGKDTIPWLGHLLVGLSGAFGFIILVYLLINCRNTGPWLKKVLKCNTPDPSKFFSQ
LSSEHGGDVQKWLSSPFPSSSFSPGGLAPEISPLEVLERDKVTQLLLQQDKVPEPASL
SSNHSLTSCFTNQGYFFFHLPDALEIEACQVYFTYDPYSEEDPDEGVAGAPTGSSPQP
LQPLSGEDDAYCTFPSRDDLLLFSPSLLGGPSPPSTAPGGSGAGEERMPPSLQERVP
RDWDPQPLGPPTPGVPDLVDFQPPPELVLREAGEEVPDAGPREGVSFPWSRPPGQG
EFRALNARLPLNTD AYLSLQELQGQDPTHLV
SEQ ID NO: 17 {part of IL-2Rβ}
   Length: 104
   Type: protein
   Organism: human
   Sequence: 
NCRNTGPWLKKVLKCNTPDPSKFFSQLSSEHGGDVQKWLSSPFPSSSFSPGGLAPEI
SPLEVLERDKVTQLLLQQDKVPEPASLSSNHSLTSCFTNQGYFFFHL
SEQ ID NO: 18 {CD3ζ}
   Length: 113
   Type: protein
   Organism: human
   Sequence: 
R V K F S R S A D A P A Y Q Q G Q N Q L Y N E L N L G R R E E Y D V L D K R R G
R D P E M G G K P Q R R K N P Q E G L Y N E L Q K D K M A E A Y S E I G M K G E
R R R G K G H D G L Y Q G L S T A T K D T Y D A L H M Q A L P P R
SEQ ID NO: 19 {TGF-βR2 exodomain}
   Length: 137
   Type: protein
   Organism: human
   Sequence: 
TIPPHVQKSVNNDMIVTDNNGAVKFPQLCKFCDVRFSTCDNQKSCMSNCSITSICEKP
QEVCVAVWRKNDENITLETVCHDPKLPYHDFILEDAASPKCIMKEKKKPGETFFMCSC
SSDECNDNIIFSEEYNTSNPD
SEQ ID NO: 20 {transmembrane domain and endodomain of IL-18R}
   Length: 219
   Type: protein
   Organism: human
   Sequence: 
PGHVFTRGMIIAVLILVAVVCLVTCVIYRVDLVLFYRHLTRRDETLTDGKTYDAFVSYLK
ECRPENGEEHTFAVEILPRVLEKHFGYKLCIFERDVVPGGAVVDEIHSLIEKSRRLIIVLS
KSYMSNEVRYELESGLHEALVERKIKIILIEFTPVTDFTFL
PQSLKLLKSHRVLKWKADKSLSYNSRFWKNLLYLMPAKTVKPGRDEPEVLPV LSES
SEQ ID NO: 21 {IL-21}
   Length: 133
   Type: protein
   Organism: human
   Sequence:
SEQ ID NO: 22 {YYB 103}
   Length: 359
   Type: protein
   Organism: human
   Sequence: 
M G W S C I I L F L V A T A T G V H S G P V P P S T A L R K L I E E L V N I T Q N Q
K A P L C N G S M V W S I N L T A G M Y C A A L E S L I N V S G C S A I E K T Q D
M L D G F C P H K V S A G Q F S S L H V R D T K I E V A Q F V K D L L L H L K K L
F K E G Q F N G G G P R K P T T T P A P R P P T P A P T I A S Q P L S L R P E A C
R P A A G G A V H T R G L D F A C D I Y I W A P L A G T C G V L L L S L V I T K R G
R K K L L Y I F K Q P F M R P V Q T T Q E E D G C S C R F P E E E E G G C E L R V
K F S R S A D A P A Y Q Q G Q N Q L Y N E L N L G R R E E Y D V L D K R R G R D
P E M G G K P Q R R K N P Q E G L Y N E L Q K D K M A E A Y S E I G M K G E R R
R G K G H D G L Y Q G L S T A T K D T Y D A L H M Q A L P P R
SEQ ID NO: 23 {#1}
   Length: 998
   Type: protein
   Organism: human
   Sequence: 
MGWSCIILFLVATATGVHSGPVPPSTALRKLIEELVNITQNQKAPLCNGSMVWSINLTA
GMYCAALESLINVSGCSAIEKTQDMLDGFCPHKVSAGQFSSLHVRDTKIEVAQFVKDLL
LHLKKLFKEGQFNGGGPRKPTTTPAPRPPTPAPTIASQPLSLRPEACRPAAGGAVHTR
GLDFACDIYIWAPLAGTCGVLLLSLVITKRGRKKLLYIFKQPFMRPVQTTQEEDGCSCR
FPEEEEGGCELKKRIKPIVWPSLPDHKKTLEHLCKKPRKNLNVSFNPESFLDCQIHRVD
DIQARDEVEGFLQDTFRVKFSRSADAPAYQQGQNQLYNELNLGRREEYDVLDKRRGR
DPEMGGKPQRRKNPQEGLYNELQKDKMAEAYSEIGMKGERRRGKGHDGLYLPQSTA
TKDTYDYVTMQALPPREGRGSLLTCGDVEENPGPMALPVTALLLPLALLLHAARPTIPP
HVQKSVNNDMIVTDNNGAVKFPQLCKFCDVRFSTCDNQKSCMSNCSITSICEKPQEV
CVAVWRKNDENITLETVCHDPKLPYHDFILEDAASPKCIMKEKKKPGETFFMCSCSSD
ECNDNIIFSEEYNTSNPDPGHVFTRGMIIAVLILVAVVCLVTVCVIYRVDLVLFYRHLTRR
DETLTDGKTYDAFVSYLKECRPENGEEHTFAVEILPRVLEKHFGYKLCIFERDVVPGGA
VVDEIHSLIEKSRRLIIVLSKSYMSNEVRYELESGLHEALVERKIKIILIEFTPVTDFTFLPQ
SLKLLKSHRVLKWKADKSLSYNSRFWKNLLYLMPAKTVKPGRDEPEVLPVLSESRRKR
SGSGATNFSLLKQAGDVEENPGPMYRMQLLSCIALSLALVTNSQGQDRHMIRMRQLID
IVDQLKNYVNDLVPEFLPAPEDVETNCEWSAFSCFQKAQLKSANTGNNERIINVSIKKL
KRKPPSTNAGRRQKHRLTCPSCDSYEKKPPKEFLERFKSLLQKMIHQHLSSRTHGSE
DS
SEQ ID NO: 24 {#2}
   Length: 818
   Type: protein
   Organism: human
   Sequence: 
MGWSCIILFLVATATGVHSGPVPPSTALRKLIEELVNITQNQKAPLCNGSMVWSINLTA
GMYCAALESLINVSGCSAIEKTQDMLDGFCPHKVSAGQFSSLHVRDTKIEVAQFVKDLL
LHLKKLFKEGQFNGGGPRKPTTTPAPRPPTPAPTIASQPLSLRPEACRPAAGGAVHTR
GLDFACDIYIWAPLAGTCGVLLLSLVITKRGRKKLLYIFKQPFMRPVQTTQEEDGCSCR
FPEEEEGGCELKKRIKPIVWPSLPDHKKTLEHLCKKPRKNLNVSFNPESFLDCQIHRVD
DIQARDEVEGFLQDTFRVKFSRSADAPAYQQGQNQLYNELNLGRREEYDVLDKRRGR
DPEMGGKPQRRKNPQEGLYNELQKDKMAEAYSEIGMKGERRRGKGHDGLYLPQSTA
TKDTYDYVTMQALPPREGRGSLLTCGDVEENPGPMALPVTALLLPLALLLHAARPTIPP
HVQKSVNNDMIVTDNNGAVKFPQLCKFCDVRFSTCDNQKSCMSNCSITSICEKPQEV
CVAVWRKNDENITLETVCHDPKLPYHDFILEDAASPKCIMKEKKKPGETFFMCSCSSD
ECNDNIIFSEEYNTSNPDPGHVFTRGMIIAVLILVAVVCLVTVCVIYRVDLVLFYRHLTRR
DETLTDGKTYDAFVSYLKECRPENGEEHTFAVEILPRVLEKHFGYKLCIFERDVVPGGA
VVDEIHSLIEKSRRLIIVLSKSYMSNEVRYELESGLHEALVERKIKIILIEFTPVTDFTFLPQ
SLKLLKSHRVLKWKADKSLSYNSRFWKNLLYLMPAKT VKPGRDEPEVLPVLSES
SEQ ID NO: 25 {#5}
   Length: 594
   Type: protein
   Organism: human
   Sequence: 
MGWSCIILFLVATATGVHSGPVPPSTALRKLIEELVNITQNQKAPLCNGSMVWSINLTA
GMYCAALESLINVSGCSAIEKTQDMLDGFCPHKVSAGQFSSLHVRDTKIEVAQFVKDLL
LHLKKLFKEGQFNGGGPRKPTTTPAPRPPTPAPTIASQPLSLRPEACRPAAGGAVHTR
GLDFACDIYIWAPLAGTCGVLLLSLVITKRGRKKLLYIFKQPFMRPVQTTQEEDGCSCR
FPEEEEGGCELKKRIKPIVWPSLPDHKKTLEHLCKKPRKNLNVSFNPESFLDCQIHRVD
DIQARDEVEGFLQDTFRVKFSRSADAPAYQQGQNQLYNELNLGRREEYDVLDKRRGR
DPEMGGKPQRRKNPQEGLYNELQKDKMAEAYSEIGMKGERRRGKGHDGLYLPQSTA
TKDTYDYVTMQALPPREGRGSLLTCGDVEENPGPMYRMQLLSCIALSLALVTNSQGQ
DRHMIRMRQLIDIVDQLKNYVNDLVPEFLPAPEDVETNCEWSAFSCFQKAQLKSANTG
NNERIINVSIKKLKRKPPSTNAGRRQKHRLTCPSCDSYEKKPPKEFLERFKSLLQKMIH
QH LSSRTHGSEDS
SEQ ID NO: 26 {#6}
   Length: 423
   Type: protein
   Organism: human
   Sequence: 
MGWSCIILFLVATATGVHSGPVPPSTALRKLIEELVNITQNQKAPLCNGSMVWSINLTA
GMYCAALESLINVSGCSAIEKTQDMLDGFCPHKVSAGQFSSLHVRDTKIEVAQFVKDLL
LHLKKLFKEGQFNGGGPRKPTTTPAPRPPTPAPTIASQPLSLRPEACRPAAGGAVHTR
GLDFACDIYIWAPLAGTCGVLLLSLVITKRGRKKLLYIFKQPFMRPVQTTQEEDGCSCR
FPEEEEGGCELKKRIKPIVWPSLPDHKKTLEHLCKKPRKNLNVSFNPESFLDCQIHRVD
DIQARDEVEGFLQDTFRVKFSRSADAPAYQQGQNQLYNELNLGRREEYDVLDKRRGR
DPEMGGKPQRRKNPQEGLYNELQKDKMAEAYSEIGMKGERRRGKGHDGLYLPQSTA
TKDTYDYVTMQALPPR
SEQ ID NO: 27 {#7}
   Length: 1038
   Type: protein
   Organism: human
   Sequence:
   KMIHQHLSSRTHGSEDS
SEQ ID NO: 28 {#8}
   Length: 858
   Type: protein
   Organism: human
   Sequence: 
MGWSCIILFLVATATGVHSGPVPPSTALRKLIEELVNITQNQKAPLCNGSMVWSINLTA
GMYCAALESLINVSGCSAIEKTQDMLDGFCPHKVSAGQFSSLHVRDTKIEVAQFVKDLL
LHLKKLFKEGQFNGGGPRKPTTTPAPRPPTPAPTIASQPLSLRPEACRPAAGGAVHTR
GLDFACDIYIWAPLAGTCGVLLLSLVITKRGRKKLLYIFKQPFMRPVQTTQEEDGCSCR
FPEEEEGGCELNCRNTGPWLKKVLKCNTPDPSKFFSQLSSEHGGDVQKWLSSPFPS
SSFSPGGLAPEISPLEVLERDKVTQLLLQQDKVPEPASLSSNHSLTSCFTNQGYFFFHL
 
RVKFSRSADAPAYQQGQNQLYNELNLGRREEYDVLDKRRGRDPEMGGKPQRRKNP
QEGLYNELQKDKMAEAYSEIGMKGERRRGKGHDGLYLSLSTATKDTYLPQHMQALPP
REGRGSLLTCGDVEENPGPMALPVTALLLPLALLLHAARPTIPPHVQKSVNNDMIVTDN
NGAVKFPQLCKFCDVRFSTCDNQKSCMSNCSITSICEKPQEVCVAVWRKNDENITLET
VCHDPKLPYHDFILEDAASPKCIMKEKKKPGETFFMCSCSSDECNDNIIFSEEYNTSNP
DPGHVFTRGMIIAVLILVAVVCLVTVCVIYRVDLVLFYRHLTRRDETLTDGKTYDAFVSY
LKECRPENGEEHTFAVEILPRVLEKHFGYKLCIFERDVVPGGAVVDEIHSLIEKSRRLIIV
LSKSYMSNEVRYELESGLHEALVERKIKIILIEFTPVTDFTFLPQSLKLLKSHRVLKWKAD
KSLSYNSRFWKNLLYLMPAKTVKPGRDEPEVLPVLSES
SEQ ID NO: 29 {#11}
   Length: 634
   Type: protein
   Organism: human
   Sequence: 
MGWSCIILFLVATATGVHSGPVPPSTALRKLIEELVNITQNQKAPLCNGSMVWSINLTA
GMYCAALESLINVSGCSAIEKTQDMLDGFCPHKVSAGQFSSLHVRDTKIEVAQFVKDLL
LHLKKLFKEGQFNGGGPRKPTTTPAPRPPTPAPTIASQPLSLRPEACRPAAGGAVHTR
GLDFACDIYIWAPLAGTCGVLLLSLVITKRGRKKLLYIFKQPFMRPVQTTQEEDGCSCR
FPEEEEGGCELNCRNTGPWLKKVLKCNTPDPSKFFSQLSSEHGGDVQKWLSSPFPS
SSFSPGGLAPEISPLEVLERDKVTQLLLQQDKVPEPASLSSNHSLTSCFTNQGYFFFHL
RVKFSRSADAPAYQQGQNQLYNELNLGRREEYDVLDKRRGRDPEMGGKPQRRKNP
QEGLYNELQKDKMAEAYSEIGMKGERRRGKGHDGLYLSLSTATKDTYLPQHMQALPP
REGRGSLLTCGDVEENPGPMYRMQLLSCIALSLALVTNSQGQDRHMIRMRQLIDIVDQ
LKNYVNDLVPEFLPAPEDVETNCEWSAFSCFQKAQLKSANTGNNERIINVSIKKLKRKP
PSTNAGRRQKHRLTCPSCDSYEKKPPKEFLERFKSLLQKMIHQHLSSRTHGSEDS
SEQ ID NO: 30 {#12}
   Length: 463
   Type: protein
   Organism: human
   Sequence: 
MGWSCIILFLVATATGVHSGPVPPSTALRKLIEELVNITQNQKAPLCNGSMVWSINLTA
GMYCAALESLINVSGCSAIEKTQDMLDGFCPHKVSAGQFSSLHVRDTKIEVAQFVKDLL
LHLKKLFKEGQFNGGGPRKPTTTPAPRPPTPAPTIASQPLSLRPEACRPAAGGAVHTR
GLDFACDIYIWAPLAGTCGVLLLSLVITKRGRKKLLYIFKQPFMRPVQTTQEEDGCSCR
FPEEEEGGCELNCRNTGPWLKKVLKCNTPDPSKFFSQLSSEHGGDVQKWLSSPFPS
SSFSPGGLAPEISPLEVLERDKVTQLLLQQDKVPEPASLSSNHSLTSCFTNQGYFFFHL
RVKFSRSADAPAYQQGQNQLYNELNLGRREEYDVLDKRRGRDPEMGGKPQRRKNP
QEGLYNELQKDKMAEAYSEI
GMKGERRRGKGHDGLYLSLSTATKDTYLPQHMQALPPR
SEQ ID NO: 31 {mutated 3rd ITAM-1}
   Length: 23
   Type: protein
   Organism: human
   Sequence:
   YLPQSTATKDTYDYVTMQALPPR
SEQ ID NO: 32 {mutated 3rd ITAM-2}
   Length: 23
   Type: protein
   Organism: human
   Sequence:
   YLSLSTATKDTYLPQHMQALPPR
SEQ ID NO: 33 {antigen binding domain binding to EGFRvIII}
   Length: 252
   Type: scFv protein
   Organism: human
   Sequence: 
Q V Q L Q E S G G G L V K P G G S L K L S C A A S G F T F S K F G M S W V R Q T
P D K R L E W V A S I D P E N D E T F Y S D N V K G R F T I S R D N A K N T L Y L
Q M S S L K S E D T A M Y Y C A R G Y G S T S F A M D Y W G Q G T M V T V S S
G S T S G S G K P G S G E G S D I Q M T Q S P S S L S A S V G D R V T I T C M T S
T D I D D D M N W Y Q Q K P G K T P K L L I Y E G N T L R P G V P S R F S G S G S
G T D F I F T I S S L Q P E D I A T Y Y C L Q S F N G P L T F G G G T K V E I K E Q
K L I S E E D L
SEQ ID NO: 34 {#13}
   Length: 499
   Type: protein
   Organism: human
   Sequence: 
M A L P V T A L L L P L A L L L H A A R P Q V Q L Q E S G G G L V K P G G S L K L
S C A A S G F T F S K F G M S W V R Q T P D K R L E W V A S I S T G G Y N T F Y
S D N V K G R F T I S R D N A K N T L Y L Q M S S L K S E D T A M Y Y C A R G Y S
S T S F A M D Y W G Q G T M V T V S S G S T S G S G K P G S G E G S D I Q M T Q
S P S S L S A S V G D R V T I T C M T S T D I D D D M N W Y Q Q K P G K T P K L L
I Y E G N T L R P G V P S R F S G S G S G T D F I F T I S S L Q P E D I A T Y Y C L
Q S F N V P L T F G G G T K V E I K E Q K L I S E E D L G G G P R K P T T T P A P
R P P T P A P T I A S Q P L S L R P E A A R P A A G G A V H T R G L D F A L A Y L L
D G I L F I Y G V I L T A L F L R V K R G R K K L L Y I F K Q P F M R P V Q T T Q E E
D G C S C R F P E E E E G G C E L K F S R S A D A P A Y Q Q G Q N Q L Y N E L N
L G R R E E Y D V L D K R R G R D P E M G G K P Q R R K N P Q E G L Y N E L Q K
D K M A E A Y S E I G M K G E R R R G K G H D G L Y Q G L S T A T K D T Y D A L
H M Q A L P P R
SEQ ID NO: 35 {#14}
   Length: 499
   Type: protein
   Organism: human
   Sequence: 
M A L P V T A L L L P L A L L L H A A R P Q V Q L Q E S G G G L V K P G G S L K L
S C A A S G F T F S K F G M S W V R Q T P D K R L E W V A S I D P E N D E T F Y
S D N V K G R F T I S R D N A K N T L Y L Q M S S L K S E D T A M Y Y C A R G Y G
S T S F A M D Y W G Q G T M V T V S S G S T S G S G K P G S G E G S D I Q M T Q
S P S S L S A S V G D R V T I T C M T S T D I D D D M N W Y Q Q K P G K T P K L L
I Y E G N T L R P G V P S R F S G S G S G T D F I F T I S S L Q P E D I A T Y Y C L
Q S F N G P L T F G G G T K V E I K E Q K L I S E E D L G G G P R K P T T T P A P
R P P T P A P T I A S Q P L S L R P E A A R P A A G G A V H T R G L D F A L A Y L L
D G I L F I Y G V I L T A L F L R V K R G R K K L L Y I F K Q P F M R P V Q T T Q E E
D G C S C R F P E E E E G G C E L K F S R S A D A P A Y Q Q G Q N Q L Y N E L N
L G R R E E Y D V L D K R R G R D P E M G G K P Q R R K N P Q E G L Y N E L Q K
D K M A E A Y S E I G M K G E R R R G K G H D G L Y Q G L S T A T K D T Y D A L
H M Q A L P P R
SEQ ID NO: 36 {#15}
   Length: 349
   Type: protein
   Organism: human
   Sequence: 
M G V K V L F A L I C I A V A E A V S D V P R D L E V V A A T P T S L L I S W D A P
A V T V R Y Y R I T Y G E T G G N S P V Q E F T V P G S K S T A T I S G L K P G V
D Y T I T V Y A V T P R G D W N E G S K P I S I N Y R T E Q K L I S E E D L G G G P
R K P T T T P A P R P P T P A P T I A S Q P L S L R P E A C R P A A G G A V H T R
G L D F A C D I Y I W A P L A G T C G V L L L S L V I T K R G R K K L L Y I F K Q P F
M R P V Q T T Q E E D G C S C R F P E E E E G G C E L R V K F S R S A D A P A Y
Q Q G Q N Q L Y N E L N L G R R E E Y D V L D K R R G R D P E M G G K P Q R R
K N P Q E G L Y N E L Q K D K M A E A Y S E I G M K G E R R R G K G H D G L Y Q
G L S T A T K D T Y D A L H M Q A L P P R
SEQ ID NO: 37 {#16}
   Length: 353
   Type: protein
   Organism: human
   Sequence: 
MALPVTALLLPLALLLHAARPVSDVPRDLEVVAATPTSLLIS
WDAPAVTVRYYRITYGETGGNSPVQEFTVPGSKSTATISGL
KPGVDYTITVYAVTPRGDWNEGSKPISINYRTEQKLISEEDL
GGGPRKPTTTPAPRPPTPAPTIASQPLSLRPEACRPAAGGA
VHTRGLDFACDIYIWAPLAGTCGVLLLSLVITKRGRKKLLYIF
 
KQPFMRPVQTTQEEDGCSCRFPEEEEGGCELRVKFSRSAD
APAYQQGQNQLYNELNLGRREEYDVLDKRRGRDPEMGGKP
QRRKNPQEGLYNELQKDKMAEAYSEIGMKGERRRGKGHDG
LYQGLSTATKDTYDALHMQALPPR
SEQ ID NO: 38 {YYB 107}
   Length: 351
   Type: protein
   Organism: human
   Sequence: 
M G W S C I I L F L V A T A T G V H S V S D V P R D L E V V A A T P T S L L I S W D
A P A V T V R Y Y R I T Y G E T G G N S P V Q E F T V P G S K S T A T I S G L K P
G V D Y T I T V Y A V T P R G D W N E G S K P I S I N Y R T E Q K L I S E E D L G G
G P R K P T T T P A P R P P T P A P T I A S Q P L S L R P E A C R P A A G G A V H
T R G L D F A C D I Y I W A P L A G T C G V L L L S L V I T K R G R K K L L Y I F K Q
P F M R P V Q T T Q E E D G C S C R F P E E E E G G C E L R V K F S R S A D A P
A Y Q Q G Q N Q L Y N E L N L G R R E E Y D V L D K R R G R D P E M G G K P Q
R R K N P Q E G L Y N E L Q K D K M A E A Y S E I G M K G E R R R G K G H D G L
Y Q G L S T A T K D T Y D A L H M Q A L P P R
SEQ ID NO: 39 {YYB 105}
   Length: 497
   Type: protein
   Organism: human
   Sequence: 
M G W S C I I L F L V A T A T G V H S Q V Q L Q E S G G G L V K P G G S L K L S C
A A S G F T F S K F G M S W V R Q T P D K R L E W V A S I S T G G Y N T F Y S D
N V K G R F T I S R D N A K N T L Y L Q M S S L K S E D T A M Y Y C A R G Y S S T
S F A M D Y W G Q G T M V T V S S G S T S G S G K P G S G E G S D I Q M T Q S P
S S L S A S V G D R V T I T C M T S T D I D D D M N W Y Q Q K P G K T P K L L I Y
E G N T L R P G V P S R F S G S G S G T D F I F T I S S L Q P E D I A T Y Y C L Q S
F N V P L T F G G G T K V E I K E Q K L I S E E D L G G G P R K P T T T P A P R P P
T P A P T I A S Q P L S L R P E A A R P A A G G A V H T R G L D F A L A Y L L D G I
L F I Y G V I L T A L F L R V K R G R K K L L Y I F K Q P F M R P V Q T T Q E E D G
C S C R F P E E E E G G C E L K F S R S A D A P A Y Q Q G Q N Q L Y N E L N L G
R R E E Y D V L D K R R G R D P E M G G K P Q R R K N P Q E G L Y N E L Q K D K
M A E A Y S E I G M K G E R R R G K G H D G L Y Q G L S T A T K D T Y D A L H M
Q A L P P R
SEQ ID NO: 40 {T2A peptide}
   Length: 21
   Type: protein
   Organism: human
   Sequence:
   GSGEGRGSLLTCGDVEENPGP
SEQ ID NO: 41 {P2A peptide}
   Length: 22
   Type: protein
   Organism: human
   Sequence:
   GSGATNFSLLKQAGDVEENPGP
SEQ ID NO: 42 {E2A peptide}
   Length: 23
   Type: protein
   Organism: human
   Sequence:
   GSGQCTNYALLKLAGDVESNPGP
SEQ ID NO: 43 {F2A peptide}
   Length: 25
   Type: protein
   Organism: human
   Sequence:
   GSGVKQTLNFDLLKLAGDVESNPGP

<110>     KONG, Seogkyoung

<120>     CHIMERIC ANTIGEN RECEPTORS AND T CELLS EXPRESSING THE CHIMERIC
          ANTIGEN RECEPTOR FOR SOLID TUMORS

<130>     KR18P0902pct

<150>     US 62/727254
<151>     2018-09-05

<160>     43

<170>     KoPatentIn 3.0

<210>     1
<211>     112
<212>     PRT
<213>     Artificial Sequence

<220>
<223>     antigen binding wild type IL13 domain binding to IL13Ra2


<400>     1
Gly Pro Val Pro Pro Ser Thr Ala Leu Arg Glu Leu Ile Glu Glu Leu
  1               5                  10                  15

Val Asn Ile Thr Gln Asn Gln Lys Ala Pro Leu Cys Asn Gly Ser Met
             20                  25                  30

Val Trp Ser Ile Asn Leu Thr Ala Gly Met Tyr Cys Ala Ala Leu Glu
         35                  40                  45

Ser Leu Ile Asn Val Ser Gly Cys Ser Ala Ile Glu Lys Thr Gln Arg
     50                  55                  60

Met Leu Ser Gly Phe Cys Pro His Lys Val Ser Ala Gly Gln Phe Ser
 65                  70                  75                  80

Ser Leu His Val Arg Asp Thr Lys Ile Glu Val Ala Gln Phe Val Lys
                 85                  90                  95

Asp Leu Leu Leu His Leu Lys Lys Leu Phe Arg Glu Gly Gln Phe Asn
            100                 105                 110




<210>     2
<211>     92
<212>     PRT
<213>     Artificial Sequence

<220>
<223>     antigen binding domain capable of binding to an antigen
          associated with an angiogenic activity


<400>     2
Glu Val Val Ala Ala Thr Pro Thr Ser Leu Leu Ile Ser Trp Arg His
  1               5                  10                  15

Pro His Phe Pro Thr Arg Tyr Tyr Arg Ile Thr Tyr Gly Glu Thr Gly

20                  25                  30

Gly Asn Ser Pro Val Gln Glu Phe Thr Val Leu Gln Pro Pro Ser Thr
        35                  40                  45

Ala Thr Ile Ser Gly Leu Lys Pro Gly Val Asp Tyr Thr Ile Thr Val
    50                  55                  60

Tyr Ala Val Val Glu Arg Asn Gly Arg Glu Leu Asn Thr Pro Pro Ile
65                  70                  75                  80

Ser Ile Asn Tyr Arg Thr His His His His His His
                85                  90


<210>    3
<211>    252
<212>    PRT
<213>    Artificial Sequence

<220>
<223>    antigen binding domain binding to EGFRvIII


<400>    3
Gln Val Gln Leu Gln Glu Ser Gly Gly Gly Leu Val Lys Pro Gly Gly
1               5                   10                  15

Ser Leu Lys Leu Ser Cys Ala Ala Ser Gly Phe Thr Phe Ser Lys Phe
            20                  25                  30

Gly Met Ser Trp Val Arg Gln Thr Pro Asp Lys Arg Leu Glu Trp Val
        35                  40                  45

Ala Ser Ile Ser Thr Gly Gly Tyr Asn Thr Phe Tyr Ser Asp Asn Val
    50                  55                  60

Lys Gly Arg Phe Thr Ile Ser Arg Asp Asn Ala Lys Asn Thr Leu Tyr
65                  70                  75                  80

Leu Gln Met Ser Ser Leu Lys Ser Glu Asp Thr Ala Met Tyr Tyr Cys
                85                  90                  95

Ala Arg Gly Tyr Ser Ser Thr Ser Phe Ala Met Asp Tyr Trp Gly Gln
            100                 105                 110

Gly Thr Met Val Thr Val Ser Ser Gly Ser Thr Ser Gly Ser Gly Lys
        115                 120                 125

Pro Gly Ser Gly Glu Gly Ser Asp Ile Gln Met Thr Gln Ser Pro Ser
    130                 135                 140

Ser Leu Ser Ala Ser Val Gly Asp Arg Val Thr Ile Thr Cys Met Thr
145                 150                 155                 160

Ser Thr Asp Ile Asp Asp Asp Met Asn Trp Tyr Gln Gln Lys Pro Gly
                165                 170                 175

Lys Thr Pro Lys Leu Leu Ile Tyr Glu Gly Asn Thr Leu Arg Pro Gly
            180                 185                 190

Val Pro Ser Arg Phe Ser Gly Ser Gly Ser Gly Thr Asp Phe Ile Phe
        195                 200                 205

Thr Ile Ser Ser Leu Gln Pro Glu Asp Ile Ala Thr Tyr Tyr Cys Leu
    210                 215                 220

Gln Ser Phe Asn Val Pro Leu Thr Phe Gly Gly Gly Thr Lys Val Glu
225                 230                 235                 240

Ile Lys Glu Gln Lys Leu Ile Ser Glu Glu Asp Leu
                245                 250

<210>    4
<211>    141
<212>    PRT
<213>    Artificial Sequence

<220>
<223>    antigen binding domain binding to EphA2

<400>    4
Asp Arg His Thr Val Phe Trp Asn Ser Ser Asn Pro Lys Phe Arg Asn
1               5                   10                  15

Glu Asp Tyr Thr Ile His Val Gln Leu Asn Asp Tyr Val Asp Ile Ile
            20                  25                  30

Cys Pro His Tyr Glu Asp His Ser Val Ala Asp Ala Ala Met Glu Gln
        35                  40                  45

Tyr Ile Leu Tyr Leu Val Glu His Glu Glu Tyr Gln Leu Cys Gln Pro
    50                  55                  60

Gln Ser Lys Asp Gln Val Arg Trp Gln Cys Asn Arg Pro Ser Ala Lys
65                  70                  75                  80

His Gly Pro Glu Lys Leu Ser Glu Lys Phe Gln Arg Phe Thr Ala Phe
                85                  90                  95

Ala Leu Ala Lys Glu Phe Lys Ala Gly His Ser Tyr Tyr Tyr Ile Ser
            100                 105                 110

Lys Pro Ile His Gln His Glu Asp Arg Cys Leu Arg Leu Lys Val Thr
        115                 120                 125

Val Ser Gly Glu Gln Lys Leu Ile Ser Glu Glu Asp Leu
    130                 135                 140

<210>    5
<211>    104
<212>    PRT
<213>    Artificial Sequence

<220>
<223>    antigen binding domain binding to alphaVbeta3

<400>    5
Val Ser Asp Val Pro Arg Asp Leu Glu Val Val Ala Ala Thr Pro Thr
1               5                   10                  15

Ser Leu Leu Ile Ser Trp Asp Ala Pro Ala Val Thr Val Arg Tyr Tyr
            20                  25                  30

Arg Ile Thr Tyr Gly Glu Thr Gly Gly Asn Ser Pro Val Gln Glu Phe
        35                  40                  45

Thr Val Pro Gly Ser Lys Ser Thr Ala Thr Ile Ser Gly Leu Lys Pro
    50                  55                  60

Gly Val Asp Tyr Thr Ile Thr Val Tyr Ala Val Thr Pro Arg Gly Asp
65                  70                  75                  80

Trp Asn Glu Gly Ser Lys Pro Ile Ser Ile Asn Tyr Arg Thr Glu Gln
                85                  90                  95

Lys Leu Ile Ser Glu Glu Asp Leu
            100

<210> 6
<211> 418
<212> PRT
<213> Artificial Sequence

<220>
<223> antigen binding domain binding to glypican one

<400> 6
Thr Ser Pro Cys Asp Asn Phe Asp Cys Gln Asn Gly Ala Gln Cys Ile
1               5                   10                  15

Val Arg Ile Asn Glu Pro Ile Cys Gln Cys Leu Pro Gly Tyr Gln Gly
            20                  25                  30

Glu Lys Cys Glu Lys Leu Val Ser Val Asn Phe Ile Asn Lys Glu Ser
        35                  40                  45

Tyr Leu Gln Ile Pro Ser Ala Lys Val Arg Pro Gln Thr Asn Ile Thr
    50                  55                  60

Leu Gln Ile Ala Thr Asp Glu Asp Ser Gly Ile Leu Leu Tyr Lys Gly
65                  70                  75                  80

Asp Lys Asp His Ile Ala Val Glu Leu Tyr Arg Gly Arg Val Arg Ala
                85                  90                  95

Ser Tyr Asp Thr Gly Ser His Pro Ala Ser Ala Ile Tyr Ser Val Glu
            100                 105                 110

Thr Ile Asn Asp Gly Asn Phe His Ile Val Glu Leu Leu Ala Leu Asp
        115                 120                 125

Gln Ser Leu Ser Leu Ser Val Asp Gly Gly Asn Pro Lys Ile Ile Thr
    130                 135                 140

Asn Leu Ser Lys Gln Ser Thr Leu Asn Phe Asp Ser Pro Leu Tyr Val
145                 150                 155                 160

Gly Gly Met Pro Gly Lys Ser Asn Val Ala Ser Leu Arg Gln Ala Pro
                165                 170                 175

Gly Gln Asn Gly Thr Ser Phe His Gly Cys Ile Arg Asn Leu Tyr Ile

180                 185                 190

Asn Ser Glu Leu Gln Asp Phe Gln Lys Val Pro Met Gln Thr Gly Ile
        195                 200                 205

Leu Pro Gly Cys Glu Pro Cys His Lys Lys Val Cys Ala His Gly Thr
    210                 215                 220

Cys Gln Pro Ser Ser Gln Ala Gly Phe Thr Cys Glu Cys Gln Glu Gly
225                 230                 235                 240

Trp Met Gly Pro Leu Cys Asp Gln Arg Thr Asn Asp Pro Cys Leu Gly
                245                 250                 255

Asn Lys Cys Val His Gly Thr Cys Leu Pro Ile Asn Ala Phe Ser Tyr
            260                 265                 270

Ser Cys Lys Cys Leu Glu Gly His Gly Gly Val Leu Cys Asp Glu Glu
        275                 280                 285

Glu Asp Leu Phe Asn Pro Cys Gln Ala Ile Lys Cys Lys His Gly Lys
    290                 295                 300

Cys Arg Leu Ser Gly Leu Gly Gln Pro Tyr Cys Glu Cys Ser Ser Gly
305                 310                 315                 320

Tyr Thr Gly Asp Ser Cys Asp Arg Glu Ile Ser Cys Arg Gly Glu Arg
                325                 330                 335

Ile Arg Asp Tyr Tyr Gln Lys Gln Gln Gly Tyr Ala Ala Cys Gln Thr
            340                 345                 350

Thr Lys Lys Val Ser Arg Leu Glu Cys Arg Gly Gly Cys Ala Gly Gly
        355                 360                 365

Gln Cys Cys Gly Pro Leu Arg Ser Lys Arg Arg Lys Tyr Ser Phe Glu
    370                 375                 380

Cys Thr Asp Gly Ser Ser Phe Val Asp Glu Val Glu Lys Val Val Lys
385                 390                 395                 400

Cys Gly Cys Thr Arg Cys Val Ser Glu Gln Lys Leu Ile Ser Glu Glu
                405                 410                 415

Asp Leu

<210>   7
<211>   262
<212>   PRT
<213>   Artificial Sequence

<220>
<223>   antigen binding domain binding to mesothelin

<400>   7
Gln Val Gln Leu Gln Glu Ser Gly Pro Gly Leu Val Lys Pro Ser Glu
1               5                   10                  15

Thr Leu Ser Leu Thr Cys Thr Val Ser Gly Gly Ser Val Ser Ser Gly
            20                  25                  30

Ser Tyr Tyr Trp Ser Trp Ile Arg Gln Pro Pro Gly Lys Gly Leu Glu
        35                  40                  45

Trp Ile Gly Tyr Ile Tyr Tyr Ser Gly Ser Thr Asn Tyr Asn Pro Ser
    50                  55                  60

Leu Lys Ser Arg Val Thr Ile Ser Val Asp Thr Ser Lys Asn Gln Phe
65                  70                  75                  80

Ser Leu Lys Leu Ser Ser Val Thr Ala Ala Asp Thr Ala Val Tyr Tyr
                85                  90                  95

Cys Ala Arg Glu Gly Lys Asn Gly Ala Phe Asp Ile Trp Gly Gln Gly
            100                 105                 110

Thr Met Val Thr Val Ser Ser Gly Ser Thr Ser Gly Ser Gly Lys Pro
        115                 120                 125

Gly Ser Gly Glu Gly Ser Gln Val Gln Leu Gln Gln Ser Gly Pro Gly
    130                 135                 140

Leu Val Thr Pro Ser Gln Thr Leu Ser Leu Thr Cys Ala Ile Ser Gly
145                 150                 155                 160

Asp Ser Val Ser Ser Asn Ser Ala Thr Trp Asn Trp Ile Arg Gln Ser
                165                 170                 175

Pro Ser Arg Gly Leu Glu Trp Leu Gly Arg Thr Tyr Tyr Arg Ser Lys
            180                 185                 190

Trp Tyr Asn Asp Tyr Ala Val Ser Val Lys Ser Arg Met Ser Ile Asn
        195                 200                 205

Pro Asp Thr Ser Lys Asn Gln Phe Ser Leu Gln Leu Asn Ser Val Thr
    210                 215                 220

Pro Glu Asp Thr Ala Val Tyr Tyr Cys Ala Arg Gly Met Met Thr Tyr
225                 230                 235                 240

Tyr Tyr Gly Met Asp Val Trp Gly Gln Gly Thr Thr Val Thr Val Ser
                245                 250                 255

Ser Gly Ile Leu Gly Ser
            260

<210> 8
<211> 47
<212> PRT
<213> Artificial Sequence

<220>
<223> hinge region sequence 1

<400> 8
Lys Pro Thr Thr Thr Pro Ala Pro Arg Pro Pro Thr Pro Ala Pro Thr
1               5                   10                  15

Ile Ala Ser Gln Pro Leu Ser Leu Arg Pro Glu Ala Cys Arg Pro Ala
            20                  25                  30

Ala Gly Gly Ala Val His Thr Arg Gly Leu Asp Phe Ala Cys Asp
        35                  40                  45

<210>   9
<211>   45
<212>   PRT
<213>   Artificial Sequence

<220>
<223>   hinge region sequence 2

<400>   9
Lys Pro Thr Thr Thr Pro Ala Pro Arg Pro Pro Thr Pro Ala Pro Thr
1               5                   10                  15

Ile Ala Ser Gln Pro Leu Ser Leu Arg Pro Glu Ala Ala Arg Pro Ala
            20                  25                  30

Ala Gly Gly Ala Val His Thr Arg Gly Leu Asp Phe Ala
        35                  40                  45

<210>   10
<211>   21
<212>   PRT
<213>   Artificial Sequence

<220>
<223>   transmembrane domain sequence 1

<400>   10
Ile Tyr Ile Trp Ala Pro Leu Ala Gly Thr Cys Gly Val Leu Leu Leu
1               5                   10                  15

Ser Leu Val Ile Thr
            20

<210>   11
<211>   23
<212>   PRT
<213>   Artificial Sequence

<220>
<223>   transmembrane domain sequence 2

<400>   11
Leu Ala Tyr Leu Leu Asp Gly Ile Leu Phe Ile Tyr Gly Val Ile Leu
1               5                   10                  15

Thr Ala Leu Phe Leu Arg Val
            20

<210>   12
<211>   42
<212>   PRT

<213>    Artificial Sequence

<220>
<223>    4-1BB


<400>    12
Lys Arg Gly Arg Lys Lys Leu Leu Tyr Ile Phe Lys Gln Pro Phe Met
1               5                   10                  15

Arg Pro Val Gln Thr Thr Gln Glu Glu Asp Gly Cys Ser Cys Arg Phe
            20                  25                  30

Pro Glu Glu Glu Glu Gly Gly Cys Glu Leu
        35                  40


<210>    13
<211>    41
<212>    PRT
<213>    Artificial Sequence

<220>
<223>    wild type CD28


<400>    13
Arg Ser Lys Arg Ser Arg Leu Leu His Ser Asp Tyr Met Asn Met Thr
1               5                   10                  15

Pro Arg Arg Pro Gly Pro Thr Arg Lys His Tyr Gln Pro Tyr Ala Pro
            20                  25                  30

Pro Arg Asp Phe Ala Ala Tyr Arg Ser
        35                  40


<210>    14
<211>    459
<212>    PRT
<213>    Artificial Sequence

<220>
<223>    IL7Ra


<400>    14


<210>   15
<211>   64
<212>   PRT
<213>   Artificial Sequence

<220>
<223>   part of IL7Ra


<400>   15
Lys Lys Arg Ile Lys Pro Ile Val Trp Pro Ser Leu Pro Asp His Lys
1               5                   10                  15

Lys Thr Leu Glu His Leu Cys Lys Lys Pro Arg Lys Asn Leu Asn Val
            20                  25                  30

Ser Phe Asn Pro Glu Ser Phe Leu Asp Cys Gln Ile His Arg Val Asp
        35                  40                  45

Asp Ile Gln Ala Arg Asp Glu Val Glu Gly Phe Leu Gln Asp Thr Phe
    50                  55                  60




<210>   16
<211>   551
<212>   PRT
<213>   Artificial Sequence

<220>
<223>   IL2Rb


<400>   16
Met Ala Ala Pro Ala Leu Ser Trp Arg Leu Pro Leu Leu Ile Leu Leu
1               5                   10                  15

Leu Pro Leu Ala Thr Ser Trp Ala Ser Ala Ala Val Asn Gly Thr Ser
            20                  25                  30

Gln Phe Thr Cys Phe Tyr Asn Ser Arg Ala Asn Ile Ser Cys Val Trp
        35                  40                  45

Ser Gln Asp Gly Ala Leu Gln Asp Thr Ser Cys Gln Val His Ala Trp
    50                  55                  60

Pro Asp Arg Arg Arg Trp Asn Gln Thr Cys Glu Leu Leu Pro Val Ser
65                  70                  75                  80

Gln Ala Ser Trp Ala Cys Asn Leu Ile Leu Gly Ala Pro Asp Ser Gln
                85                  90                  95

Lys Leu Thr Thr Val Asp Ile Val Thr Leu Arg Val Leu Cys Arg Glu

            100                 105                 110

Gly Val Arg Trp Arg Val Met Ala Ile Gln Asp Phe Lys Pro Phe Glu
        115                 120                 125

Asn Leu Arg Leu Met Ala Pro Ile Ser Leu Gln Val Val His Val Glu
    130                 135                 140

Thr His Arg Cys Asn Ile Ser Trp Glu Ile Ser Gln Ala Ser His Tyr
145                 150                 155                 160

Phe Glu Arg His Leu Glu Phe Glu Ala Arg Thr Leu Ser Pro Gly His
                165                 170                 175

Thr Trp Glu Glu Ala Pro Leu Leu Thr Leu Lys Gln Lys Gln Glu Trp
            180                 185                 190

Ile Cys Leu Glu Thr Leu Thr Pro Asp Thr Gln Tyr Glu Phe Gln Val
        195                 200                 205

Arg Val Lys Pro Leu Gln Gly Glu Phe Thr Thr Trp Ser Pro Trp Ser
    210                 215                 220

Gln Pro Leu Ala Phe Arg Thr Lys Pro Ala Ala Leu Gly Lys Asp Thr
225                 230                 235                 240

Ile Pro Trp Leu Gly His Leu Leu Val Gly Leu Ser Gly Ala Phe Gly
                245                 250                 255

Phe Ile Ile Leu Val Tyr Leu Leu Ile Asn Cys Arg Asn Thr Gly Pro
            260                 265                 270

Trp Leu Lys Lys Val Leu Lys Cys Asn Thr Pro Asp Pro Ser Lys Phe
        275                 280                 285

Phe Ser Gln Leu Ser Ser Glu His Gly Gly Asp Val Gln Lys Trp Leu
    290                 295                 300

Ser Ser Pro Phe Pro Ser Ser Ser Phe Ser Pro Gly Gly Leu Ala Pro
305                 310                 315                 320

Glu Ile Ser Pro Leu Glu Val Leu Glu Arg Asp Lys Val Thr Gln Leu
                325                 330                 335

Leu Leu Gln Gln Asp Lys Val Pro Glu Pro Ala Ser Leu Ser Ser Asn
            340                 345                 350

His Ser Leu Thr Ser Cys Phe Thr Asn Gln Gly Tyr Phe Phe Phe His
        355                 360                 365

Leu Pro Asp Ala Leu Glu Ile Glu Ala Cys Gln Val Tyr Phe Thr Tyr
    370                 375                 380

Asp Pro Tyr Ser Glu Glu Asp Pro Asp Glu Gly Val Ala Gly Ala Pro
385                 390                 395                 400

Thr Gly Ser Ser Pro Gln Pro Leu Gln Pro Leu Ser Gly Glu Asp Asp
                405                 410                 415

Ala Tyr Cys Thr Phe Pro Ser Arg Asp Asp Leu Leu Leu Phe Ser Pro
            420                 425                 430

Ser Leu Leu Gly Gly Pro Ser Pro Pro Ser Thr Ala Pro Gly Gly Ser

435                 440                 445

Gly Ala Gly Glu Glu Arg Met Pro Pro Ser Leu Gln Glu Arg Val Pro
    450                 455                 460

Arg Asp Trp Asp Pro Gln Pro Leu Gly Pro Pro Thr Pro Gly Val Pro
465                 470                 475                 480

Asp Leu Val Asp Phe Gln Pro Pro Pro Glu Leu Val Leu Arg Glu Ala
                485                 490                 495

Gly Glu Glu Val Pro Asp Ala Gly Pro Arg Glu Gly Val Ser Phe Pro
            500                 505                 510

Trp Ser Arg Pro Pro Gly Gln Gly Glu Phe Arg Ala Leu Asn Ala Arg
        515                 520                 525

Leu Pro Leu Asn Thr Asp Ala Tyr Leu Ser Leu Gln Glu Leu Gln Gly
    530                 535                 540

Gln Asp Pro Thr His Leu Val
545                 550


<210>    17
<211>    104
<212>    PRT
<213>    Artificial Sequence

<220>
<223>    part of IL2Rb


<400>    17
Asn Cys Arg Asn Thr Gly Pro Trp Leu Lys Lys Val Leu Lys Cys Asn
1               5                   10                  15

Thr Pro Asp Pro Ser Lys Phe Phe Ser Gln Leu Ser Ser Glu His Gly
            20                  25                  30

Gly Asp Val Gln Lys Trp Leu Ser Ser Pro Phe Pro Ser Ser Ser Phe
        35                  40                  45

Ser Pro Gly Gly Leu Ala Pro Glu Ile Ser Pro Leu Glu Val Leu Glu
    50                  55                  60

Arg Asp Lys Val Thr Gln Leu Leu Leu Gln Gln Asp Lys Val Pro Glu
65                  70                  75                  80

Pro Ala Ser Leu Ser Ser Asn His Ser Leu Thr Ser Cys Phe Thr Asn
                85                  90                  95

Gln Gly Tyr Phe Phe Phe His Leu
            100


<210>    18
<211>    113
<212>    PRT
<213>    Artificial Sequence

<220>
<223>    CD3zeta

<400>    18
Arg Val Lys Phe Ser Arg Ser Ala Asp Ala Pro Ala Tyr Gln Gln Gly
1               5                   10                  15

Gln Asn Gln Leu Tyr Asn Glu Leu Asn Leu Gly Arg Arg Glu Glu Tyr
            20                  25                  30

Asp Val Leu Asp Lys Arg Arg Gly Arg Asp Pro Glu Met Gly Gly Lys
        35                  40                  45

Pro Gln Arg Arg Lys Asn Pro Gln Glu Gly Leu Tyr Asn Glu Leu Gln
    50                  55                  60

Lys Asp Lys Met Ala Glu Ala Tyr Ser Glu Ile Gly Met Lys Gly Glu
65                  70                  75                  80

Arg Arg Arg Gly Lys Gly His Asp Gly Leu Tyr Gln Gly Leu Ser Thr
                85                  90                  95

Ala Thr Lys Asp Thr Tyr Asp Ala Leu His Met Gln Ala Leu Pro Pro
            100                 105                 110

Arg



<210>    19
<211>    137
<212>    PRT
<213>    Artificial Sequence

<220>
<223>    TGF bR2 exodomain


<400>    19
Thr Ile Pro Pro His Val Gln Lys Ser Val Asn Asn Asp Met Ile Val
1               5                   10                  15

Thr Asp Asn Asn Gly Ala Val Lys Phe Pro Gln Leu Cys Lys Phe Cys
            20                  25                  30

Asp Val Arg Phe Ser Thr Cys Asp Asn Gln Lys Ser Cys Met Ser Asn
        35                  40                  45

Cys Ser Ile Thr Ser Ile Cys Glu Lys Pro Gln Glu Val Cys Val Ala
    50                  55                  60

Val Trp Arg Lys Asn Asp Glu Asn Ile Thr Leu Glu Thr Val Cys His
65                  70                  75                  80

Asp Pro Lys Leu Pro Tyr His Asp Phe Ile Leu Glu Asp Ala Ala Ser
                85                  90                  95

Pro Lys Cys Ile Met Lys Glu Lys Lys Lys Pro Gly Glu Thr Phe Phe
            100                 105                 110

Met Cys Ser Cys Ser Ser Asp Glu Cys Asn Asp Asn Ile Ile Phe Ser
        115                 120                 125

Glu Glu Tyr Asn Thr Ser Asn Pro Asp

130                 135

<210>    20
<211>    219
<212>    PRT
<213>    Artificial Sequence

<220>
<223>    transmembrane domain and endodomain of IL18R

<400>    20
Pro Gly His Val Phe Thr Arg Gly Met Ile Ile Ala Val Leu Ile Leu
1               5                   10                  15

Val Ala Val Val Cys Leu Val Thr Val Cys Val Ile Tyr Arg Val Asp
            20                  25                  30

Leu Val Leu Phe Tyr Arg His Leu Thr Arg Arg Asp Glu Thr Leu Thr
        35                  40                  45

Asp Gly Lys Thr Tyr Asp Ala Phe Val Ser Tyr Leu Lys Glu Cys Arg
    50                  55                  60

Pro Glu Asn Gly Glu Glu His Thr Phe Ala Val Glu Ile Leu Pro Arg
65                  70                  75                  80

Val Leu Glu Lys His Phe Gly Tyr Lys Leu Cys Ile Phe Glu Arg Asp
                85                  90                  95

Val Val Pro Gly Gly Ala Val Val Asp Glu Ile His Ser Leu Ile Glu
            100                 105                 110

Lys Ser Arg Arg Leu Ile Ile Val Leu Ser Lys Ser Tyr Met Ser Asn
        115                 120                 125

Glu Val Arg Tyr Glu Leu Glu Ser Gly Leu His Glu Ala Leu Val Glu
    130                 135                 140

Arg Lys Ile Lys Ile Ile Leu Ile Glu Phe Thr Pro Val Thr Asp Phe
145                 150                 155                 160

Thr Phe Leu Pro Gln Ser Leu Lys Leu Leu Lys Ser His Arg Val Leu
                165                 170                 175

Lys Trp Lys Ala Asp Lys Ser Leu Ser Tyr Asn Ser Arg Phe Trp Lys
            180                 185                 190

Asn Leu Leu Tyr Leu Met Pro Ala Lys Thr Val Lys Pro Gly Arg Asp
        195                 200                 205

Glu Pro Glu Val Leu Pro Val Leu Ser Glu Ser
    210                 215

<210>    21
<211>    133
<212>    PRT
<213>    Artificial Sequence

<220>
<223>    IL21

<400>    21


<210>    22
<211>    359
<212>    PRT
<213>    Artificial Sequence

<220>
<223>    YYB 103 CAR


<400>    22
Met Gly Trp Ser Cys Ile Ile Leu Phe Leu Val Ala Thr Ala Thr Gly
1               5                   10                  15

Val His Ser Gly Pro Val Pro Pro Ser Thr Ala Leu Arg Lys Leu Ile
            20                  25                  30

Glu Glu Leu Val Asn Ile Thr Gln Asn Gln Lys Ala Pro Leu Cys Asn
        35                  40                  45

Gly Ser Met Val Trp Ser Ile Asn Leu Thr Ala Gly Met Tyr Cys Ala
    50                  55                  60

Ala Leu Glu Ser Leu Ile Asn Val Ser Gly Cys Ser Ala Ile Glu Lys
65                  70                  75                  80

Thr Gln Asp Met Leu Asp Gly Phe Cys Pro His Lys Val Ser Ala Gly
                85                  90                  95

Gln Phe Ser Ser Leu His Val Arg Asp Thr Lys Ile Glu Val Ala Gln
            100                 105                 110

Phe Val Lys Asp Leu Leu Leu His Leu Lys Lys Leu Phe Lys Glu Gly

115                 120                 125

Gln Phe Asn Gly Gly Gly Pro Arg Lys Pro Thr Thr Thr Pro Ala Pro
    130                 135                 140

Arg Pro Pro Thr Pro Ala Pro Thr Ile Ala Ser Gln Pro Leu Ser Leu
145                 150                 155                 160

Arg Pro Glu Ala Cys Arg Pro Ala Ala Gly Gly Ala Val His Thr Arg
                165                 170                 175

Gly Leu Asp Phe Ala Cys Asp Ile Tyr Ile Trp Ala Pro Leu Ala Gly
            180                 185                 190

Thr Cys Gly Val Leu Leu Leu Ser Leu Val Ile Thr Lys Arg Gly Arg
        195                 200                 205

Lys Lys Leu Leu Tyr Ile Phe Lys Gln Pro Phe Met Arg Pro Val Gln
    210                 215                 220

Thr Thr Gln Glu Glu Asp Gly Cys Ser Cys Arg Phe Pro Glu Glu Glu
225                 230                 235                 240

Glu Gly Gly Cys Glu Leu Arg Val Lys Phe Ser Arg Ser Ala Asp Ala
                245                 250                 255

Pro Ala Tyr Gln Gln Gly Gln Asn Gln Leu Tyr Asn Glu Leu Asn Leu
            260                 265                 270

Gly Arg Arg Glu Glu Tyr Asp Val Leu Asp Lys Arg Arg Gly Arg Asp
        275                 280                 285

Pro Glu Met Gly Gly Lys Pro Gln Arg Arg Lys Asn Pro Gln Glu Gly
    290                 295                 300

Leu Tyr Asn Glu Leu Gln Lys Asp Lys Met Ala Glu Ala Tyr Ser Glu
305                 310                 315                 320

Ile Gly Met Lys Gly Glu Arg Arg Arg Gly Lys Gly His Asp Gly Leu
                325                 330                 335

Tyr Gln Gly Leu Ser Thr Ala Thr Lys Asp Thr Tyr Asp Ala Leu His
            340                 345                 350

Met Gln Ala Leu Pro Pro Arg
        355


<210>   23
<211>   998
<212>   PRT
<213>   Artificial Sequence

<220>
<223>   polypeptide No 1


<400>   23
Met Gly Trp Ser Cys Ile Ile Leu Phe Leu Val Ala Thr Ala Thr Gly
1               5                   10                  15

Val His Ser Gly Pro Val Pro Pro Ser Thr Ala Leu Arg Lys Leu Ile
            20                  25                  30

Glu Glu Leu Val Asn Ile Thr Gln Asn Gln Lys Ala Pro Leu Cys Asn
            35                  40                  45

Gly Ser Met Val Trp Ser Ile Asn Leu Thr Ala Gly Met Tyr Cys Ala
    50                  55                  60

Ala Leu Glu Ser Leu Ile Asn Val Ser Gly Cys Ser Ala Ile Glu Lys
65                  70                  75                  80

Thr Gln Asp Met Leu Asp Gly Phe Cys Pro His Lys Val Ser Ala Gly
                85                  90                  95

Gln Phe Ser Ser Leu His Val Arg Asp Thr Lys Ile Glu Val Ala Gln
            100                 105                 110

Phe Val Lys Asp Leu Leu Leu His Leu Lys Lys Leu Phe Lys Glu Gly
        115                 120                 125

Gln Phe Asn Gly Gly Gly Pro Arg Lys Pro Thr Thr Thr Pro Ala Pro
    130                 135                 140

Arg Pro Pro Thr Pro Ala Pro Thr Ile Ala Ser Gln Pro Leu Ser Leu
145                 150                 155                 160

Arg Pro Glu Ala Cys Arg Pro Ala Ala Gly Gly Ala Val His Thr Arg
                165                 170                 175

Gly Leu Asp Phe Ala Cys Asp Ile Tyr Ile Trp Ala Pro Leu Ala Gly
            180                 185                 190

Thr Cys Gly Val Leu Leu Leu Ser Leu Val Ile Thr Lys Arg Gly Arg
        195                 200                 205

Lys Lys Leu Leu Tyr Ile Phe Lys Gln Pro Phe Met Arg Pro Val Gln
    210                 215                 220

Thr Thr Gln Glu Glu Asp Gly Cys Ser Cys Arg Phe Pro Glu Glu Glu
225                 230                 235                 240

Glu Gly Gly Cys Glu Leu Lys Lys Arg Ile Lys Pro Ile Val Trp Pro
                245                 250                 255

Ser Leu Pro Asp His Lys Lys Thr Leu Glu His Leu Cys Lys Lys Pro
            260                 265                 270

Arg Lys Asn Leu Asn Val Ser Phe Asn Pro Glu Ser Phe Leu Asp Cys
        275                 280                 285

Gln Ile His Arg Val Asp Asp Ile Gln Ala Arg Asp Glu Val Glu Gly
    290                 295                 300

Phe Leu Gln Asp Thr Phe Arg Val Lys Phe Ser Arg Ser Ala Asp Ala
305                 310                 315                 320

Pro Ala Tyr Gln Gln Gly Gln Asn Gln Leu Tyr Asn Glu Leu Asn Leu
                325                 330                 335

Gly Arg Arg Glu Glu Tyr Asp Val Leu Asp Lys Arg Arg Gly Arg Asp
            340                 345                 350

Pro Glu Met Gly Gly Lys Pro Gln Arg Arg Lys Asn Pro Gln Glu Gly
        355                 360                 365

Leu Tyr Asn Glu Leu Gln Lys Asp Lys Met Ala Glu Ala Tyr Ser Glu
    370                 375                 380

Ile Gly Met Lys Gly Glu Arg Arg Arg Gly Lys Gly His Asp Gly Leu
385                 390                 395                 400

Tyr Leu Pro Gln Ser Thr Ala Thr Lys Asp Thr Tyr Asp Tyr Val Thr
                405                 410                 415

Met Gln Ala Leu Pro Pro Arg Glu Gly Arg Gly Ser Leu Leu Thr Cys
            420                 425                 430

Gly Asp Val Glu Glu Asn Pro Gly Pro Met Ala Leu Pro Val Thr Ala
        435                 440                 445

Leu Leu Leu Pro Leu Ala Leu Leu Leu His Ala Ala Arg Pro Thr Ile
    450                 455                 460

Pro Pro His Val Gln Lys Ser Val Asn Asn Asp Met Ile Val Thr Asp
465                 470                 475                 480

Asn Asn Gly Ala Val Lys Phe Pro Gln Leu Cys Lys Phe Cys Asp Val
                485                 490                 495

Arg Phe Ser Thr Cys Asp Asn Gln Lys Ser Cys Met Ser Asn Cys Ser
            500                 505                 510

Ile Thr Ser Ile Cys Glu Lys Pro Gln Glu Val Cys Val Ala Val Trp
        515                 520                 525

Arg Lys Asn Asp Glu Asn Ile Thr Leu Glu Thr Val Cys His Asp Pro
    530                 535                 540

Lys Leu Pro Tyr His Asp Phe Ile Leu Glu Asp Ala Ala Ser Pro Lys
545                 550                 555                 560

Cys Ile Met Lys Glu Lys Lys Lys Pro Gly Glu Thr Phe Phe Met Cys
                565                 570                 575

Ser Cys Ser Ser Asp Glu Cys Asn Asp Asn Ile Ile Phe Ser Glu Glu
            580                 585                 590

Tyr Asn Thr Ser Asn Pro Asp Pro Gly His Val Phe Thr Arg Gly Met
        595                 600                 605

Ile Ile Ala Val Leu Ile Leu Val Ala Val Val Cys Leu Val Thr Val
    610                 615                 620

Cys Val Ile Tyr Arg Val Asp Leu Val Leu Phe Tyr Arg His Leu Thr
625                 630                 635                 640

Arg Arg Asp Glu Thr Leu Thr Asp Gly Lys Thr Tyr Asp Ala Phe Val
                645                 650                 655

Ser Tyr Leu Lys Glu Cys Arg Pro Glu Asn Gly Glu Glu His Thr Phe
            660                 665                 670

Ala Val Glu Ile Leu Pro Arg Val Leu Glu Lys His Phe Gly Tyr Lys
        675                 680                 685

Leu Cys Ile Phe Glu Arg Asp Val Val Pro Gly Gly Ala Val Val Asp
    690                 695                 700

Glu Ile His Ser Leu Ile Glu Lys Ser Arg Arg Leu Ile Ile Val Leu
705                 710                 715                 720

Ser Lys Ser Tyr Met Ser Asn Glu Val Arg Tyr Glu Leu Glu Ser Gly
                725                 730                 735

Leu His Glu Ala Leu Val Glu Arg Lys Ile Lys Ile Ile Leu Ile Glu
            740                 745                 750

Phe Thr Pro Val Thr Asp Phe Thr Phe Leu Pro Gln Ser Leu Lys Leu
        755                 760                 765

Leu Lys Ser His Arg Val Leu Lys Trp Lys Ala Asp Lys Ser Leu Ser
    770                 775                 780

Tyr Asn Ser Arg Phe Trp Lys Asn Leu Leu Tyr Leu Met Pro Ala Lys
785                 790                 795                 800

Thr Val Lys Pro Gly Arg Asp Glu Pro Glu Val Leu Pro Val Leu Ser
                805                 810                 815

Glu Ser Arg Arg Lys Arg Ser Gly Ser Gly Ala Thr Asn Phe Ser Leu
            820                 825                 830

Leu Lys Gln Ala Gly Asp Val Glu Glu Asn Pro Gly Pro Met Tyr Arg
        835                 840                 845

Met Gln Leu Leu Ser Cys Ile Ala Leu Ser Leu Ala Leu Val Thr Asn
    850                 855                 860

Ser Gln Gly Gln Asp Arg His Met Ile Arg Met Arg Gln Leu Ile Asp
865                 870                 875                 880

Ile Val Asp Gln Leu Lys Asn Tyr Val Asn Asp Leu Val Pro Glu Phe
                885                 890                 895

Leu Pro Ala Pro Glu Asp Val Glu Thr Asn Cys Glu Trp Ser Ala Phe
            900                 905                 910

Ser Cys Phe Gln Lys Ala Gln Leu Lys Ser Ala Asn Thr Gly Asn Asn
        915                 920                 925

Glu Arg Ile Ile Asn Val Ser Ile Lys Lys Leu Lys Arg Lys Pro Pro
    930                 935                 940

Ser Thr Asn Ala Gly Arg Arg Gln Lys His Arg Leu Thr Cys Pro Ser
945                 950                 955                 960

Cys Asp Ser Tyr Glu Lys Lys Pro Pro Lys Glu Phe Leu Glu Arg Phe
                965                 970                 975

Lys Ser Leu Leu Gln Lys Met Ile His Gln His Leu Ser Ser Arg Thr
            980                 985                 990

His Gly Ser Glu Asp Ser
        995


<210>  24
<211>  818
<212>  PRT
<213>  Artificial Sequence

<220>
<223>    polypeptide No 2

<400>    24
Met Gly Trp Ser Cys Ile Ile Leu Phe Leu Val Ala Thr Ala Thr Gly
1               5                   10                  15

Val His Ser Gly Pro Val Pro Pro Ser Thr Ala Leu Arg Lys Leu Ile
            20                  25                  30

Glu Glu Leu Val Asn Ile Thr Gln Asn Gln Lys Ala Pro Leu Cys Asn
        35                  40                  45

Gly Ser Met Val Trp Ser Ile Asn Leu Thr Ala Gly Met Tyr Cys Ala
    50                  55                  60

Ala Leu Glu Ser Leu Ile Asn Val Ser Gly Cys Ser Ala Ile Glu Lys
65                  70                  75                  80

Thr Gln Asp Met Leu Asp Gly Phe Cys Pro His Lys Val Ser Ala Gly
                85                  90                  95

Gln Phe Ser Ser Leu His Val Arg Asp Thr Lys Ile Glu Val Ala Gln
            100                 105                 110

Phe Val Lys Asp Leu Leu Leu His Leu Lys Lys Leu Phe Lys Glu Gly
        115                 120                 125

Gln Phe Asn Gly Gly Gly Pro Arg Lys Pro Thr Thr Thr Pro Ala Pro
    130                 135                 140

Arg Pro Pro Thr Pro Ala Pro Thr Ile Ala Ser Gln Pro Leu Ser Leu
145                 150                 155                 160

Arg Pro Glu Ala Cys Arg Pro Ala Ala Gly Gly Ala Val His Thr Arg
                165                 170                 175

Gly Leu Asp Phe Ala Cys Asp Ile Tyr Ile Trp Ala Pro Leu Ala Gly
            180                 185                 190

Thr Cys Gly Val Leu Leu Leu Ser Leu Val Ile Thr Lys Arg Gly Arg
        195                 200                 205

Lys Lys Leu Leu Tyr Ile Phe Lys Gln Pro Phe Met Arg Pro Val Gln
    210                 215                 220

Thr Thr Gln Glu Glu Asp Gly Cys Ser Cys Arg Phe Pro Glu Glu Glu
225                 230                 235                 240

Glu Gly Gly Cys Glu Leu Lys Lys Arg Ile Lys Pro Ile Val Trp Pro
                245                 250                 255

Ser Leu Pro Asp His Lys Lys Thr Leu Glu His Leu Cys Lys Lys Pro
            260                 265                 270

Arg Lys Asn Leu Asn Val Ser Phe Asn Pro Glu Ser Phe Leu Asp Cys
        275                 280                 285

Gln Ile His Arg Val Asp Asp Ile Gln Ala Arg Asp Glu Val Glu Gly
    290                 295                 300

Phe Leu Gln Asp Thr Phe Arg Val Lys Phe Ser Arg Ser Ala Asp Ala
305                 310                 315                 320

Pro Ala Tyr Gln Gln Gly Gln Asn Gln Leu Tyr Asn Glu Leu Asn Leu
                325                 330                 335

Gly Arg Arg Glu Glu Tyr Asp Val Leu Asp Lys Arg Arg Gly Arg Asp
            340                 345                 350

Pro Glu Met Gly Gly Lys Pro Gln Arg Arg Lys Asn Pro Gln Glu Gly
        355                 360                 365

Leu Tyr Asn Glu Leu Gln Lys Asp Lys Met Ala Glu Ala Tyr Ser Glu
    370                 375                 380

Ile Gly Met Lys Gly Glu Arg Arg Arg Gly Lys Gly His Asp Gly Leu
385                 390                 395                 400

Tyr Leu Pro Gln Ser Thr Ala Thr Lys Asp Thr Tyr Asp Tyr Val Thr
                405                 410                 415

Met Gln Ala Leu Pro Pro Arg Glu Gly Arg Gly Ser Leu Leu Thr Cys
            420                 425                 430

Gly Asp Val Glu Glu Asn Pro Gly Pro Met Ala Leu Pro Val Thr Ala
        435                 440                 445

Leu Leu Leu Pro Leu Ala Leu Leu Leu His Ala Ala Arg Pro Thr Ile
    450                 455                 460

Pro Pro His Val Gln Lys Ser Val Asn Asn Asp Met Ile Val Thr Asp
465                 470                 475                 480

Asn Asn Gly Ala Val Lys Phe Pro Gln Leu Cys Lys Phe Cys Asp Val
                485                 490                 495

Arg Phe Ser Thr Cys Asp Asn Gln Lys Ser Cys Met Ser Asn Cys Ser
            500                 505                 510

Ile Thr Ser Ile Cys Glu Lys Pro Gln Glu Val Cys Val Ala Val Trp
        515                 520                 525

Arg Lys Asn Asp Glu Asn Ile Thr Leu Glu Thr Val Cys His Asp Pro
    530                 535                 540

Lys Leu Pro Tyr His Asp Phe Ile Leu Glu Asp Ala Ala Ser Pro Lys
545                 550                 555                 560

Cys Ile Met Lys Glu Lys Lys Lys Pro Gly Glu Thr Phe Phe Met Cys
                565                 570                 575

Ser Cys Ser Ser Asp Glu Cys Asn Asp Asn Ile Ile Phe Ser Glu Glu
            580                 585                 590

Tyr Asn Thr Ser Asn Pro Asp Pro Gly His Val Phe Thr Arg Gly Met
        595                 600                 605

Ile Ile Ala Val Leu Ile Leu Val Ala Val Val Cys Leu Val Thr Val
    610                 615                 620

Cys Val Ile Tyr Arg Val Asp Leu Val Leu Phe Tyr Arg His Leu Thr
625                 630                 635                 640

Arg Arg Asp Glu Thr Leu Thr Asp Gly Lys Thr Tyr Asp Ala Phe Val
                645                 650                 655

Ser Tyr Leu Lys Glu Cys Arg Pro Glu Asn Gly Glu Glu His Thr Phe
            660                 665                 670

Ala Val Glu Ile Leu Pro Arg Val Leu Glu Lys His Phe Gly Tyr Lys
        675                 680                 685

Leu Cys Ile Phe Glu Arg Asp Val Val Pro Gly Gly Ala Val Val Asp
    690                 695                 700

Glu Ile His Ser Leu Ile Glu Lys Ser Arg Arg Leu Ile Ile Val Leu
705                 710                 715                 720

Ser Lys Ser Tyr Met Ser Asn Glu Val Arg Tyr Glu Leu Glu Ser Gly
                725                 730                 735

Leu His Glu Ala Leu Val Glu Arg Lys Ile Lys Ile Ile Leu Ile Glu
            740                 745                 750

Phe Thr Pro Val Thr Asp Phe Thr Phe Leu Pro Gln Ser Leu Lys Leu
        755                 760                 765

Leu Lys Ser His Arg Val Leu Lys Trp Lys Ala Asp Lys Ser Leu Ser
    770                 775                 780

Tyr Asn Ser Arg Phe Trp Lys Asn Leu Leu Tyr Leu Met Pro Ala Lys
785                 790                 795                 800

Thr Val Lys Pro Gly Arg Asp Glu Pro Glu Val Leu Pro Val Leu Ser
                805                 810                 815

Glu Ser

<210>    25
<211>    594
<212>    PRT
<213>    Artificial Sequence

<220>
<223>    polypeptide No 5

<400>    25
Met Gly Trp Ser Cys Ile Ile Leu Phe Leu Val Ala Thr Ala Thr Gly
1               5                   10                  15

Val His Ser Gly Pro Val Pro Pro Ser Thr Ala Leu Arg Lys Leu Ile
            20                  25                  30

Glu Glu Leu Val Asn Ile Thr Gln Asn Gln Lys Ala Pro Leu Cys Asn
        35                  40                  45

Gly Ser Met Val Trp Ser Ile Asn Leu Thr Ala Gly Met Tyr Cys Ala
    50                  55                  60

Ala Leu Glu Ser Leu Ile Asn Val Ser Gly Cys Ser Ala Ile Glu Lys
65                  70                  75                  80

Thr Gln Asp Met Leu Asp Gly Phe Cys Pro His Lys Val Ser Ala Gly

85                  90                  95

Gln Phe Ser Ser Leu His Val Arg Asp Thr Lys Ile Glu Val Ala Gln
            100                 105                 110

Phe Val Lys Asp Leu Leu Leu His Leu Lys Lys Leu Phe Lys Glu Gly
        115                 120                 125

Gln Phe Asn Gly Gly Gly Pro Arg Lys Pro Thr Thr Thr Pro Ala Pro
    130                 135                 140

Arg Pro Pro Thr Pro Ala Pro Thr Ile Ala Ser Gln Pro Leu Ser Leu
145                 150                 155                 160

Arg Pro Glu Ala Cys Arg Pro Ala Ala Gly Gly Ala Val His Thr Arg
                165                 170                 175

Gly Leu Asp Phe Ala Cys Asp Ile Tyr Ile Trp Ala Pro Leu Ala Gly
            180                 185                 190

Thr Cys Gly Val Leu Leu Leu Ser Leu Val Ile Thr Lys Arg Gly Arg
        195                 200                 205

Lys Lys Leu Leu Tyr Ile Phe Lys Gln Pro Phe Met Arg Pro Val Gln
    210                 215                 220

Thr Thr Gln Glu Glu Asp Gly Cys Ser Cys Arg Phe Pro Glu Glu Glu
225                 230                 235                 240

Glu Gly Gly Cys Glu Leu Lys Lys Arg Ile Lys Pro Ile Val Trp Pro
                245                 250                 255

Ser Leu Pro Asp His Lys Lys Thr Leu Glu His Leu Cys Lys Lys Pro
            260                 265                 270

Arg Lys Asn Leu Asn Val Ser Phe Asn Pro Glu Ser Phe Leu Asp Cys
        275                 280                 285

Gln Ile His Arg Val Asp Asp Ile Gln Ala Arg Asp Glu Val Glu Gly
    290                 295                 300

Phe Leu Gln Asp Thr Phe Arg Val Lys Phe Ser Arg Ser Ala Asp Ala
305                 310                 315                 320

Pro Ala Tyr Gln Gln Gly Gln Asn Gln Leu Tyr Asn Glu Leu Asn Leu
                325                 330                 335

Gly Arg Arg Glu Glu Tyr Asp Val Leu Asp Lys Arg Arg Gly Arg Asp
            340                 345                 350

Pro Glu Met Gly Gly Lys Pro Gln Arg Arg Lys Asn Pro Gln Glu Gly
        355                 360                 365

Leu Tyr Asn Glu Leu Gln Lys Asp Lys Met Ala Glu Ala Tyr Ser Glu
    370                 375                 380

Ile Gly Met Lys Gly Glu Arg Arg Arg Gly Lys Gly His Asp Gly Leu
385                 390                 395                 400

Tyr Leu Pro Gln Ser Thr Ala Thr Lys Asp Thr Tyr Asp Tyr Val Thr
                405                 410                 415

Met Gln Ala Leu Pro Pro Arg Glu Gly Arg Gly Ser Leu Leu Thr Cys

                420                 425                 430

Gly Asp Val Glu Glu Asn Pro Gly Pro Met Tyr Arg Met Gln Leu Leu
        435                 440                 445

Ser Cys Ile Ala Leu Ser Leu Ala Leu Val Thr Asn Ser Gln Gly Gln
    450                 455                 460

Asp Arg His Met Ile Arg Met Arg Gln Leu Ile Asp Ile Val Asp Gln
465                 470                 475                 480

Leu Lys Asn Tyr Val Asn Asp Leu Val Pro Glu Phe Leu Pro Ala Pro
                485                 490                 495

Glu Asp Val Glu Thr Asn Cys Glu Trp Ser Ala Phe Ser Cys Phe Gln
            500                 505                 510

Lys Ala Gln Leu Lys Ser Ala Asn Thr Gly Asn Asn Glu Arg Ile Ile
        515                 520                 525

Asn Val Ser Ile Lys Lys Leu Lys Arg Lys Pro Pro Ser Thr Asn Ala
    530                 535                 540

Gly Arg Arg Gln Lys His Arg Leu Thr Cys Pro Ser Cys Asp Ser Tyr
545                 550                 555                 560

Glu Lys Lys Pro Pro Lys Glu Phe Leu Glu Arg Phe Lys Ser Leu Leu
                565                 570                 575

Gln Lys Met Ile His Gln His Leu Ser Ser Arg Thr His Gly Ser Glu
            580                 585                 590

Asp Ser

<210>   26
<211>   423
<212>   PRT
<213>   Artificial Sequence

<220>
<223>   polypeptide No 6

<400>   26
Met Gly Trp Ser Cys Ile Ile Leu Phe Leu Val Ala Thr Ala Thr Gly
1               5                   10                  15

Val His Ser Gly Pro Val Pro Pro Ser Thr Ala Leu Arg Lys Leu Ile
            20                  25                  30

Glu Glu Leu Val Asn Ile Thr Gln Asn Gln Lys Ala Pro Leu Cys Asn
        35                  40                  45

Gly Ser Met Val Trp Ser Ile Asn Leu Thr Ala Gly Met Tyr Cys Ala
    50                  55                  60

Ala Leu Glu Ser Leu Ile Asn Val Ser Gly Cys Ser Ala Ile Glu Lys
65                  70                  75                  80

Thr Gln Asp Met Leu Asp Gly Phe Cys Pro His Lys Val Ser Ala Gly
                85                  90                  95

Gln Phe Ser Ser Leu His Val Arg Asp Thr Lys Ile Glu Val Ala Gln
        100                 105                 110

Phe Val Lys Asp Leu Leu Leu His Leu Lys Lys Leu Phe Lys Glu Gly
    115                 120                 125

Gln Phe Asn Gly Gly Gly Pro Arg Lys Pro Thr Thr Thr Pro Ala Pro
130                 135                 140

Arg Pro Pro Thr Pro Ala Pro Thr Ile Ala Ser Gln Pro Leu Ser Leu
145                 150                 155                 160

Arg Pro Glu Ala Cys Arg Pro Ala Ala Gly Gly Ala Val His Thr Arg
                165                 170                 175

Gly Leu Asp Phe Ala Cys Asp Ile Tyr Ile Trp Ala Pro Leu Ala Gly
            180                 185                 190

Thr Cys Gly Val Leu Leu Leu Ser Leu Val Ile Thr Lys Arg Gly Arg
        195                 200                 205

Lys Lys Leu Leu Tyr Ile Phe Lys Gln Pro Phe Met Arg Pro Val Gln
    210                 215                 220

Thr Thr Gln Glu Glu Asp Gly Cys Ser Cys Arg Phe Pro Glu Glu Glu
225                 230                 235                 240

Glu Gly Gly Cys Glu Leu Lys Lys Arg Ile Lys Pro Ile Val Trp Pro
                245                 250                 255

Ser Leu Pro Asp His Lys Lys Thr Leu Glu His Leu Cys Lys Lys Pro
            260                 265                 270

Arg Lys Asn Leu Asn Val Ser Phe Asn Pro Glu Ser Phe Leu Asp Cys
        275                 280                 285

Gln Ile His Arg Val Asp Asp Ile Gln Ala Arg Asp Glu Val Glu Gly
    290                 295                 300

Phe Leu Gln Asp Thr Phe Arg Val Lys Phe Ser Arg Ser Ala Asp Ala
305                 310                 315                 320

Pro Ala Tyr Gln Gln Gly Gln Asn Gln Leu Tyr Asn Glu Leu Asn Leu
                325                 330                 335

Gly Arg Arg Glu Glu Tyr Asp Val Leu Asp Lys Arg Arg Gly Arg Asp
            340                 345                 350

Pro Glu Met Gly Gly Lys Pro Gln Arg Arg Lys Asn Pro Gln Glu Gly
        355                 360                 365

Leu Tyr Asn Glu Leu Gln Lys Asp Lys Met Ala Glu Ala Tyr Ser Glu
    370                 375                 380

Ile Gly Met Lys Gly Glu Arg Arg Arg Gly Lys Gly His Asp Gly Leu
385                 390                 395                 400

Tyr Leu Pro Gln Ser Thr Ala Thr Lys Asp Thr Tyr Asp Tyr Val Thr
                405                 410                 415

Met Gln Ala Leu Pro Pro Arg
            420

<210>    27
<211>    1038
<212>    PRT
<213>    Artificial Sequence

<220>
<223>    polypeptide No 7


<400>    27
Met Gly Trp Ser Cys Ile Ile Leu Phe Leu Val Ala Thr Ala Thr Gly
1               5                   10                  15

Val His Ser Gly Pro Val Pro Pro Ser Thr Ala Leu Arg Lys Leu Ile
            20                  25                  30

Glu Glu Leu Val Asn Ile Thr Gln Asn Gln Lys Ala Pro Leu Cys Asn
        35                  40                  45

Gly Ser Met Val Trp Ser Ile Asn Leu Thr Ala Gly Met Tyr Cys Ala
    50                  55                  60

Ala Leu Glu Ser Leu Ile Asn Val Ser Gly Cys Ser Ala Ile Glu Lys
65                  70                  75                  80

Thr Gln Asp Met Leu Asp Gly Phe Cys Pro His Lys Val Ser Ala Gly
                85                  90                  95

Gln Phe Ser Ser Leu His Val Arg Asp Thr Lys Ile Glu Val Ala Gln
            100                 105                 110

Phe Val Lys Asp Leu Leu Leu His Leu Lys Lys Leu Phe Lys Glu Gly
        115                 120                 125

Gln Phe Asn Gly Gly Gly Pro Arg Lys Pro Thr Thr Thr Pro Ala Pro
    130                 135                 140

Arg Pro Pro Thr Pro Ala Pro Thr Ile Ala Ser Gln Pro Leu Ser Leu
145                 150                 155                 160

Arg Pro Glu Ala Cys Arg Pro Ala Ala Gly Gly Ala Val His Thr Arg
                165                 170                 175

Gly Leu Asp Phe Ala Cys Asp Ile Tyr Ile Trp Ala Pro Leu Ala Gly
            180                 185                 190

Thr Cys Gly Val Leu Leu Leu Ser Leu Val Ile Thr Lys Arg Gly Arg
        195                 200                 205

Lys Lys Leu Leu Tyr Ile Phe Lys Gln Pro Phe Met Arg Pro Val Gln
    210                 215                 220

Thr Thr Gln Glu Glu Asp Gly Cys Ser Cys Arg Phe Pro Glu Glu Glu
225                 230                 235                 240

Glu Gly Gly Cys Glu Leu Asn Cys Arg Asn Thr Gly Pro Trp Leu Lys
                245                 250                 255

Lys Val Leu Lys Cys Asn Thr Pro Asp Pro Ser Lys Phe Phe Ser Gln
            260                 265                 270

Leu Ser Ser Glu His Gly Gly Asp Val Gln Lys Trp Leu Ser Ser Pro
        275                 280                 285

Phe Pro Ser Ser Ser Phe Ser Pro Gly Gly Leu Ala Pro Glu Ile Ser
    290                 295                 300

Pro Leu Glu Val Leu Glu Arg Asp Lys Val Thr Gln Leu Leu Leu Gln
305                 310                 315                 320

Gln Asp Lys Val Pro Glu Pro Ala Ser Leu Ser Ser Asn His Ser Leu
                325                 330                 335

Thr Ser Cys Phe Thr Asn Gln Gly Tyr Phe Phe Phe His Leu Arg Val
            340                 345                 350

Lys Phe Ser Arg Ser Ala Asp Ala Pro Ala Tyr Gln Gln Gly Gln Asn
        355                 360                 365

Gln Leu Tyr Asn Glu Leu Asn Leu Gly Arg Arg Glu Glu Tyr Asp Val
    370                 375                 380

Leu Asp Lys Arg Arg Gly Arg Asp Pro Glu Met Gly Gly Lys Pro Gln
385                 390                 395                 400

Arg Arg Lys Asn Pro Gln Glu Gly Leu Tyr Asn Glu Leu Gln Lys Asp
                405                 410                 415

Lys Met Ala Glu Ala Tyr Ser Glu Ile Gly Met Lys Gly Glu Arg Arg
            420                 425                 430

Arg Gly Lys Gly His Asp Gly Leu Tyr Leu Ser Leu Ser Thr Ala Thr
        435                 440                 445

Lys Asp Thr Tyr Leu Pro Gln His Met Gln Ala Leu Pro Pro Arg Glu
    450                 455                 460

Gly Arg Gly Ser Leu Leu Thr Cys Gly Asp Val Glu Glu Asn Pro Gly
465                 470                 475                 480

Pro Met Ala Leu Pro Val Thr Ala Leu Leu Leu Pro Leu Ala Leu Leu
                485                 490                 495

Leu His Ala Ala Arg Pro Thr Ile Pro Pro His Val Gln Lys Ser Val
            500                 505                 510

Asn Asn Asp Met Ile Val Thr Asp Asn Asn Gly Ala Val Lys Phe Pro
        515                 520                 525

Gln Leu Cys Lys Phe Cys Asp Val Arg Phe Ser Thr Cys Asp Asn Gln
    530                 535                 540

Lys Ser Cys Met Ser Asn Cys Ser Ile Thr Ser Ile Cys Glu Lys Pro
545                 550                 555                 560

Gln Glu Val Cys Val Ala Val Trp Arg Lys Asn Asp Glu Asn Ile Thr
                565                 570                 575

Leu Glu Thr Val Cys His Asp Pro Lys Leu Pro Tyr His Asp Phe Ile
            580                 585                 590

Leu Glu Asp Ala Ala Ser Pro Lys Cys Ile Met Lys Glu Lys Lys Lys
        595                 600                 605

Pro Gly Glu Thr Phe Phe Met Cys Ser Cys Ser Ser Asp Glu Cys Asn
    610                 615                 620

Asp Asn Ile Ile Phe Ser Glu Glu Tyr Asn Thr Ser Asn Pro Asp Pro
625                 630                 635                 640

Gly His Val Phe Thr Arg Gly Met Ile Ile Ala Val Leu Ile Leu Val
                645                 650                 655

Ala Val Val Cys Leu Val Thr Val Cys Val Ile Tyr Arg Val Asp Leu
            660                 665                 670

Val Leu Phe Tyr Arg His Leu Thr Arg Arg Asp Glu Thr Leu Thr Asp
        675                 680                 685

Gly Lys Thr Tyr Asp Ala Phe Val Ser Tyr Leu Lys Glu Cys Arg Pro
    690                 695                 700

Glu Asn Gly Glu Glu His Thr Phe Ala Val Glu Ile Leu Pro Arg Val
705                 710                 715                 720

Leu Glu Lys His Phe Gly Tyr Lys Leu Cys Ile Phe Glu Arg Asp Val
                725                 730                 735

Val Pro Gly Gly Ala Val Val Asp Glu Ile His Ser Leu Ile Glu Lys
            740                 745                 750

Ser Arg Arg Leu Ile Ile Val Leu Ser Lys Ser Tyr Met Ser Asn Glu
        755                 760                 765

Val Arg Tyr Glu Leu Glu Ser Gly Leu His Glu Ala Leu Val Glu Arg
    770                 775                 780

Lys Ile Lys Ile Ile Leu Ile Glu Phe Thr Pro Val Thr Asp Phe Thr
785                 790                 795                 800

Phe Leu Pro Gln Ser Leu Lys Leu Leu Lys Ser His Arg Val Leu Lys
                805                 810                 815

Trp Lys Ala Asp Lys Ser Leu Ser Tyr Asn Ser Arg Phe Trp Lys Asn
            820                 825                 830

Leu Leu Tyr Leu Met Pro Ala Lys Thr Val Lys Pro Gly Arg Asp Glu
        835                 840                 845

Pro Glu Val Leu Pro Val Leu Ser Glu Ser Arg Arg Lys Arg Ser Gly
    850                 855                 860

Ser Gly Ala Thr Asn Phe Ser Leu Leu Lys Gln Ala Gly Asp Val Glu
865                 870                 875                 880

Glu Asn Pro Gly Pro Met Tyr Arg Met Gln Leu Leu Ser Cys Ile Ala
                885                 890                 895

Leu Ser Leu Ala Leu Val Thr Asn Ser Gln Gly Gln Asp Arg His Met
            900                 905                 910

Ile Arg Met Arg Gln Leu Ile Asp Ile Val Asp Gln Leu Lys Asn Tyr
        915                 920                 925

Val Asn Asp Leu Val Pro Glu Phe Leu Pro Ala Pro Glu Asp Val Glu
    930                 935                 940

Thr Asn Cys Glu Trp Ser Ala Phe Ser Cys Phe Gln Lys Ala Gln Leu
945                 950                 955                 960

Lys Ser Ala Asn Thr Gly Asn Asn Glu Arg Ile Ile Asn Val Ser Ile
                965                 970                 975

Lys Lys Leu Lys Arg Lys Pro Pro Ser Thr Asn Ala Gly Arg Arg Gln
            980                 985                 990

Lys His Arg Leu Thr Cys Pro Ser Cys Asp Ser Tyr Glu Lys Lys Pro
        995                 1000                1005

Pro Lys Glu Phe Leu Glu Arg Phe Lys Ser Leu Leu Gln Lys Met Ile
    1010                1015                1020

His Gln His Leu Ser Ser Arg Thr His Gly Ser Glu Asp Ser
1025                1030                1035


<210>   28
<211>   858
<212>   PRT
<213>   Artificial Sequence

<220>
<223>   polypeptide No 8


<400>   28
Met Gly Trp Ser Cys Ile Ile Leu Phe Leu Val Ala Thr Ala Thr Gly
1               5                   10                  15

Val His Ser Gly Pro Val Pro Pro Ser Thr Ala Leu Arg Lys Leu Ile
            20                  25                  30

Glu Glu Leu Val Asn Ile Thr Gln Asn Gln Lys Ala Pro Leu Cys Asn
        35                  40                  45

Gly Ser Met Val Trp Ser Ile Asn Leu Thr Ala Gly Met Tyr Cys Ala
    50                  55                  60

Ala Leu Glu Ser Leu Ile Asn Val Ser Gly Cys Ser Ala Ile Glu Lys
65                  70                  75                  80

Thr Gln Asp Met Leu Asp Gly Phe Cys Pro His Lys Val Ser Ala Gly
                85                  90                  95

Gln Phe Ser Ser Leu His Val Arg Asp Thr Lys Ile Glu Val Ala Gln
            100                 105                 110

Phe Val Lys Asp Leu Leu Leu His Leu Lys Lys Leu Phe Lys Glu Gly
        115                 120                 125

Gln Phe Asn Gly Gly Gly Pro Arg Lys Pro Thr Thr Thr Pro Ala Pro
    130                 135                 140

Arg Pro Pro Thr Pro Ala Pro Thr Ile Ala Ser Gln Pro Leu Ser Leu
145                 150                 155                 160

Arg Pro Glu Ala Cys Arg Pro Ala Ala Gly Gly Ala Val His Thr Arg
                165                 170                 175

Gly Leu Asp Phe Ala Cys Asp Ile Tyr Ile Trp Ala Pro Leu Ala Gly

180                 185                 190

Thr Cys Gly Val Leu Leu Leu Ser Leu Val Ile Thr Lys Arg Gly Arg
        195                 200                 205

Lys Lys Leu Leu Tyr Ile Phe Lys Gln Pro Phe Met Arg Pro Val Gln
    210                 215                 220

Thr Thr Gln Glu Glu Asp Gly Cys Ser Cys Arg Phe Pro Glu Glu Glu
225                 230                 235                 240

Glu Gly Gly Cys Glu Leu Asn Cys Arg Asn Thr Gly Pro Trp Leu Lys
                245                 250                 255

Lys Val Leu Lys Cys Asn Thr Pro Asp Pro Ser Lys Phe Phe Ser Gln
            260                 265                 270

Leu Ser Ser Glu His Gly Gly Asp Val Gln Lys Trp Leu Ser Ser Pro
        275                 280                 285

Phe Pro Ser Ser Ser Phe Ser Pro Gly Gly Leu Ala Pro Glu Ile Ser
    290                 295                 300

Pro Leu Glu Val Leu Glu Arg Asp Lys Val Thr Gln Leu Leu Leu Gln
305                 310                 315                 320

Gln Asp Lys Val Pro Glu Pro Ala Ser Leu Ser Ser Asn His Ser Leu
                325                 330                 335

Thr Ser Cys Phe Thr Asn Gln Gly Tyr Phe Phe Phe His Leu Arg Val
            340                 345                 350

Lys Phe Ser Arg Ser Ala Asp Ala Pro Ala Tyr Gln Gln Gly Gln Asn
        355                 360                 365

Gln Leu Tyr Asn Glu Leu Asn Leu Gly Arg Arg Glu Glu Tyr Asp Val
    370                 375                 380

Leu Asp Lys Arg Arg Gly Arg Asp Pro Glu Met Gly Gly Lys Pro Gln
385                 390                 395                 400

Arg Arg Lys Asn Pro Gln Glu Gly Leu Tyr Asn Glu Leu Gln Lys Asp
                405                 410                 415

Lys Met Ala Glu Ala Tyr Ser Glu Ile Gly Met Lys Gly Glu Arg Arg
            420                 425                 430

Arg Gly Lys Gly His Asp Gly Leu Tyr Leu Ser Leu Ser Thr Ala Thr
        435                 440                 445

Lys Asp Thr Tyr Leu Pro Gln His Met Gln Ala Leu Pro Pro Arg Glu
    450                 455                 460

Gly Arg Gly Ser Leu Leu Thr Cys Gly Asp Val Glu Glu Asn Pro Gly
465                 470                 475                 480

Pro Met Ala Leu Pro Val Thr Ala Leu Leu Leu Pro Leu Ala Leu Leu
                485                 490                 495

Leu His Ala Ala Arg Pro Thr Ile Pro Pro His Val Gln Lys Ser Val
            500                 505                 510

Asn Asn Asp Met Ile Val Thr Asp Asn Asn Gly Ala Val Lys Phe Pro

515                 520                 525

Gln Leu Cys Lys Phe Cys Asp Val Arg Phe Ser Thr Cys Asp Asn Gln
    530                 535                 540

Lys Ser Cys Met Ser Asn Cys Ser Ile Thr Ser Ile Cys Glu Lys Pro
545                 550                 555                 560

Gln Glu Val Cys Val Ala Val Trp Arg Lys Asn Asp Glu Asn Ile Thr
                565                 570                 575

Leu Glu Thr Val Cys His Asp Pro Lys Leu Pro Tyr His Asp Phe Ile
            580                 585                 590

Leu Glu Asp Ala Ala Ser Pro Lys Cys Ile Met Lys Glu Lys Lys Lys
        595                 600                 605

Pro Gly Glu Thr Phe Phe Met Cys Ser Cys Ser Ser Asp Glu Cys Asn
    610                 615                 620

Asp Asn Ile Ile Phe Ser Glu Glu Tyr Asn Thr Ser Asn Pro Asp Pro
625                 630                 635                 640

Gly His Val Phe Thr Arg Gly Met Ile Ile Ala Val Leu Ile Leu Val
                645                 650                 655

Ala Val Val Cys Leu Val Thr Val Cys Val Ile Tyr Arg Val Asp Leu
            660                 665                 670

Val Leu Phe Tyr Arg His Leu Thr Arg Arg Asp Glu Thr Leu Thr Asp
        675                 680                 685

Gly Lys Thr Tyr Asp Ala Phe Val Ser Tyr Leu Lys Glu Cys Arg Pro
    690                 695                 700

Glu Asn Gly Glu Glu His Thr Phe Ala Val Glu Ile Leu Pro Arg Val
705                 710                 715                 720

Leu Glu Lys His Phe Gly Tyr Lys Leu Cys Ile Phe Glu Arg Asp Val
                725                 730                 735

Val Pro Gly Gly Ala Val Val Asp Glu Ile His Ser Leu Ile Glu Lys
            740                 745                 750

Ser Arg Arg Leu Ile Ile Val Leu Ser Lys Ser Tyr Met Ser Asn Glu
        755                 760                 765

Val Arg Tyr Glu Leu Glu Ser Gly Leu His Glu Ala Leu Val Glu Arg
    770                 775                 780

Lys Ile Lys Ile Ile Leu Ile Glu Phe Thr Pro Val Thr Asp Phe Thr
785                 790                 795                 800

Phe Leu Pro Gln Ser Leu Lys Leu Leu Lys Ser His Arg Val Leu Lys
                805                 810                 815

Trp Lys Ala Asp Lys Ser Leu Ser Tyr Asn Ser Arg Phe Trp Lys Asn
            820                 825                 830

Leu Leu Tyr Leu Met Pro Ala Lys Thr Val Lys Pro Gly Arg Asp Glu
        835                 840                 845

Pro Glu Val Leu Pro Val Leu Ser Glu Ser

850                 855

<210>    29
<211>    634
<212>    PRT
<213>    Artificial Sequence

<220>
<223>    polypeptide No 11

<400>    29
Met Gly Trp Ser Cys Ile Ile Leu Phe Leu Val Ala Thr Ala Thr Gly
1               5                   10                  15

Val His Ser Gly Pro Val Pro Pro Ser Thr Ala Leu Arg Lys Leu Ile
            20                  25                  30

Glu Glu Leu Val Asn Ile Thr Gln Asn Gln Lys Ala Pro Leu Cys Asn
        35                  40                  45

Gly Ser Met Val Trp Ser Ile Asn Leu Thr Ala Gly Met Tyr Cys Ala
    50                  55                  60

Ala Leu Glu Ser Leu Ile Asn Val Ser Gly Cys Ser Ala Ile Glu Lys
65                  70                  75                  80

Thr Gln Asp Met Leu Asp Gly Phe Cys Pro His Lys Val Ser Ala Gly
                85                  90                  95

Gln Phe Ser Ser Leu His Val Arg Asp Thr Lys Ile Glu Val Ala Gln
            100                 105                 110

Phe Val Lys Asp Leu Leu Leu His Leu Lys Lys Leu Phe Lys Glu Gly
        115                 120                 125

Gln Phe Asn Gly Gly Gly Pro Arg Lys Pro Thr Thr Thr Pro Ala Pro
    130                 135                 140

Arg Pro Pro Thr Pro Ala Pro Thr Ile Ala Ser Gln Pro Leu Ser Leu
145                 150                 155                 160

Arg Pro Glu Ala Cys Arg Pro Ala Ala Gly Gly Ala Val His Thr Arg
                165                 170                 175

Gly Leu Asp Phe Ala Cys Asp Ile Tyr Ile Trp Ala Pro Leu Ala Gly
            180                 185                 190

Thr Cys Gly Val Leu Leu Leu Ser Leu Val Ile Thr Lys Arg Gly Arg
        195                 200                 205

Lys Lys Leu Leu Tyr Ile Phe Lys Gln Pro Phe Met Arg Pro Val Gln
    210                 215                 220

Thr Thr Gln Glu Glu Asp Gly Cys Ser Cys Arg Phe Pro Glu Glu Glu
225                 230                 235                 240

Glu Gly Gly Cys Glu Leu Asn Cys Arg Asn Thr Gly Pro Trp Leu Lys
                245                 250                 255

Lys Val Leu Lys Cys Asn Thr Pro Asp Pro Ser Lys Phe Phe Ser Gln
            260                 265                 270

Leu Ser Ser Glu His Gly Gly Asp Val Gln Lys Trp Leu Ser Ser Pro
        275                 280                 285

Phe Pro Ser Ser Ser Phe Ser Pro Gly Gly Leu Ala Pro Glu Ile Ser
    290                 295                 300

Pro Leu Glu Val Leu Glu Arg Asp Lys Val Thr Gln Leu Leu Leu Gln
305                 310                 315                 320

Gln Asp Lys Val Pro Glu Pro Ala Ser Leu Ser Ser Asn His Ser Leu
                325                 330                 335

Thr Ser Cys Phe Thr Asn Gln Gly Tyr Phe Phe Phe His Leu Arg Val
            340                 345                 350

Lys Phe Ser Arg Ser Ala Asp Ala Pro Ala Tyr Gln Gln Gly Gln Asn
        355                 360                 365

Gln Leu Tyr Asn Glu Leu Asn Leu Gly Arg Arg Glu Glu Tyr Asp Val
    370                 375                 380

Leu Asp Lys Arg Arg Gly Arg Asp Pro Glu Met Gly Gly Lys Pro Gln
385                 390                 395                 400

Arg Arg Lys Asn Pro Gln Glu Gly Leu Tyr Asn Glu Leu Gln Lys Asp
                405                 410                 415

Lys Met Ala Glu Ala Tyr Ser Glu Ile Gly Met Lys Gly Glu Arg Arg
            420                 425                 430

Arg Gly Lys Gly His Asp Gly Leu Tyr Leu Ser Leu Ser Thr Ala Thr
        435                 440                 445

Lys Asp Thr Tyr Leu Pro Gln His Met Gln Ala Leu Pro Pro Arg Glu
    450                 455                 460

Gly Arg Gly Ser Leu Leu Thr Cys Gly Asp Val Glu Glu Asn Pro Gly
465                 470                 475                 480

Pro Met Tyr Arg Met Gln Leu Leu Ser Cys Ile Ala Leu Ser Leu Ala
                485                 490                 495

Leu Val Thr Asn Ser Gln Gly Gln Asp Arg His Met Ile Arg Met Arg
            500                 505                 510

Gln Leu Ile Asp Ile Val Asp Gln Leu Lys Asn Tyr Val Asn Asp Leu
        515                 520                 525

Val Pro Glu Phe Leu Pro Ala Pro Glu Asp Val Glu Thr Asn Cys Glu
    530                 535                 540

Trp Ser Ala Phe Ser Cys Phe Gln Lys Ala Gln Leu Lys Ser Ala Asn
545                 550                 555                 560

Thr Gly Asn Asn Glu Arg Ile Ile Asn Val Ser Ile Lys Lys Leu Lys
                565                 570                 575

Arg Lys Pro Pro Ser Thr Asn Ala Gly Arg Arg Gln Lys His Arg Leu
            580                 585                 590

Thr Cys Pro Ser Cys Asp Ser Tyr Glu Lys Lys Pro Pro Lys Glu Phe
        595                 600                 605

Leu Glu Arg Phe Lys Ser Leu Leu Gln Lys Met Ile His Gln His Leu
    610                 615                 620

Ser Ser Arg Thr His Gly Ser Glu Asp Ser
625                 630


<210>    30
<211>    463
<212>    PRT
<213>    Artificial Sequence

<220>
<223>    polypeptide No 12


<400>    30
Met Gly Trp Ser Cys Ile Ile Leu Phe Leu Val Ala Thr Ala Thr Gly
  1               5                  10                  15

Val His Ser Gly Pro Val Pro Pro Ser Thr Ala Leu Arg Lys Leu Ile
             20                  25                  30

Glu Glu Leu Val Asn Ile Thr Gln Asn Gln Lys Ala Pro Leu Cys Asn
         35                  40                  45

Gly Ser Met Val Trp Ser Ile Asn Leu Thr Ala Gly Met Tyr Cys Ala
    50                  55                  60

Ala Leu Glu Ser Leu Ile Asn Val Ser Gly Cys Ser Ala Ile Glu Lys
 65                  70                  75                  80

Thr Gln Asp Met Leu Asp Gly Phe Cys Pro His Lys Val Ser Ala Gly
                 85                  90                  95

Gln Phe Ser Ser Leu His Val Arg Asp Thr Lys Ile Glu Val Ala Gln
            100                 105                 110

Phe Val Lys Asp Leu Leu Leu His Leu Lys Lys Leu Phe Lys Glu Gly
        115                 120                 125

Gln Phe Asn Gly Gly Gly Pro Arg Lys Pro Thr Thr Thr Pro Ala Pro
    130                 135                 140

Arg Pro Pro Thr Pro Ala Pro Thr Ile Ala Ser Gln Pro Leu Ser Leu
145                 150                 155                 160

Arg Pro Glu Ala Cys Arg Pro Ala Ala Gly Gly Ala Val His Thr Arg
                165                 170                 175

Gly Leu Asp Phe Ala Cys Asp Ile Tyr Ile Trp Ala Pro Leu Ala Gly
            180                 185                 190

Thr Cys Gly Val Leu Leu Leu Ser Leu Val Ile Thr Lys Arg Gly Arg
        195                 200                 205

Lys Lys Leu Leu Tyr Ile Phe Lys Gln Pro Phe Met Arg Pro Val Gln
    210                 215                 220

Thr Thr Gln Glu Glu Asp Gly Cys Ser Cys Arg Phe Pro Glu Glu Glu
225                 230                 235                 240

Glu Gly Gly Cys Glu Leu Asn Cys Arg Asn Thr Gly Pro Trp Leu Lys
                245                 250                 255

Lys Val Leu Lys Cys Asn Thr Pro Asp Pro Ser Lys Phe Phe Ser Gln
            260                 265                 270

Leu Ser Ser Glu His Gly Gly Asp Val Gln Lys Trp Leu Ser Ser Pro
        275                 280                 285

Phe Pro Ser Ser Ser Phe Ser Pro Gly Gly Leu Ala Pro Glu Ile Ser
    290                 295                 300

Pro Leu Glu Val Leu Glu Arg Asp Lys Val Thr Gln Leu Leu Leu Gln
305                 310                 315                 320

Gln Asp Lys Val Pro Glu Pro Ala Ser Leu Ser Ser Asn His Ser Leu
                325                 330                 335

Thr Ser Cys Phe Thr Asn Gln Gly Tyr Phe Phe Phe His Leu Arg Val
            340                 345                 350

Lys Phe Ser Arg Ser Ala Asp Ala Pro Ala Tyr Gln Gln Gly Gln Asn
        355                 360                 365

Gln Leu Tyr Asn Glu Leu Asn Leu Gly Arg Arg Glu Glu Tyr Asp Val
    370                 375                 380

Leu Asp Lys Arg Arg Gly Arg Asp Pro Glu Met Gly Gly Lys Pro Gln
385                 390                 395                 400

Arg Arg Lys Asn Pro Gln Glu Gly Leu Tyr Asn Glu Leu Gln Lys Asp
                405                 410                 415

Lys Met Ala Glu Ala Tyr Ser Glu Ile Gly Met Lys Gly Glu Arg Arg
            420                 425                 430

Arg Gly Lys Gly His Asp Gly Leu Tyr Leu Ser Leu Ser Thr Ala Thr
        435                 440                 445

Lys Asp Thr Tyr Leu Pro Gln His Met Gln Ala Leu Pro Pro Arg
    450                 455                 460

<210>    31
<211>    23
<212>    PRT
<213>    Artificial Sequence

<220>
<223>    mutated 3rd ITAM 1

<400>    31
Tyr Leu Pro Gln Ser Thr Ala Thr Lys Asp Thr Tyr Asp Tyr Val Thr
1               5                   10                  15

Met Gln Ala Leu Pro Pro Arg
            20

<210>    32

<211>    23
<212>    PRT
<213>    Artificial Sequence

<220>
<223>    mutated 3rd ITAM 2


<400>    32
Tyr Leu Ser Leu Ser Thr Ala Thr Lys Asp Thr Tyr Leu Pro Gln His
1               5                   10                  15

Met Gln Ala Leu Pro Pro Arg
            20


<210>    33
<211>    252
<212>    PRT
<213>    Artificial Sequence

<220>
<223>    antigen binding domain binding to EGFRvIII


<400>    33
Gln Val Gln Leu Gln Glu Ser Gly Gly Gly Leu Val Lys Pro Gly Gly
1               5                   10                  15

Ser Leu Lys Leu Ser Cys Ala Ala Ser Gly Phe Thr Phe Ser Lys Phe
            20                  25                  30

Gly Met Ser Trp Val Arg Gln Thr Pro Asp Lys Arg Leu Glu Trp Val
        35                  40                  45

Ala Ser Ile Asp Pro Glu Asn Asp Glu Thr Phe Tyr Ser Asp Asn Val
    50                  55                  60

Lys Gly Arg Phe Thr Ile Ser Arg Asp Asn Ala Lys Asn Thr Leu Tyr
65                  70                  75                  80

Leu Gln Met Ser Ser Leu Lys Ser Glu Asp Thr Ala Met Tyr Tyr Cys
                85                  90                  95

Ala Arg Gly Tyr Gly Ser Thr Ser Phe Ala Met Asp Tyr Trp Gly Gln
            100                 105                 110

Gly Thr Met Val Thr Val Ser Ser Gly Ser Thr Ser Gly Ser Gly Lys
        115                 120                 125

Pro Gly Ser Gly Glu Gly Ser Asp Ile Gln Met Thr Gln Ser Pro Ser
    130                 135                 140

Ser Leu Ser Ala Ser Val Gly Asp Arg Val Thr Ile Thr Cys Met Thr
145                 150                 155                 160

Ser Thr Asp Ile Asp Asp Asp Met Asn Trp Tyr Gln Gln Lys Pro Gly
                165                 170                 175

Lys Thr Pro Lys Leu Leu Ile Tyr Glu Gly Asn Thr Leu Arg Pro Gly
            180                 185                 190

Val Pro Ser Arg Phe Ser Gly Ser Gly Ser Gly Thr Asp Phe Ile Phe

195                 200                 205

Thr Ile Ser Ser Leu Gln Pro Glu Asp Ile Ala Thr Tyr Tyr Cys Leu
    210                 215                 220

Gln Ser Phe Asn Gly Pro Leu Thr Phe Gly Gly Gly Thr Lys Val Glu
225                 230                 235                 240

Ile Lys Glu Gln Lys Leu Ile Ser Glu Glu Asp Leu
                245                 250


<210>    34
<211>    499
<212>    PRT
<213>    Artificial Sequence

<220>
<223>    polypeptide No 13


<400>    34
Met Ala Leu Pro Val Thr Ala Leu Leu Leu Pro Leu Ala Leu Leu Leu
1               5                   10                  15

His Ala Ala Arg Pro Gln Val Gln Leu Gln Glu Ser Gly Gly Gly Leu
            20                  25                  30

Val Lys Pro Gly Gly Ser Leu Lys Leu Ser Cys Ala Ala Ser Gly Phe
        35                  40                  45

Thr Phe Ser Lys Phe Gly Met Ser Trp Val Arg Gln Thr Pro Asp Lys
    50                  55                  60

Arg Leu Glu Trp Val Ala Ser Ile Ser Thr Gly Gly Tyr Asn Thr Phe
65                  70                  75                  80

Tyr Ser Asp Asn Val Lys Gly Arg Phe Thr Ile Ser Arg Asp Asn Ala
                85                  90                  95

Lys Asn Thr Leu Tyr Leu Gln Met Ser Ser Leu Lys Ser Glu Asp Thr
            100                 105                 110

Ala Met Tyr Tyr Cys Ala Arg Gly Tyr Ser Ser Thr Ser Phe Ala Met
        115                 120                 125

Asp Tyr Trp Gly Gln Gly Thr Met Val Thr Val Ser Ser Gly Ser Thr
    130                 135                 140

Ser Gly Ser Gly Lys Pro Gly Ser Gly Glu Gly Ser Asp Ile Gln Met
145                 150                 155                 160

Thr Gln Ser Pro Ser Ser Leu Ser Ala Ser Val Gly Asp Arg Val Thr
                165                 170                 175

Ile Thr Cys Met Thr Ser Thr Asp Ile Asp Asp Asp Met Asn Trp Tyr
            180                 185                 190

Gln Gln Lys Pro Gly Lys Thr Pro Lys Leu Leu Ile Tyr Glu Gly Asn
        195                 200                 205

Thr Leu Arg Pro Gly Val Pro Ser Arg Phe Ser Gly Ser Gly Ser Gly
    210                 215                 220

Thr Asp Phe Ile Phe Thr Ile Ser Ser Leu Gln Pro Glu Asp Ile Ala
225                 230                 235                 240

Thr Tyr Tyr Cys Leu Gln Ser Phe Asn Val Pro Leu Thr Phe Gly Gly
                245                 250                 255

Gly Thr Lys Val Glu Ile Lys Glu Gln Lys Leu Ile Ser Glu Glu Asp
            260                 265                 270

Leu Gly Gly Gly Pro Arg Lys Pro Thr Thr Thr Pro Ala Pro Arg Pro
        275                 280                 285

Pro Thr Pro Ala Pro Thr Ile Ala Ser Gln Pro Leu Ser Leu Arg Pro
    290                 295                 300

Glu Ala Ala Arg Pro Ala Ala Gly Gly Ala Val His Thr Arg Gly Leu
305                 310                 315                 320

Asp Phe Ala Leu Ala Tyr Leu Leu Asp Gly Ile Leu Phe Ile Tyr Gly
                325                 330                 335

Val Ile Leu Thr Ala Leu Phe Leu Arg Val Lys Arg Gly Arg Lys Lys
            340                 345                 350

Leu Leu Tyr Ile Phe Lys Gln Pro Phe Met Arg Pro Val Gln Thr Thr
        355                 360                 365

Gln Glu Glu Asp Gly Cys Ser Cys Arg Phe Pro Glu Glu Glu Glu Gly
    370                 375                 380

Gly Cys Glu Leu Lys Phe Ser Arg Ser Ala Asp Ala Pro Ala Tyr Gln
385                 390                 395                 400

Gln Gly Gln Asn Gln Leu Tyr Asn Glu Leu Asn Leu Gly Arg Arg Glu
                405                 410                 415

Glu Tyr Asp Val Leu Asp Lys Arg Arg Gly Arg Asp Pro Glu Met Gly
            420                 425                 430

Gly Lys Pro Gln Arg Arg Lys Asn Pro Gln Glu Gly Leu Tyr Asn Glu
        435                 440                 445

Leu Gln Lys Asp Lys Met Ala Glu Ala Tyr Ser Glu Ile Gly Met Lys
    450                 455                 460

Gly Glu Arg Arg Arg Gly Lys Gly His Asp Gly Leu Tyr Gln Gly Leu
465                 470                 475                 480

Ser Thr Ala Thr Lys Asp Thr Tyr Asp Ala Leu His Met Gln Ala Leu
                485                 490                 495

Pro Pro Arg



<210>   35
<211>   499
<212>   PRT
<213>   Artificial Sequence

<220>
<223>   polypeptide No 14

<400>    35
Met Ala Leu Pro Val Thr Ala Leu Leu Leu Pro Leu Ala Leu Leu Leu
1               5                   10                  15

His Ala Ala Arg Pro Gln Val Gln Leu Gln Glu Ser Gly Gly Gly Leu
            20                  25                  30

Val Lys Pro Gly Gly Ser Leu Lys Leu Ser Cys Ala Ala Ser Gly Phe
        35                  40                  45

Thr Phe Ser Lys Phe Gly Met Ser Trp Val Arg Gln Thr Pro Asp Lys
    50                  55                  60

Arg Leu Glu Trp Val Ala Ser Ile Asp Pro Glu Asn Asp Glu Thr Phe
65                  70                  75                  80

Tyr Ser Asp Asn Val Lys Gly Arg Phe Thr Ile Ser Arg Asp Asn Ala
                85                  90                  95

Lys Asn Thr Leu Tyr Leu Gln Met Ser Ser Leu Lys Ser Glu Asp Thr
            100                 105                 110

Ala Met Tyr Tyr Cys Ala Arg Gly Tyr Gly Ser Thr Ser Phe Ala Met
        115                 120                 125

Asp Tyr Trp Gly Gln Gly Thr Met Val Thr Val Ser Ser Gly Ser Thr
    130                 135                 140

Ser Gly Ser Gly Lys Pro Gly Ser Gly Glu Gly Ser Asp Ile Gln Met
145                 150                 155                 160

Thr Gln Ser Pro Ser Ser Leu Ser Ala Ser Val Gly Asp Arg Val Thr
                165                 170                 175

Ile Thr Cys Met Thr Ser Thr Asp Ile Asp Asp Asp Met Asn Trp Tyr
            180                 185                 190

Gln Gln Lys Pro Gly Lys Thr Pro Lys Leu Leu Ile Tyr Glu Gly Asn
        195                 200                 205

Thr Leu Arg Pro Gly Val Pro Ser Arg Phe Ser Gly Ser Gly Ser Gly
    210                 215                 220

Thr Asp Phe Ile Phe Thr Ile Ser Ser Leu Gln Pro Glu Asp Ile Ala
225                 230                 235                 240

Thr Tyr Tyr Cys Leu Gln Ser Phe Asn Gly Pro Leu Thr Phe Gly Gly
                245                 250                 255

Gly Thr Lys Val Glu Ile Lys Glu Gln Lys Leu Ile Ser Glu Glu Asp
            260                 265                 270

Leu Gly Gly Gly Pro Arg Lys Pro Thr Thr Thr Pro Ala Pro Arg Pro
        275                 280                 285

Pro Thr Pro Ala Pro Thr Ile Ala Ser Gln Pro Leu Ser Leu Arg Pro
    290                 295                 300

Glu Ala Ala Arg Pro Ala Ala Gly Gly Ala Val His Thr Arg Gly Leu
305                 310                 315                 320

Asp Phe Ala Leu Ala Tyr Leu Leu Asp Gly Ile Leu Phe Ile Tyr Gly
                325                 330                 335

Val Ile Leu Thr Ala Leu Phe Leu Arg Val Lys Arg Gly Arg Lys Lys
            340                 345                 350

Leu Leu Tyr Ile Phe Lys Gln Pro Phe Met Arg Pro Val Gln Thr Thr
        355                 360                 365

Gln Glu Glu Asp Gly Cys Ser Cys Arg Phe Pro Glu Glu Glu Glu Gly
    370                 375                 380

Gly Cys Glu Leu Lys Phe Ser Arg Ser Ala Asp Ala Pro Ala Tyr Gln
385                 390                 395                 400

Gln Gly Gln Asn Gln Leu Tyr Asn Glu Leu Asn Leu Gly Arg Arg Glu
                405                 410                 415

Glu Tyr Asp Val Leu Asp Lys Arg Arg Gly Arg Asp Pro Glu Met Gly
            420                 425                 430

Gly Lys Pro Gln Arg Arg Lys Asn Pro Gln Glu Gly Leu Tyr Asn Glu
        435                 440                 445

Leu Gln Lys Asp Lys Met Ala Glu Ala Tyr Ser Glu Ile Gly Met Lys
    450                 455                 460

Gly Glu Arg Arg Arg Gly Lys Gly His Asp Gly Leu Tyr Gln Gly Leu
465                 470                 475                 480

Ser Thr Ala Thr Lys Asp Thr Tyr Asp Ala Leu His Met Gln Ala Leu
                485                 490                 495

Pro Pro Arg



<210>    36
<211>    349
<212>    PRT
<213>    Artificial Sequence

<220>
<223>    polypeptide No 15


<400>    36
Met Gly Val Lys Val Leu Phe Ala Leu Ile Cys Ile Ala Val Ala Glu
1               5                   10                  15

Ala Val Ser Asp Val Pro Arg Asp Leu Glu Val Val Ala Ala Thr Pro
            20                  25                  30

Thr Ser Leu Leu Ile Ser Trp Asp Ala Pro Ala Val Thr Val Arg Tyr
        35                  40                  45

Tyr Arg Ile Thr Tyr Gly Glu Thr Gly Gly Asn Ser Pro Val Gln Glu
    50                  55                  60

Phe Thr Val Pro Gly Ser Lys Ser Thr Ala Thr Ile Ser Gly Leu Lys
65                  70                  75                  80

Pro Gly Val Asp Tyr Thr Ile Thr Val Tyr Ala Val Thr Pro Arg Gly

85                  90                  95

Asp Trp Asn Glu Gly Ser Lys Pro Ile Ser Ile Asn Tyr Arg Thr Glu
            100                 105                 110

Gln Lys Leu Ile Ser Glu Glu Asp Leu Gly Gly Gly Pro Arg Lys Pro
        115                 120                 125

Thr Thr Thr Pro Ala Pro Arg Pro Pro Thr Pro Ala Pro Thr Ile Ala
    130                 135                 140

Ser Gln Pro Leu Ser Leu Arg Pro Glu Ala Cys Arg Pro Ala Ala Gly
145                 150                 155                 160

Gly Ala Val His Thr Arg Gly Leu Asp Phe Ala Cys Asp Ile Tyr Ile
                165                 170                 175

Trp Ala Pro Leu Ala Gly Thr Cys Gly Val Leu Leu Leu Ser Leu Val
            180                 185                 190

Ile Thr Lys Arg Gly Arg Lys Lys Leu Leu Tyr Ile Phe Lys Gln Pro
        195                 200                 205

Phe Met Arg Pro Val Gln Thr Thr Gln Glu Glu Asp Gly Cys Ser Cys
    210                 215                 220

Arg Phe Pro Glu Glu Glu Glu Gly Gly Cys Glu Leu Arg Val Lys Phe
225                 230                 235                 240

Ser Arg Ser Ala Asp Ala Pro Ala Tyr Gln Gln Gly Gln Asn Gln Leu
                245                 250                 255

Tyr Asn Glu Leu Asn Leu Gly Arg Arg Glu Glu Tyr Asp Val Leu Asp
            260                 265                 270

Lys Arg Arg Gly Arg Asp Pro Glu Met Gly Gly Lys Pro Gln Arg Arg
        275                 280                 285

Lys Asn Pro Gln Glu Gly Leu Tyr Asn Glu Leu Gln Lys Asp Lys Met
    290                 295                 300

Ala Glu Ala Tyr Ser Glu Ile Gly Met Lys Gly Glu Arg Arg Arg Gly
305                 310                 315                 320

Lys Gly His Asp Gly Leu Tyr Gln Gly Leu Ser Thr Ala Thr Lys Asp
                325                 330                 335

Thr Tyr Asp Ala Leu His Met Gln Ala Leu Pro Pro Arg
            340                 345


<210>   37
<211>   353
<212>   PRT
<213>   Artificial Sequence

<220>
<223>   polypeptide No 16


<400>   37
Met Ala Leu Pro Val Thr Ala Leu Leu Leu Pro Leu Ala Leu Leu Leu
1               5                   10                  15

His Ala Ala Arg Pro Val Ser Asp Val Pro Arg Asp Leu Glu Val Val
            20                  25                  30

Ala Ala Thr Pro Thr Ser Leu Leu Ile Ser Trp Asp Ala Pro Ala Val
        35                  40                  45

Thr Val Arg Tyr Tyr Arg Ile Thr Tyr Gly Glu Thr Gly Gly Asn Ser
    50                  55                  60

Pro Val Gln Glu Phe Thr Val Pro Gly Ser Lys Ser Thr Ala Thr Ile
65                  70                  75                  80

Ser Gly Leu Lys Pro Gly Val Asp Tyr Thr Ile Thr Val Tyr Ala Val
                85                  90                  95

Thr Pro Arg Gly Asp Trp Asn Glu Gly Ser Lys Pro Ile Ser Ile Asn
            100                 105                 110

Tyr Arg Thr Glu Gln Lys Leu Ile Ser Glu Glu Asp Leu Gly Gly Gly
        115                 120                 125

Pro Arg Lys Pro Thr Thr Thr Pro Ala Pro Arg Pro Pro Thr Pro Ala
    130                 135                 140

Pro Thr Ile Ala Ser Gln Pro Leu Ser Leu Arg Pro Glu Ala Cys Arg
145                 150                 155                 160

Pro Ala Ala Gly Gly Ala Val His Thr Arg Gly Leu Asp Phe Ala Cys
                165                 170                 175

Asp Ile Tyr Ile Trp Ala Pro Leu Ala Gly Thr Cys Gly Val Leu Leu
            180                 185                 190

Leu Ser Leu Val Ile Thr Lys Arg Gly Arg Lys Lys Leu Leu Tyr Ile
        195                 200                 205

Phe Lys Gln Pro Phe Met Arg Pro Val Gln Thr Thr Gln Glu Glu Asp
    210                 215                 220

Gly Cys Ser Cys Arg Phe Pro Glu Glu Glu Glu Gly Gly Cys Glu Leu
225                 230                 235                 240

Arg Val Lys Phe Ser Arg Ser Ala Asp Ala Pro Ala Tyr Gln Gln Gly
                245                 250                 255

Gln Asn Gln Leu Tyr Asn Glu Leu Asn Leu Gly Arg Arg Glu Glu Tyr
            260                 265                 270

Asp Val Leu Asp Lys Arg Arg Gly Arg Asp Pro Glu Met Gly Gly Lys
        275                 280                 285

Pro Gln Arg Arg Lys Asn Pro Gln Glu Gly Leu Tyr Asn Glu Leu Gln
    290                 295                 300

Lys Asp Lys Met Ala Glu Ala Tyr Ser Glu Ile Gly Met Lys Gly Glu
305                 310                 315                 320

Arg Arg Arg Gly Lys Gly His Asp Gly Leu Tyr Gln Gly Leu Ser Thr
                325                 330                 335

Ala Thr Lys Asp Thr Tyr Asp Ala Leu His Met Gln Ala Leu Pro Pro
            340                 345                 350

Arg

<210> 38
<211> 351
<212> PRT
<213> Artificial Sequence

<220>
<223> YYB 107 CAR

<400> 38
Met Gly Trp Ser Cys Ile Ile Leu Phe Leu Val Ala Thr Ala Thr Gly
1               5                   10                  15

Val His Ser Val Ser Asp Val Pro Arg Asp Leu Glu Val Val Ala Ala
            20                  25                  30

Thr Pro Thr Ser Leu Leu Ile Ser Trp Asp Ala Pro Ala Val Thr Val
        35                  40                  45

Arg Tyr Tyr Arg Ile Thr Tyr Gly Glu Thr Gly Gly Asn Ser Pro Val
    50                  55                  60

Gln Glu Phe Thr Val Pro Gly Ser Lys Ser Thr Ala Thr Ile Ser Gly
65                  70                  75                  80

Leu Lys Pro Gly Val Asp Tyr Thr Ile Thr Val Tyr Ala Val Thr Pro
                85                  90                  95

Arg Gly Asp Trp Asn Glu Gly Ser Lys Pro Ile Ser Ile Asn Tyr Arg
            100                 105                 110

Thr Glu Gln Lys Leu Ile Ser Glu Glu Asp Leu Gly Gly Gly Pro Arg
        115                 120                 125

Lys Pro Thr Thr Thr Pro Ala Pro Arg Pro Pro Thr Pro Ala Pro Thr
    130                 135                 140

Ile Ala Ser Gln Pro Leu Ser Leu Arg Pro Glu Ala Cys Arg Pro Ala
145                 150                 155                 160

Ala Gly Gly Ala Val His Thr Arg Gly Leu Asp Phe Ala Cys Asp Ile
                165                 170                 175

Tyr Ile Trp Ala Pro Leu Ala Gly Thr Cys Gly Val Leu Leu Leu Ser
            180                 185                 190

Leu Val Ile Thr Lys Arg Gly Arg Lys Lys Leu Leu Tyr Ile Phe Lys
        195                 200                 205

Gln Pro Phe Met Arg Pro Val Gln Thr Thr Gln Glu Glu Asp Gly Cys
    210                 215                 220

Ser Cys Arg Phe Pro Glu Glu Glu Glu Gly Gly Cys Glu Leu Arg Val
225                 230                 235                 240

Lys Phe Ser Arg Ser Ala Asp Ala Pro Ala Tyr Gln Gln Gly Gln Asn
                245                 250                 255

Gln Leu Tyr Asn Glu Leu Asn Leu Gly Arg Arg Glu Glu Tyr Asp Val
            260                 265                 270

Leu Asp Lys Arg Arg Gly Arg Asp Pro Glu Met Gly Gly Lys Pro Gln
        275                 280                 285

Arg Arg Lys Asn Pro Gln Glu Gly Leu Tyr Asn Glu Leu Gln Lys Asp
    290                 295                 300

Lys Met Ala Glu Ala Tyr Ser Glu Ile Gly Met Lys Gly Glu Arg Arg
305                 310                 315                 320

Arg Gly Lys Gly His Asp Gly Leu Tyr Gln Gly Leu Ser Thr Ala Thr
                325                 330                 335

Lys Asp Thr Tyr Asp Ala Leu His Met Gln Ala Leu Pro Pro Arg
            340                 345                 350

<210>    39
<211>    497
<212>    PRT
<213>    Artificial Sequence

<220>
<223>    YYB 105 CAR

<400>    39
Met Gly Trp Ser Cys Ile Ile Leu Phe Leu Val Ala Thr Ala Thr Gly
 1               5                  10                  15

Val His Ser Gln Val Gln Leu Gln Glu Ser Gly Gly Gly Leu Val Lys
            20                  25                  30

Pro Gly Gly Ser Leu Lys Leu Ser Cys Ala Ala Ser Gly Phe Thr Phe
        35                  40                  45

Ser Lys Phe Gly Met Ser Trp Val Arg Gln Thr Pro Asp Lys Arg Leu
    50                  55                  60

Glu Trp Val Ala Ser Ile Ser Thr Gly Gly Tyr Asn Thr Phe Tyr Ser
65                  70                  75                  80

Asp Asn Val Lys Gly Arg Phe Thr Ile Ser Arg Asp Asn Ala Lys Asn
                85                  90                  95

Thr Leu Tyr Leu Gln Met Ser Ser Leu Lys Ser Glu Asp Thr Ala Met
            100                 105                 110

Tyr Tyr Cys Ala Arg Gly Tyr Ser Ser Thr Ser Phe Ala Met Asp Tyr
        115                 120                 125

Trp Gly Gln Gly Thr Met Val Thr Val Ser Ser Gly Ser Thr Ser Gly
    130                 135                 140

Ser Gly Lys Pro Gly Ser Gly Glu Gly Ser Asp Ile Gln Met Thr Gln
145                 150                 155                 160

Ser Pro Ser Ser Leu Ser Ala Ser Val Gly Asp Arg Val Thr Ile Thr
                165                 170                 175

Cys Met Thr Ser Thr Asp Ile Asp Asp Asp Met Asn Trp Tyr Gln Gln
            180                 185                 190

Lys Pro Gly Lys Thr Pro Lys Leu Leu Ile Tyr Glu Gly Asn Thr Leu
        195                 200                 205

Arg Pro Gly Val Pro Ser Arg Phe Ser Gly Ser Gly Ser Gly Thr Asp
    210                 215                 220

Phe Ile Phe Thr Ile Ser Ser Leu Gln Pro Glu Asp Ile Ala Thr Tyr
225                 230                 235                 240

Tyr Cys Leu Gln Ser Phe Asn Val Pro Leu Thr Phe Gly Gly Gly Thr
                245                 250                 255

Lys Val Glu Ile Lys Glu Gln Lys Leu Ile Ser Glu Glu Asp Leu Gly
            260                 265                 270

Gly Gly Pro Arg Lys Pro Thr Thr Thr Pro Ala Pro Arg Pro Pro Thr
        275                 280                 285

Pro Ala Pro Thr Ile Ala Ser Gln Pro Leu Ser Leu Arg Pro Glu Ala
    290                 295                 300

Ala Arg Pro Ala Ala Gly Gly Ala Val His Thr Arg Gly Leu Asp Phe
305                 310                 315                 320

Ala Leu Ala Tyr Leu Leu Asp Gly Ile Leu Phe Ile Tyr Gly Val Ile
                325                 330                 335

Leu Thr Ala Leu Phe Leu Arg Val Lys Arg Gly Arg Lys Lys Leu Leu
            340                 345                 350

Tyr Ile Phe Lys Gln Pro Phe Met Arg Pro Val Gln Thr Thr Gln Glu
        355                 360                 365

Glu Asp Gly Cys Ser Cys Arg Phe Pro Glu Glu Glu Glu Gly Gly Cys
    370                 375                 380

Glu Leu Lys Phe Ser Arg Ser Ala Asp Ala Pro Ala Tyr Gln Gln Gly
385                 390                 395                 400

Gln Asn Gln Leu Tyr Asn Glu Leu Asn Leu Gly Arg Arg Glu Glu Tyr
                405                 410                 415

Asp Val Leu Asp Lys Arg Arg Gly Arg Asp Pro Glu Met Gly Gly Lys
            420                 425                 430

Pro Gln Arg Arg Lys Asn Pro Gln Glu Gly Leu Tyr Asn Glu Leu Gln
        435                 440                 445

Lys Asp Lys Met Ala Glu Ala Tyr Ser Glu Ile Gly Met Lys Gly Glu
    450                 455                 460

Arg Arg Arg Gly Lys Gly His Asp Gly Leu Tyr Gln Gly Leu Ser Thr
465                 470                 475                 480

Ala Thr Lys Asp Thr Tyr Asp Ala Leu His Met Gln Ala Leu Pro Pro
                485                 490                 495

Arg

<210>    40
<211>    21
<212>    PRT
<213>    Artificial Sequence

<220>
<223>    T2A peptide


<400>    40
Gly Ser Gly Glu Gly Arg Gly Ser Leu Leu Thr Cys Gly Asp Val Glu
1               5                   10                  15

Glu Asn Pro Gly Pro
            20


<210>    41
<211>    22
<212>    PRT
<213>    Artificial Sequence

<220>
<223>    P2A peptide


<400>    41
Gly Ser Gly Ala Thr Asn Phe Ser Leu Leu Lys Gln Ala Gly Asp Val
1               5                   10                  15

Glu Glu Asn Pro Gly Pro
            20


<210>    42
<211>    23
<212>    PRT
<213>    Artificial Sequence

<220>
<223>    E2A peptide


<400>    42
Gly Ser Gly Gln Cys Thr Asn Tyr Ala Leu Leu Lys Leu Ala Gly Asp
1               5                   10                  15

Val Glu Ser Asn Pro Gly Pro
            20


<210>    43
<211>    25
<212>    PRT
<213>    Artificial Sequence

<220>
<223>    F2A peptide


<400>    43
Gly Ser Gly Val Lys Gln Thr Leu Asn Phe Asp Leu Leu Lys Leu Ala

1               5                   10                  15
Gly Asp Val Glu Ser Asn Pro Gly Pro
            20                  25

## Claims

1. A polypeptide comprising a chimeric antigen receptor, which comprises an antigen binding domain; a hinge region; a transmembrane domain; a costimulatory domain; and a signaling domain,
wherein the signaling domain comprises a CD3ζ domain.

2. The polypeptide of claim 1, which comprises IL-7Rα or a part thereof interposed between the costimulatory domain and the CD3ζ domain.

3. The polypeptide of claim 1, which comprises IL-2Rβ or a part thereof interposed between the costimulatory domain and the CD3ζ domain.

4. The polypeptide of claim 2, wherein a part of IL-7Rα is a sequence of SEQ ID NO: 15.

5. The polypeptide of claim 3, wherein a part of IL-2Rβ is a sequence of SEQ ID NO: 17.

6. The polypeptide of claim 2, which comprises three immunoreceptor tyrosine-based activation motifs (ITAMs) within the CD3ζ domain,
wherein in a third ITAM region, a first motif, YxxL, is substituted with YxxQ; and a second motif region, YxxLHM, is substituted with YxYVTM.

7. The polypeptide of claim 3, which comprises three immunoreceptor tyrosine-based activation motifs (ITAMs) interposed within the CD3ζ domain,
wherein in a third ITAM region, a first motif, YxxL, is substituted with YyyL; and a second motif, YxxL, is substituted with YxxQ.

8. The polypeptide of claim 4, which comprises three immunoreceptor tyrosine-based activation motifs (ITAMs) within the CD3ζ domain,
wherein a third ITAM region is mutated to a sequence of SEQ ID NO: 31.

9. The polypeptide of claim 5, which comprises three immunoreceptor tyrosine-based activation motifs (ITAMs) within the CD3ζ domain,
wherein a third ITAM region is mutated to a sequence of SEQ ID NO: 32.

10. The polypeptide of claim 4 or 5, wherein the CD3ζ domain is a sequence represented by SEQ ID NO: 18.

11. The polypeptide of claim 4 or 5, wherein a third ITAM region is a sequence between the 91^{st} and the 113^{th} positions in a sequence represented by SEQ ID NO: 18.

12. The polypeptide of claim 4, wherein in a third ITAM region, a first motif, YxxL, is substituted with YxxQ; and a second motif, YxxLHM, is substituted with YxYVTM, wherein x is any amino acid.

13. The polypeptide of claim 5, wherein in a third ITAM region, a first motif, YxxL, is substituted with YyyL; and a second motif, YxxL, is substituted with YxxQ,
wherein x and y are each any amino acid.

14. The polypeptide of any one of claims 2 to 7, which further comprises a cytokine.

15. The polypeptide of claim 14, wherein the cytokine is IL-21.

16. The polypeptide of claim 15, wherein the cytokine is linked to a chimeric antigen receptor by a self-cleaving peptide.

17. The polypeptide of claim 14, wherein the cytokine expressed in a T cell is separated from the chimeric antigen derivative and then released to the outside of the T cell.

18. The polypeptide of any one of claims 2 to 7, which further comprises a TGF-βR2 exodomain and an IL18R endodomain, wherein an IL18R transmembrane domain is comprised between the TGF-βR2 exodomain and the IL18R endodomain.

19. The polypeptide of claim 18, wherein the TGF-βR2 exodomain is linked to a chimeric antigen receptor by a self-cleaving peptide.

20. The polypeptide of claim 19, wherein the TGF-β exodomain, IL18R transmembrane domain, and IL18R endodomain, which is expressed in a T cell, is separated from the chimeric antigen receptor, and among these, the TGF-βR2 domain is exposed to the outside of the T cell,
wherein the IL18R endodomain is activated by the linking of the TGF-β present outside of the T cell to the TGF-β exodomain.

21. The polypeptide of claim 18, which further comprises a cytokine.

22. The polypeptide of claim 21, wherein the cytokine is IL-21.

23. The polypeptide of claim 21, wherein the cytokine is linked to an IL18R endodomain by a self-cleaving peptide.

24. The polypeptide of claim 23, wherein the cytokine expressed in a T cell is separated from the IL18R endodomain and then released to the outside of the T cell.

25. The polypeptide of claim 2 or 3, wherein the antigen binding domain binds to an antigen selected from the group consisting of IL13Rα2, an antigen associated with an angiogenesis activity, EGFRvIII, EphA2, αVβ3, mesothelin, and glypican.

26. The polypeptide of claim 1, wherein the costimulatory domain comprises one or more selected from the group consisting of 4-1BB and a CD28 domain.

27. A polypeptide represented by any one sequence of SEQ ID NOS: 23 to 30 and 34 to 37.

28. The polypeptide of any one of claims 1 to 26 for the treatment of solid cancer.

29. A CAR-T cell, wherein a polypeptide comprising the chimeric antigen receptor according to any one of claims 1 to 26 is expressed.

30. A CAR-T cell, wherein a polypeptide comprising the chimeric antigen receptor according to claim 27 is expressed.

31. A CAR expression vector, which is a vector expressing a chimeric antigen receptor (CAR) and which comprises an antigen binding domain; a hinge region; a transmembrane domain; a costimulatory domain; and a cytoplasmic signaling domain,
wherein the CAR expression vector comprises a nucleic acid encoding a CAR,
wherein the nucleic acid encoding a CAR comprises a nucleic acid encoding the costimulatory domain and a nucleic acid encoding a CD3ζ domain as the signaling domain, and which further comprises a nucleic acid encoding IL-7Rα or a part thereof that is interposed between the nucleic acid encoding the costimulatory domain and the nucleic acid encoding the CD3ζ domain.

32. A CAR expression vector, which is a vector expressing a chimeric antigen receptor (CAR) and which comprises an antigen binding domain; a hinge region; a transmembrane domain; a costimulatory domain; and a cytoplasmic signaling domain,
wherein the CAR expression vector comprises a nucleic acid encoding a CAR,
wherein the nucleic acid encoding a CAR comprises a nucleic acid encoding the costimulatory domain and a nucleic acid encoding a CD3ζ domain as the signaling domain, and which further comprises a nucleic acid encoding IL-2Rβ or a part thereof that is interposed between the nucleic acid encoding the costimulatory domain and the nucleic acid encoding the CD3ζ domain.

33. The CAR expression vector of claim 31, wherein a part of IL-7Ra is a sequence of SEQ ID NO: 15.

34. The CAR expression vector of claim 32, wherein a part of IL-2Rβ is a sequence of SEQ ID NO: 17.

35. The CAR expression vector of claim 31, wherein the nucleic acid encoding the CD3ζ domain is a nucleic acid encoding a CD3ζ domain, in which in a third ITAM region among the three ITAMs present within the CD3ζ domain, a first motif, YxxL, and a second motif, YxxLHM, are substituted.

36. The CAR expression vector of claim 32, wherein the nucleic acid encoding the CD3ζ domain is a nucleic acid encoding a CD3ζ domain, in which in a third ITAM region among the three ITAMs present within the CD3ζ domain, a first motif, YxxL, and a second motif, YxxL, are substituted.

37. The CAR expression vector of claim 35, wherein the nucleic acid encoding the CD3ζ domain is a nucleic acid encoding a CD3ζ domain, in which in a third ITAM region, a first motif, YxxL, is substituted with YxxQ; and a second motif region, YxxLHM, is substituted with YxYVTM, wherein x is any amino acid.

38. The CAR expression vector of claim 36, wherein the nucleic acid encoding the CD3ζ domain is a nucleic acid encoding a CD3ζ domain, in which in a third ITAM region, a first motif, YxxL, is substituted with YyyL; and a second motif, YxxL, is substituted with YxxQ, wherein x and y are each any amino acid.

39. The CAR expression vector of any one of claims 31 to 36, which further comprises a nucleic acid encoding cytokine IL-21.

40. The CAR expression vector of claim 39, wherein the nucleic acid encoding cytokine IL-21 is linked to a nucleic acid encoding a CAR through a nucleic acid encoding a self-cleaving peptide.

41. The CAR expression vector of any one of claims 31 to 36, which further comprises a nucleic acid encoding a TGF-βR2 exodomain and a nucleic acid encoding an IL18R transmembrane domain and an IL18R endodomain.

42. The CAR expression vector of claim 41, wherein the nucleic acid encoding a TGF-βR2 exodomain is linked to a nucleic acid encoding a CAR through a nucleic acid encoding a self-cleaving peptide.

43. The CAR expression vector of claim 41, which further comprises a nucleic acid encoding cytokine IL-21.

44. The CAR expression vector of claim 43, wherein the nucleic acid encoding cytokine IL-21 is linked to a nucleic acid encoding IL18R endodomain through a self-cleaving peptide-encoding nucleic acid.

45. A CAR-T cell, which is prepared by introducing the vector according to any one of claims 31 to 39.

46. A CAR-T cell, which is prepared by introducing the vector according to claim 41.

47. A CAR-T cell, which is prepared by introducing the vector according to claim 43.

48. An anticancer agent comprising the CAR-T cell according to claim 45.

49. The CAR expression vector of claim 37 or 38, wherein the costimulatory domain is one or more selected from the group consisting of 4-1BB and a CD28 domain.
